# EUROPEAN PATENT APPLICATION

(11) **EP 4 822 026 A1**
(43) Date of publication of application: **14.10.2026**
(21) Application number: 24899941.9
(22) Date of filing: 06.12.2024
(51) Int. Cl.: C12N 15/85, C12N 15/113, C12N 15/62, C07K 19/00, C12N 5/10, A61K 38/50, A61K 48/00, A61P 27/02

(54) **OPTIMIZED QBE BASE EDITING SYSTEM AND USE THEREOF**

(30) Priority: 06.12.2023 CN 202311662136; 11.01.2024 CN 202410044584; 04.06.2024 CN 202410718712
(71) Applicant: Beijing Qi Biodesign Biotechnology Company Limited, Beijing 102209 (CN)
(72) Inventor: ZHAO, Kevin T., Beijing 102206 (CN); HU, Jiacheng, Beijing 102206 (CN)
(74) Representative: Weickmann & Weickmann PartmbB
(86) International application number: PCT/CN2024/137281
(87) International publication number: WO 2025/119307

(57) **Abstract**

The present invention belongs to the field of genetic engineering. Specifically, the present invention relates to a system for modifying or editing a target nucleotide base, a tool and a method, and more specifically, relates to an optimized QBE base editing system and the use thereof. The base editing system has high gene editing tool efficiency, high product purity, low Indel frequency, low off-target frequency and rich gene editing windows, and therefore has important value for expanding the application scenarios of base editing technology in the fields of life science basic research, agriculture, biomedicine and the like.

## Description

### CROSS-REFERENCE TO RELATED APPLICATIONS

The present application claims the benefits of priority of the Chinese Patent Application Nos. 202311662136.4, 202410044584.6, and 202410718712.0, filed on December 6, 2023, January 11, 2024, and June 4, 2024 respectively and entitled "OPTIMIZED BASE EDITING SYSTEM AND USE THEREOF", the entire contents of which, including the appendices are incorporated herein by reference.

### TECHNICAL FIELD

The present disclosure belongs to the field of genetic engineering. Specifically, the present disclosure relates to a system, a tool, and a method for modifying or editing a target nucleotide base. More specifically, the present disclosure relates to an optimized QBE base editing system and use thereof.

### BACKGROUND

Base editing refers to a process of nucleotide substitution at a specific DNA site by means of genetic engineering. Base editing is a type of genome editing technology. In plants, many desirable agronomic traits arise from single nucleotide variations (SNVs). In humans and animals, many genetic diseases are caused by point mutations in functional genes. Base editing technology enables targeted modifications and engineering of genomic DNA in plants, animals, and microorganisms to create new genotypes and acquire target traits beneficial for production and application. It can also be used to correct some severe congenital genetic variations in the treatment of genetic diseases. Thus, base editing holds significant application prospects in the creation of elite germplasm and in the disease treatment.

Base editing is achieved by virtue of a base editor. According to the types of base on which the base editors act, the base editors can be classified into two categories: 1) a cytosine base editor (CBE) and 2) an adenine base editor (ABE). Of these, the CBE can act on the cytosine base of cytosine deoxyribonucleotides (simply referred to as cytidine, C), and the ABE can act on the adenine base of adenine deoxyribonucleotides (simply referred to as adenosine, A). The CBE can convert the C-G base pair on the double-stranded DNA to the T-A base pair; and the ABE can convert the A-T base pair on the double-stranded DNA to the G-C base pair.

Because the DNA-binding proteins used in base editing systems typically target only single-stranded DNA, any non-targeted single-stranded DNA is not encapsulated by the proteins. As a result, the free single-stranded DNA forms an editing window having multiple bases. For example, CBE has an editing window of 4 to 5 bases, and ABE has an editing window of 3 to 4 bases. Within the editing window, every cytidine or adenosine has a chance of being edited.

Modulating the size of the editing window in a base editing system is of practical significance. In one aspect, because the Cas protein targeting is PAM sequence-dependent, certain bases in the genome are restricted by PAM and cannot be edited. For example, only approximately 20% of cytidines or guanines in the human genome are editable. Therefore, expanding the base editing window and increasing the probability of editing bases within the editing window distal to the PAM contribute to expansion of the range of editable sites in the genome.

Therefore, expanding the editing window while maintaining the efficiency and indel frequency of gene editing tools is of significant value for broadening the application scenarios of base editing technology in the fields, such as basic life science research, agriculture, and biomedicine.

### SUMMARY

### Technical Problems

To enrich the toolbox of base editing systems and broaden their application scenarios, the present disclosure provides a series of new base editing systems that can achieve cytosine base editing or adenine base editing.

### Solutions to Problems

In the first aspect of the present disclosure, there is provided a base editing fusion protein, wherein the base editing fusion protein comprises a deaminase domain and paired DNA-binding protein domains having a DNA single-strand cleavage function; and
the paired DNA-binding protein domains having the DNA single-strand cleavage function comprise a deaminase N-terminal linker domain and a deaminase C-terminal linker domain that are linked via an optional linker to the N-terminus and the C-terminus of the deaminase domain, respectively.

In some embodiments, the base editing fusion protein comprises the following structure: [deaminase N-terminal linker domain]-[deaminase]-[deaminase C-terminal linker domain], wherein "-" is an optional linker.

In some embodiments, the deaminase N-terminal linker domain and the deaminase C-terminal linker domain are independently composed of 1 or 2 single domains.

In some optional embodiments, the base editing fusion protein comprises the following structure: [X₁]-[X₂]-[deaminase]-[X₃]-[X₄], wherein X₁₋₄ are single domains, and "-" is an optional linker.

In some optional embodiments, the X₁₋₄ single domains are selected from the following combinations:
(1) X₁ is SEQ ID NO: 158, X₂ is SEQ ID NO: 159, X₃ is SEQ ID NO: 160, and X₄ is absent;
(2) X₁ is absent, X₂ is SEQ ID NO: 161, X₃ is SEQ ID NO: 162, and X₄ is SEQ ID NO: 163;
(3) X₁ is absent, X₂ is SEQ ID NO: 164, X₃ is SEQ ID NO: 165, and X₄ is SEQ ID NO: 166;
(4) X₁ is SEQ ID NO: 181, X₂ is SEQ ID NO: 182, X₃ is a sequence Q, and X₄ is absent;
(5) X₁ is absent, X₂ is SEQ ID NO: 183, X₃ is SEQ ID NO: 184, and X₄ is SEQ ID NO: 185;
(6) X₁ is absent, X₂ is SEQ ID NO: 186, X₃ is SEQ ID NO: 187, and X₄ is SEQ ID NO: 188;
(7) X₁ is SEQ ID NO: 189, X₂ is SEQ ID NO: 190, X₃ is SEQ ID NO: 191, and X₄ is absent;
(8) X₁ is SEQ ID NO: 192, X₂ is SEQ ID NO: 193, X₃ is SEQ ID NO: 194, and X₄ is absent;
(9) X₁ is SEQ ID NO: 195, X₂ is SEQ ID NO: 196, X₃ is SEQ ID NO: 197, and X₄ is absent; and
(10) X₁ is SEQ ID NO: 198, X₂ is SEQ ID NO: 199, X₃ is SEQ ID NO: 200, and X₄ is absent.

In some embodiments, the deaminase is a cytidine deaminase or an adenosine deaminase.

In some embodiments, the cytidine deaminase is an APOBEC family deaminase, an SCP1.201 family deaminase or a homolog thereof.

In some preferred embodiments, the APOBEC family deaminase is an AID deaminase, an APOBEC1 deaminase, an APOBEC2 deaminase, an APOBEC3A deaminase, an APOBEC3B deaminase, an APOBEC3C deaminase, an APOBEC3D deaminase, an APOBEC3F deaminase, an APOBEC3G deaminase, an APOBEC3H deaminase, or a variant thereof.

In some preferred embodiments, the SCP1.201 family deaminase is an Sdd2 deaminase, an Sdd3 deaminase, an Sdd4 deaminase, an Sdd6 deaminase, an Sdd7 deaminase, an Sdd9 deaminase, an Sdd10 deaminase, an Sdd59 deaminase, a mini-Sdd3 deaminase, a mini-Sdd6 deaminase, a mini-Sdd7 deaminase, a mini-Sdd9 deaminase, or a variant thereof.

In some embodiments, the adenosine deaminase is QB34, TadA8e, TadA7.10, a TadA dimer, or a variant thereof.

In some optional embodiments, the TadA dimer comprises TadA7.10 and wild-type TadA.

In some embodiments, the base editing fusion protein further comprises a uracil DNA glycosylase inhibitor (UGI) domain and/or a nuclear localization sequence (NLS).

In some embodiments, the linker has a length of 0 to 100 amino acids.

In some preferred embodiments, the linker has a length of 12 to 100 amino acids.

In some optional embodiments, the linker comprises an amino acid sequence (GGGS)n (SEQ ID NO: 47), (GGGGS)n (SEQ ID NO: 48), (G)n, (EAAAK)n (SEQ ID NO: 49), (GGS)n, (SGGS)n (SEQ ID NO: 51), or SGSETPGTSESATPES (SEQ ID NO: 52), or an (XP)n motif, or a combination thereof, wherein n is independently an integer from 1 to 32, and wherein X is any amino acid.

In some embodiments, the base editing fusion protein comprises one or more of the following sequences:
(i) an amino acid sequence set forth in any one of SEQ ID NOs: 89-92, 94, 96, 99, 101, 103, 105-115, 118, 179, and 201-206;
(ii) an amino acid sequence having at least 80%, 82%, 85%, 87%, 90%, 92%, 95%, 96%, 97%, 98% or 99% sequence identity to the amino acid sequence set forth in any one of SEQ ID NOs: 89-92, 94, 96, 99, 101, 103, 105-115, 118, 179, and 201-206;
(iii) an amino acid sequence obtained by adding, substituting, deleting or inserting one or more amino acid residues in the amino acid sequence set forth in any one of SEQ ID NOs: 89-92, 94, 96, 99, 101, 103, 105-115, 118, 179, and 201-206; or
(iv) an amino acid sequence encoded by a nucleotide sequence that hybridizes with a polynucleotide sequence encoding the amino acid sequence set forth in any one of SEQ ID NOs: 89-92, 94, 96, 99, 101, 103, 105-115, 118, 179, and 201-206 under stringency conditions, wherein the stringency conditions are moderate stringency conditions, moderate-high stringency conditions, high stringency conditions, or very high stringency conditions.

In some embodiments, the base editing fusion protein forms a complex with a guide RNA and binds to a target DNA.

In the second aspect of the present disclosure, there is provided a base editing system, wherein the base editing system comprises at least one of the following components i) to v):
i) a base editing fusion protein, and a guide RNA;
ii) an expression construct comprising a nucleotide sequence encoding a base editing fusion protein, and a guide RNA;
iii) a base editing fusion protein, and an expression construct comprising a nucleotide sequence encoding a guide RNA;
iv) an expression construct comprising a nucleotide sequence encoding a base editing fusion protein, and an expression construct comprising a nucleotide sequence encoding a guide RNA; and
v) an expression construct comprising a nucleotide sequence encoding a base editing fusion protein and a nucleotide sequence encoding a guide RNA;

wherein the base editing fusion protein comprises a deaminase domain and paired DNA-binding protein domains having a DNA single-strand cleavage function; and
the paired DNA-binding protein domains having the DNA single-strand cleavage function comprise a deaminase N-terminal linker domain and a deaminase C-terminal linker domain that are linked via an optional linker to the N-terminus and the C-terminus of the deaminase domain, respectively.

In some embodiments, the base editing fusion protein comprises the following structure: [deaminase N-terminal linker domain]-[deaminase]-[deaminase C-terminal linker domain], wherein "-" is an optional linker.

In some embodiments, the deaminase N-terminal linker domain and the deaminase C-terminal linker domain are independently composed of 1 or 2 single domains.

In some optional embodiments, the base editing fusion protein comprises the following structure: [X₁]-[X₂]-[deaminase]-[X₃]-[X₄], wherein X₁₋₄ are single domains, and "-" is an optional linker.

In some embodiments, the X₁₋₄ single domains are selected from the following combinations:
(1) X₁ is SEQ ID NO: 158, X₂ is SEQ ID NO: 159, X₃ is SEQ ID NO: 160, and X₄ is absent;
(2) X₁ is absent, X₂ is SEQ ID NO: 161, X₃ is SEQ ID NO: 162, and X₄ is SEQ ID NO: 163;
(3) X₁ is absent, X₂ is SEQ ID NO: 164, X₃ is SEQ ID NO: 165, and X₄ is SEQ ID NO: 166;
(4) X₁ is SEQ ID NO: 181, X₂ is SEQ ID NO: 182, X₃ is a sequence Q, and X₄ is absent;
(5) X₁ is absent, X₂ is SEQ ID NO: 183, X₃ is SEQ ID NO: 184, and X₄ is SEQ ID NO: 185;
(6) X₁ is absent, X₂ is SEQ ID NO: 186, X₃ is SEQ ID NO: 187, and X₄ is SEQ ID NO: 188;
(7) X₁ is SEQ ID NO: 189, X₂ is SEQ ID NO: 190, X₃ is SEQ ID NO: 191, and X₄ is absent;
(8) X₁ is SEQ ID NO: 192, X₂ is SEQ ID NO: 193, X₃ is SEQ ID NO: 194, and X₄ is absent;
(9) X₁ is SEQ ID NO: 195, X₂ is SEQ ID NO: 196, X₃ is SEQ ID NO: 197, and X₄ is absent; and
(10) X₁ is SEQ ID NO: 198, X₂ is SEQ ID NO: 199, X₃ is SEQ ID NO: 200, and X₄ is absent.

In some embodiments, the deaminase is a cytidine deaminase or an adenosine deaminase.

In some embodiments, the cytidine deaminase is an APOBEC family deaminase, an SCP1.201 family deaminase or a homolog thereof.

In some preferred embodiments, the APOBEC family deaminase is an AID deaminase, an APOBEC1 deaminase, an APOBEC2 deaminase, an APOBEC3A deaminase, an APOBEC3B deaminase, an APOBEC3C deaminase, an APOBEC3D deaminase, an APOBEC3F deaminase, an APOBEC3G deaminase, an APOBEC3H deaminase, or a variant thereof.

In some preferred embodiments, the SCP1.201 family deaminase is an Sdd2 deaminase, an Sdd3 deaminase, an Sdd4 deaminase, an Sdd6 deaminase, an Sdd7 deaminase, an Sdd9 deaminase, an Sdd10 deaminase, an Sdd59 deaminase, a mini-Sdd3 deaminase, a mini-Sdd6 deaminase, a mini-Sdd7 deaminase, a mini-Sdd9 deaminase, or a variant thereof.

In some embodiments, the adenosine deaminase is QB34, TadA8e, TadA7.10, a TadA dimer, or a variant thereof, and optionally, the TadA dimer comprises TadA7.10 and wild-type TadA.

In some embodiments, the base editing fusion protein further comprises a uracil DNA glycosylase inhibitor (UGI) domain and/or a nuclear localization sequence (NLS).

In some embodiments, the linker has a length of 0 to 100 amino acids.

In some preferred embodiments, the linker has a length of 12 to 100 amino acids.

In some optional embodiments, the linker comprises an amino acid sequence (GGGS)n (SEQ ID NO: 47), (GGGGS)n (SEQ ID NO: 48), (G)n, (EAAAK)n (SEQ ID NO: 49), (GGS)n, (SGGS)n (SEQ ID NO: 51), or SGSETPGTSESATPES (SEQ ID NO: 52), or an (XP)n motif, or a combination thereof, wherein n is independently an integer from 1 to 32, and wherein X is any amino acid.

In some embodiments, the base editing fusion protein comprises one or more of the following sequences:
(i) an amino acid sequence set forth in any one of SEQ ID NOs: 89-92, 94, 96, 99, 101, 103, 105-115, 118, 179, and 201-206;
(ii) an amino acid sequence having at least 80%, 82%, 85%, 87%, 90%, 92%, 95%, 96%, 97%, 98% or 99% sequence identity to the amino acid sequence set forth in any one of SEQ ID NOs: 89-92, 94, 96, 99, 101, 103, 105-115, 118, 179, and 201-206;
(iii) an amino acid sequence obtained by adding, substituting, deleting or inserting one or more amino acid residues in the amino acid sequence set forth in any one of SEQ ID NOs: 89-92, 94, 96, 99, 101, 103, 105-115, 118, 179, and 201-206; or
(iv) an amino acid sequence encoded by a nucleotide sequence that hybridizes with a polynucleotide sequence encoding the amino acid sequence set forth in any one of SEQ ID NOs: 89-92, 94, 96, 99, 101, 103, 105-115, 118, 179, and 201-206 under stringency conditions, wherein the stringency conditions are moderate stringency conditions, moderate-high stringency conditions, high stringency conditions, or very high stringency conditions.

In some embodiments, the base editing fusion protein forms a complex with a guide RNA and binds to a target DNA.

In the third aspect of the present disclosure, there is provided a polynucleotide, wherein the polynucleotide encodes:
(a) the base editing fusion protein according to the first aspect of the present disclosure; or
(b) the base editing system according to the second aspect of the present disclosure.

In the fourth aspect of the present disclosure, there is provided an expression construct, wherein the expression construct comprises the polynucleotide according to the third aspect of the present disclosure.

In some embodiments, the expression construct is selected from expression constructs from viruses, bacteria, yeast, plants, and mammalian cells.

In the fifth aspect of the present disclosure, there is provided a host cell, wherein the host cell contains the base editing fusion protein according to the first aspect of the present disclosure, the base editing system according to the second aspect of the present disclosure, the polynucleotide according to the third aspect of the present disclosure, or the expression construct according to the fourth aspect of the present disclosure.

In the sixth aspect of the present disclosure, there is provided a method for producing at least one genetically modified cell, comprising introducing the base editing fusion protein according to the first aspect of the present disclosure, the base editing system according to the second aspect of the present disclosure, the polynucleotide according to the third aspect of the present disclosure, or the expression construct according to the fourth aspect of the present disclosure into the at least one cell to cause a base substitution in a nucleic acid sequence within a target nucleotide region in the at least one cell.

In some embodiments, the base editing system is introduced into the cell by a method selected from: calcium phosphate transfection, protoplast fusion, electroporation, liposome transfection, microinjection, viral infection (such as baculovirus, vaccinia virus, adenovirus, adeno-associated virus, lentivirus, or other viruses), N-acetylgalactosamine (GalNAc)-mediated transformation, particle bombardment, PEG-mediated protoplast transformation and/or *Agrobacterium*-mediated transformation.

In some embodiments, the cell is a cell derived from a prokaryote such as bacteria; or a eukaryote such as plants, fungi or vertebrates.

In some embodiments, the vertebrates are mammals such as human, mouse, rat, monkey, dog, pig, sheep, cattle, and cat; and the plants are crop plants, such as wheat, rice, corn, soybean, sunflower, sorghum, oilseed rape, alfalfa, cotton, barley, millet, sugar cane, tomato, tobacco, cassava or potato.

In the seventh aspect of the present disclosure, there is provided a pharmaceutical composition, wherein the pharmaceutical composition comprises the base editing fusion protein according to the first aspect of the present disclosure, the base editing system according to the second aspect of the present disclosure, the polynucleotide according to the third aspect of the present disclosure, the expression construct according to the fourth aspect of the present disclosure, or the host cell according to the fifth aspect of the present disclosure.

In some optional embodiments, the pharmaceutical composition further comprises a pharmaceutically acceptable carrier.

In the eighth aspect of the present disclosure, there is provided a kit, wherein the kit comprises the base editing fusion protein according to the first aspect of the present disclosure, the base editing system according to the second aspect of the present disclosure, the polynucleotide according to the third aspect of the present disclosure, the expression construct according to the fourth aspect of the present disclosure, or the host cell according to the fifth aspect of the present disclosure.

In the ninth aspect of the present disclosure, there is provided a method for modifying a target nucleic acid, wherein the method comprises a step of bringing the target nucleic acid into contact with the base editing fusion protein according to the first aspect of the present disclosure, the base editing system according to the second aspect of the present disclosure, the polynucleotide according to the third aspect of the present disclosure, the expression construct according to the fourth aspect of the present disclosure, the pharmaceutical composition according to the seventh aspect of the present disclosure, or the kit according to the eighth aspect of the present disclosure.

In the tenth aspect of the present disclosure, there is provided use of the base editing fusion protein according to the first aspect of the present disclosure, the base editing system according to the second aspect of the present disclosure, the polynucleotide according to the third aspect of the present disclosure, the expression construct according to the fourth aspect of the present disclosure, the host cell according to the fifth aspect of the present disclosure, or the pharmaceutical composition according to the seventh aspect of the present disclosure in preparation of a reagent for modifying a target nucleic acid.

In the eleventh aspect of the present disclosure, there is provided use of the base editing fusion protein according to the first aspect of the present disclosure, the base editing system according to the second aspect of the present disclosure, the polynucleotide according to the third aspect of the present disclosure, the expression construct according to the fourth aspect of the present disclosure, or the host cell according to the fifth aspect of the present disclosure in preparation of a medicament for preventing and/or treating a disease associated with or caused by a gene mutation.

### Advantageous Effects of the Invention

(1) The novel QBE base editing system provided in the present disclosure exhibits comparable or higher editing efficiency and lower indel frequency, as compared to prior-art BE base editing systems;
(2) the novel QBE base editing system provided in the present disclosure exhibits higher product purity, as compared to prior-art BE base editing systems;
(3) the novel QBE base editing system provided in the present disclosure has an editing window different from those of conventional BE editing systems, which is characterized by an expanded base editing window, enabling base editing at more positions within the editing windows; and
(4) the novel QBE base editing system provided in the present disclosure exhibits a lower off-target effect, as compared to conventional BE base editing systems.

### BRIEF DESCRIPTION OF THE DRAWINGS

FIG. 1: Schematic diagram of the configuration of QBE with a deaminase linked thereto, where the linker and NLS are selected according to the well-known techniques in the art.
FIG. 2: Schematic diagram of the structures of base editing fusion proteins constituted by QBE1-4 respectively, where the promoter, linker, NLS, and UGI are selected according to the well-known techniques in the art; it contains no UGI when the base editing system is an adenosine deaminase base editing system.
FIG. 3: Base editing efficiency of base editing systems linked to different deaminases.
FIG. 4: Indel efficiency of base editing systems linked to different deaminases.
FIG. 5: Editing efficiency/indel of base editing systems linked to different deaminases.
FIG. 6: Editing efficiency and editing products of the base editing systems at endogenous targets, where in the site sequence, black represents the protospacer sequence and blue represents the PAM sequence.
FIG. 7: Editing efficiency of the adenine base editing system at each site within the base editing window.
FIG. 8: Editing efficiency of the cytosine base editing system at each site within the base editing window.
FIG. 9: Grayscale diagram of the cytosine base editing system at each site within the base editing window.
FIG. 10: Structural prediction of ternary complexes of base editors (BE4max and QBEmax) with sgRNA and DNA using AlphaFold3.
FIG. 11: Editing effects of BE4max, QBEmax, CBE-CP1031, and CBE-Inlaid1244.
FIG. 12: Influence of QBEmax base editing systems with linkers of different lengths on editing efficiency.
FIG. 13: Base editing effects of BE4max and QBEmax in rice plant cells, where in the site sequence, black represents the protospacer sequence, and red represents the PAM sequence.
FIG. 14: Base editing effects of QBE84 to QBE90.

### DETAILED DESCRIPTION

### I. Definitions

In the present disclosure, the scientific and technical terms used herein have the meaning as commonly understood by those skilled in the art unless otherwise specified. Also, the terms related to the protein and nucleic acid chemistry, molecular biology, cell and tissue culture, microbiology, and immunology as well as the laboratory procedures described herein are terms and conventional steps widely used in the pertinent fields. For example, standard recombinant DNA and molecular cloning techniques employed in the present disclosure are well known to those skilled in the art and are described in greater details in the following literature: Sambrook, Joseph Frank et al. "Molecular Cloning: A Laboratory Manual." (2001) (hereinafter referred to as "Sambrook"). Meanwhile, to facilitate understanding of the present disclosure, definitions and explanations of the relevant terms are described below.

As used herein, the term "and/or" encompasses all combinations of the items connected by this term, and should be regarded as if each of the combinations has been separately listed herein. For example, "A and/or B" encompasses "A", "A and B", and "B". For example, "A, B and/or C" encompasses "A", "B", "C", "A and B", "A and C", "B and C", and "A and B and C".

The term "comprising", when used herein to describe the sequence of a protein or nucleic acid, means that the protein or nucleic acid may consist of the sequence, or may have an additional amino acid or nucleotide at either or both ends, while still retaining the activity described in the present disclosure. Furthermore, those skilled in the art are aware that methionine encoded by a start codon at the N-terminus of a polypeptide will be retained under certain practical circumstances (*e.g.*, when expressed in a particular expression system), without any substantive impact on the functions of the polypeptide. Therefore, when describing the amino acid sequence of a specific polypeptide in the specification and claims of the present application, even if the sequence may not comprise the methionine encoded by a start codon at the N-terminus, the sequence containing this methionine is also encompassed herein at this time, and correspondingly, its encoding nucleotide sequence may also comprise a start codon; and vice versa.

"Gene" or "genome", as used herein, encompasses not only chromosomal DNA present in the nucleus, but also organellar DNA present in the subcellular components (*e.g.*, mitochondria and plastids) of the cell.

As used herein, the "organism" includes any organism, preferably eukaryotic organism, suitable for genome editing. Examples of the organism include, but are not limited to, mammals such as humans, mice, rats, monkeys, dogs, pigs, sheep, cattle, and cats; poultry such as chickens, ducks, and geese; and plants including monocots and dicots, such as rice, corn, wheat, sorghum, barley, soybean, peanut, and arabidopsis.

"Genetically modified organism" or "genetically modified cell" means an organism or cell containing, within its genome, an exogenous polynucleotide or a modified gene or expression regulatory sequence. For example, the exogenous polynucleotide may be stably integrated into the genome of the organism or cell and inherited through successive generations. The exogenous polynucleotide may be integrated into the genome individually or as part of a recombinant DNA construct. The modified gene or expression regulatory sequence means that the sequence comprises one or more deoxynucleotide substitutions, deletions and additions in the organism or cell genome.

"Polynucleotide", "nucleic acid sequence", "nucleotide sequence", and "nucleic acid fragment" may be used interchangeably to denote a single-stranded or double-stranded RNA or DNA polymer, which may optionally contain synthetic, non-natural or altered nucleotide bases. Nucleotides are designated by their single-letter names as follows: "A" denotes adenosine or deoxyadenosine (corresponding to RNA or DNA, respectively), "C" denotes cytidine or deoxycytidine, "G" denotes guanosine or deoxyguanosine, "U" denotes uridine, "T" denotes deoxythymidine, "R" denotes purine (A or G), "Y" denotes pyrimidine (C or T), "K" denotes G or T, "H" denotes A or C or T, "I" denotes inosine, and "N" denotes any nucleotide.

In the present specification, "target nucleic acid", "target DNA", "target nucleotide", "target nucleotide region", and "target nucleic acid region" may be used interchangeably to denote a nucleic acid sequence within the genome, to which the DNA-binding protein specifically binds under the guidance of a guide nucleic acid.

In the present specification, "target sequence" or "target DNA sequence" is a sequence with which a guide nucleic acid will hybridize.

In the present specification, "protospacer sequence", "protospacer", and "editing window sequence" may be used interchangeably to denote a nucleic acid sequence within a genome that is complementary to the target sequence. In the native state, the protospacer sequence is adjacent to a protospacer adjacent motif (PAM). During the base editing, nucleotides within this sequence may be subjected to the deamination reaction and the nucleotide substitution under the action of the base editing system.

"Polypeptide", "peptide", and "protein" are used interchangeably in the present disclosure to denote a polymer of amino acid residues. These terms apply to amino acid polymers in which one or more amino acid residues are artificial chemical analogue(s) of the corresponding naturally occurring amino acid(s) and to naturally occurring amino acid polymers. The terms "polypeptide", "peptide," "amino acid sequence", and "protein" may also include modified forms thereof, including but not limited to: glycosylation, lipid ligation, sulfation, γ-carboxylation of glutamic acid residues, hydroxylation, and ADP-ribosylation.

Sequence "identity" has a meaning generally recognized in the art, and the percent sequence identity between two nucleic acid or polypeptide molecules or regions can be calculated using publicly available techniques. The sequence identity may be measured along the full length of a polynucleotide or polypeptide or along a region of the molecule. (See, *e.g.*: Lesk, A.M., ed., "Computational Molecular Biology", (1988); Smith, D.W., ed., "Biocomputing: Informatics and Genome Projects", (1993); Griffin, A.M., and Griffin, H.G., eds., "Computer Analysis of Sequence Data", Part I, (1994); von Heinje, G., "Sequence Analysis in Molecular Biology", (1987); Gribskov, M. and Devereux, J., eds., "Sequence Analysis Primer", (1991)). Although there are many methods for measuring the identity between two polynucleotides or polypeptides, the term "identity" is well known to those skilled in the art (Carrillo, H. & Lipman, D., "The multiple sequence alignment problem in biology", SIAM J Applied Math 48:1073 (1988)).

In peptides or proteins, suitable conservative amino acid substitutions are known to those skilled in the art and can generally be made without altering the biological activity of the resulting molecule. Typically, those skilled in the art will recognize that substituting a single amino acid in a non-essential region of a polypeptide does not substantially alter the biological activity (see, *e.g.*, Watson et al., "Molecular Biology of the Gene", 4th Edition, Development 1 August 1988; 103 (4): 619-623.

"Base editing system", "base editor", or "Base Editor (BE)" is a system or formulation that binds to a polynucleotide and has a nucleobase editing activity. In some embodiments, it comprises a nucleobase editing polypeptide (*e.g.*, a deaminase) and a DNA-binding protein domain (*e.g.*, a nucleic acid programmable domain), and forms a complex with a guide polynucleotide (*e*.*g*., a gRNA). In some embodiments, the nucleobase editing polypeptide is fused to a DNA-binding protein domain by fusion, linking, or inlay to form a fusion protein. In some embodiments, the base editing system is a cytosine base editor (CBE) capable of deaminating cytosine (C) in a DNA molecule. In some embodiments, the base editing system is an adenine base editor (ABE) capable of deaminating adenine (A) in a DNA molecule.

As used herein, "expression construct" or "construct" refers to a vector (such as a recombinant vector) suitable for expression of a nucleotide sequence of interest in an organism. The "expression" refers to production of a functional product. For example, the expression of a nucleotide sequence may refer to the transcription of a nucleotide sequence (*e.g.* transcription to produce mRNA or functional RNA) and/or the translation of RNA into a precursor or a mature protein.

The "expression construct" of the present disclosure may be a linear nucleic acid fragment, a circular plasmid, a viral vector or, in some embodiments, a translatable RNA (such as mRNA).

The "expression construct" of the present disclosure may include regulatory sequences and nucleotide sequences of interest that are derived from different origins, or regulatory sequences and nucleotide sequences of interest that, while derived from the same origin, are arranged in a different manner from the conventional native one.

The term "mutation" refers to a substitution of a residue in a sequence (such as a nucleic acid or amino acid sequence) with another residue, or a deletion or insertion of one or more residues in the sequence. Mutations are typically described herein by identifying the initial residue, then the position of the residue in the sequence, and the identity of the new residue.

"Promoter" refers to a nucleic acid fragment capable of controlling the transcription of another nucleic acid fragment. In some embodiments of the present disclosure, the promoter is a promoter capable of controlling the gene transcription in a cell, regardless of whether it originates from that cell. The promoter may be a constitutive promoter, a tissue-specific promoter, a developmental regulatory promoter, or an inducible promoter.

As used herein, the term "operably linked" means linking a regulatory element (for example, but not limited to, a promoter sequence or a transcription termination sequence) to a nucleic acid sequence (*e.g.*, a coding sequence or an open reading frame), such that transcription of the nucleotide sequence is controlled and regulated by the transcriptional regulatory element. Techniques for operably linking a regulatory element region to a nucleic acid molecule are known in the art.

"Introducing" a nucleic acid molecule (*e.g.*, a plasmid, a linear nucleic acid fragment, an RNA, etc.) or a protein into an organism means transforming cells of an organism with the nucleic acid or protein, such that the nucleic acid or protein can function in the cells. The "transformation" as used in the present disclosure includes stable transformation and transient transformation.

"Transient transformation" means introducing a nucleic acid molecule or protein into a cell, such that it functions but without stable inheritance of the exogenous nucleotide sequence. In transient transformation, the exogenous nucleic acid sequence is not integrated into the genome.

"Trait" refers to a physiological, morphological, biochemical, or physical characteristic of a cell or organism.

"Agronomic trait" refers particularly to measurable index parameters of crop plants, including but not limited to: leaf greenness, grain yield, growth rate, total biomass or accumulation rate, fresh weight at maturity, dry weight at maturity, fruit yield, seed yield, total nitrogen content of plants, nitrogen content of fruits, nitrogen content of seeds, nitrogen content of plant vegetative tissues, total free amino acid content of plants, free amino acid content of fruits, free amino acid content of seeds, free amino acid content of plant vegetative tissues, total protein content of plants, protein content of fruits, protein content of seeds, protein content of plant vegetative tissues, herbicide resistance and drought resistance, nitrogen uptake, root lodging, harvest index, stem lodging, plant height, ear height, ear length, disease resistance, cold resistance, salt tolerance, tiller number, and the like.

An organism that can be genomically modified by the base editing system of the present disclosure includes any organisms suitable for base editing, preferably eukaryotes. Examples of the organism include, but are not limited to, mammals such as humans, mice, rats, monkeys, dogs, pigs, sheep, cattle, and cats; poultry such as chickens, ducks, and geese; and plants including monocots and dicots, for example, the plants are crop plants including, but not limited to, wheat, rice, corn, soybean, sunflower, sorghum, oilseed rape, alfalfa, cotton, barley, millet, sugar cane, tomato, tobacco, cassava, and potato.

### II. Base Editing Fusion Protein

In one aspect of the present disclosure, there is provided a base editing fusion protein, wherein the base editing fusion protein comprises a deaminase domain and paired DNA-binding protein domains having a DNA single-strand cleavage function; and the paired DNA-binding protein domains having the DNA single-strand cleavage function comprise a deaminase N-terminal linker domain and a deaminase C-terminal linker domain that are linked via an optional linker to the N-terminus and the C-terminus of the deaminase domain, respectively.

As used herein, "DNA-binding protein" is a protein that binds to a target DNA, for example, a protein binding to a guide nucleic acid (DNA or RNA) that guides the DNA-binding protein to the target DNA sequence. For instance, a guide RNA guides the DNA-binding protein to a target DNA sequence complementary to the guide RNA. In some embodiments, the DNA-binding protein may be a binding domain of a type II or type V CRISPR system-associated protein, such as a Cas9 protein or a Cas9 protein with an inactivated or partially inactivated DNA cleavage domain. A complex of Cas9, CRISPR RNA (crRNA), trans-activating small RNA (tracrRNA) recognizes a short protospacer adjacent motif (PAM), and cleaves the target DNA in an endonucleic manner. "Guide RNA", "sgRNA" or "gRNA" may be a single RNA obtained by integrating crRNA and tracrRNA (see Jinek M., et al., Science 337:816-821(2012)). The sequences, structures, and mechanisms of action of CRISPR-associated proteins are known to those skilled in the art (see, *e.g.*, Ferretti, J J et al. "Complete genome sequence of an M1 strain of Streptococcus pyogenes." Proceedings of the National Academy of Sciences of the United States of America vol. 98,8 (2001): 4658-63.; Deltcheva E. et al. "CRISPR RNA maturation by trans-encoded small RNA and host factor RNase III." Nature 471:602-607(2011); and Jinek, Martin et al. "A programmable dual-RNA-guided DNA endonuclease in adaptive bacterial immunity." Science (New York, N.Y.) vol. 337, 6096 (2012): 816-21.). In some embodiments, sgRNA may also be a structure-predicted RNA with a guiding function (see, *e.g.*, Nguyen E, Poli M, Durrant MG, Kang B, Katrekar D, Li DB, Bartie LJ, Thomas AW, King SH, Brixi G, Sullivan J, Ng MY, Lewis A, Lou A, Ermon S, Baccus SA, Hernandez-Boussard T, Ré C, Hsu PD, Hie BL. Sequence modeling and design from molecular to genome scale with Evo. Science. 2024 Nov 15; 386(6723)).

In some embodiments, the scaffold sequence of sgRNA is set forth in SEQ ID NO: 151, 220, or 221.

In some embodiments, the DNA-binding protein described herein has a cleavage activity against a target single-stranded DNA. For example, the DNA-binding protein, after binding to the target DNA sequence under the guidance of a guide nucleic acid, cleaves only single-stranded DNA. In some embodiments of the present disclosure, the DNA-binding protein having a single-stranded DNA cleavage activity is two paired DNA-binding protein domains that are linked, via a linker of 0 to 500 (preferably 0 to 100) amino acids in length, to the N-terminus and C-terminus of the deaminase domain, respectively. In some embodiments of the present disclosure, the paired DNA-binding protein domains may be obtained by the following method: subjecting the amino acid sequence of the DNA-binding protein having a single-strand cleavage function to circular permutation to obtain a circularly permuted sequence (see, *e.g.*, Oakes, Benjamin L et al. "CRISPR-Cas9 Circular Permutants as Programmable Scaffolds for Genome Modification." Cell vol. 176, 1-2 (2019): 254-267.e16.). The circularly permuted sequence is cleaved at two sites to obtain a pair of separate polypeptides, which are the paired DNA-binding protein domains.

In some exemplary embodiments, the amino acid sequences of the paired DNA-binding protein domain of the present disclosure are:
amino acid sequences set forth in SEQ ID NO: 1 and sequence Q;
amino acid sequences set forth in SEQ ID NO: 3 and SEQ ID NO: 4;
amino acid sequences set forth in SEQ ID NO: 5 and SEQ ID NO: 6;
amino acid sequences set forth in SEQ ID NO: 7 and SEQ ID NO: 8;
amino acid sequences set forth in SEQ ID NO: 9 and SEQ ID NO: 10;
amino acid sequences set forth in SEQ ID NO: 11 and SEQ ID NO: 12;
amino acid sequences set forth in SEQ ID NO: 13 and SEQ ID NO: 14;
amino acid sequences set forth in SEQ ID NO: 15 and SEQ ID NO: 16;
amino acid sequences set forth in SEQ ID NO: 17 and SEQ ID NO: 18;
amino acid sequences set forth in SEQ ID NO: 19 and SEQ ID NO: 20;
amino acid sequences set forth in SEQ ID NO: 21 and SEQ ID NO: 22;
amino acid sequences set forth in SEQ ID NO: 23 and SEQ ID NO: 24;
amino acid sequences set forth in SEQ ID NO: 25 and SEQ ID NO: 26;
amino acid sequences set forth in SEQ ID NO: 27 and SEQ ID NO: 28;
amino acid sequences set forth in SEQ ID NO: 207 and SEQ ID NO: 208;
amino acid sequences set forth in SEQ ID NO: 209 and SEQ ID NO: 210;
amino acid sequences set forth in SEQ ID NO: 211 and SEQ ID NO: 212;
amino acid sequences set forth in SEQ ID NO: 213 and SEQ ID NO: 214;
amino acid sequences set forth in SEQ ID NO: 215 and SEQ ID NO: 216; or
amino acid sequences set forth in SEQ ID NO: 217 and SEQ ID NO: 218.

In some exemplary embodiments, the amino acid sequence of the deaminase N-terminal linker domain according to the present disclosure is selected from SEQ ID NOs: 1, 3, 5, 7, 9, 11, 13, 15, 17, 19, 21, 23, 25, 27, 207, 209, 211, 213, 215, and 217; and the amino acid sequence of the deaminase C-terminal linker domain is selected from the sequence Q, and SEQ ID NOs: 4, 6, 8, 10, 12, 14, 16, 18, 20, 22, 24, 26, 28, 208, 210, 212, 214, 216, and 218.

In some embodiments, the base editing fusion protein comprises the following structure: [deaminase N-terminal linker domain]-[deaminase]-[deaminase C-terminal linker domain], wherein "-" is an optional linker, that is, the domains may be linked via an optional linker.

In some embodiments, the deaminase N-terminal linker domain and the deaminase C-terminal linker domain are independently composed of 1 or 2 single domains; and the base editing fusion protein comprises the following structure: [X₁]-[X₂]-[deaminase]-[X₃]-[X₄], wherein X₁₋₄ are single domains, and "-" is an optional linker, that is, the domains may be linked via an optional linker.

As used herein, "single domain" is a portion of the DNA-binding protein domain, and all single domains are assembled into a complete DNA-binding protein domain. The single domain does not contain a linker, but has linkers at both ends that link it to other single domains or functional regions (such as deaminase, NLS or UGI).

In some specific embodiments, the X₁₋₄ single domains are selected from the following combinations:
(1) X₁ is SEQ ID NO: 158, X₂ is SEQ ID NO: 159, X₃ is SEQ ID NO: 160, and X₄ is absent;
(2) X₁ is absent, X₂ is SEQ ID NO: 161, X₃ is SEQ ID NO: 162, and X₄ is SEQ ID NO: 163;
(3) X₁ is absent, X₂ is SEQ ID NO: 164, X₃ is SEQ ID NO: 165, and X₄ is SEQ ID NO: 166;
(4) X₁ is SEQ ID NO: 181, X₂ is SEQ ID NO: 182, X₃ is a sequence Q, and X₄ is absent;
(5) X₁ is absent, X₂ is SEQ ID NO: 183, X₃ is SEQ ID NO: 184, and X₄ is SEQ ID NO: 185;
(6) X₁ is absent, X₂ is SEQ ID NO: 186, X₃ is SEQ ID NO: 187, and X₄ is SEQ ID NO: 188;
(7) X₁ is SEQ ID NO: 189, X₂ is SEQ ID NO: 190, X₃ is SEQ ID NO: 191, and X₄ is absent;
(8) X₁ is SEQ ID NO: 192, X₂ is SEQ ID NO: 193, X₃ is SEQ ID NO: 194, and X₄ is absent;
(9) X₁ is SEQ ID NO: 195, X₂ is SEQ ID NO: 196, X₃ is SEQ ID NO: 197, and X₄ is absent; and
(10) X₁ is SEQ ID NO: 198, X₂ is SEQ ID NO: 199, X₃ is SEQ ID NO: 200, and X₄ is absent.

As used herein, "deaminase" refers to an enzyme that catalyzes a deamination reaction. In some embodiments, the deaminase is a cytidine deaminase or an adenosine deaminase, *i.e.*, an enzyme capable of removing an amino group from a cytidine or adenosine molecule.

In some embodiments, the cytidine deaminase is an APOBEC family deaminase, an SCP1.201 family deaminase or a homolog thereof; preferably, the APOBEC family deaminase is an AID deaminase, an APOBEC1 deaminase, an APOBEC2 deaminase, an APOBEC3A deaminase, an APOBEC3B deaminase, an APOBEC3C deaminase, an APOBEC3D deaminase, an APOBEC3F deaminase, an APOBEC3G deaminase, an APOBEC3H deaminase, or a variant thereof. The SCP1.201 family deaminase is an Sdd2 deaminase, an Sdd3 deaminase, an Sdd4 deaminase, an Sdd6 deaminase, an Sdd7 deaminase, an Sdd9 deaminase, an Sdd10 deaminase, an Sdd59 deaminase, a mini-Sdd3 deaminase, a mini-Sdd6 deaminase, a mini-Sdd7 deaminase, a mini-Sdd9 deaminase, or a variant thereof. In some embodiments, the cytidine deaminase domain comprises an amino acid sequence set forth in any one of SEQ ID NOs: 54-82, 127, and 128.

In some embodiments, the adenosine deaminase is QB34, TadA8e, TadA7.10, a TadA dimer, or a variant thereof, and optionally, the TadA dimer comprises TadA7.10 and wild-type TadA.

In some specific embodiments, the adenosine deaminase of the present disclosure is TadA8e, TadA7.10 or their respective mutants, for example, an adenosine deaminase comprising an amino acid sequence set forth in any one of SEQ ID NOs: 83-88, or other adenosine deaminases useful in the base editing systems described later in detail.

In some embodiments, the fusion protein further comprises a uracil DNA glycosylase inhibitor (UGI) domain and/or a nuclear localization sequence (NLS).

As used herein, "uracil DNA glycosylase inhibitor" or "UGI" refers to a protein capable of inhibiting a uracil-DNA glycosylase, which is a base excision repair enzyme. In some embodiments, the UGI domain comprises an amino acid sequence set forth in SEQ ID NO: 124.

As used herein, "nuclear localization sequence (NLS)" or "nuclear localization signal" may be used interchangeably to denote a domain of a protein (typically a short amino acid sequence) capable of interacting with a nuclear vector to have the protein transported into the cell nucleus. Generally, one or more NLSs in the fusion protein should have sufficient strength to drive the fusion protein to accumulate in the nucleus of a cell in an amount sufficient to achieve its base editing function. Generally, the strength of nuclear localization activity is determined by the number and location of NLSs of the fusion protein, one or more specific NLSs used, or a combination of these factors. In some embodiments of the present disclosure, the NLS of the fusion protein of the present disclosure may be located at the N-terminus and/or C-terminus. In some embodiments of the present disclosure, the NLS of the fusion protein of the present disclosure may be located between the adenosine deaminase domain, the cytidine deaminase domain, the DNA-binding protein domain and/or UGI. In some embodiments, the fusion protein includes about 1, 2, 3, 4, 5, 6, 7, 8, 9, 10 or more NLSs. In some embodiments, the fusion protein includes about 1, 2, 3, 4, 5, 6, 7, 8, 9, 10 or more NLSs at or near the N-terminus. In some embodiments, the fusion protein includes about 1, 2, 3, 4, 5, 6, 7, 8, 9, 10 or more NLSs at or near the C-terminus. In some embodiments, the polypeptide comprises a combination of these, such as one or more NLSs at the N-terminus and one or more NLSs at the C-terminus. When more than one NLS is present, each may be selected to be independent of the other NLSs. Generally, an NLS is composed of one or more short sequences of positively charged lysine or arginine exposed on the protein surface, but other types of NLSs are also known. Non-limiting examples of NLS include the amino acid sequences of SEQ ID NOs: 30-46.

As used herein, "linker" or "linker protein" refers a linker that links two molecules or moieties, such as two domains of a fusion protein. Typically, a linker is located between or flanks two groups, molecules or other moieties, and is linked thereto via a covalent bond, thereby connecting the two. In some embodiments, a linker may link different components or different parts of a base editing system. In some embodiments, a linker may link a DNA-specific binding component and a deamination component in a base editing fusion protein or base editing system. In some embodiments, the linker is an organic molecule, group, polymer, or chemical moiety. In some embodiments, the linker may be a polynucleotide. In some embodiments, the linker may be a DNA molecule. In some embodiments, the linker may be an RNA molecule. In some embodiments, the linker may comprise an aptamer capable of binding a ligand. Aptamer-derived riboswitches may be selected from theophylline riboswitches, thiamine pyrophosphate (TPP) riboswitches, adenosylcobalamin (AdoCbl) riboswitches, S-adenosylmethionine (SAM) riboswitches, SAH riboswitches, flavin mononucleotide (FMN) riboswitches, tetrahydrofolate riboswitches, lysine riboswitches, glycine riboswitches, purine riboswitches, GlmS riboswitches, or pre-queosine1 (PreQ1) riboswitches. In some embodiments, the linker may comprise an aptamer that binds to a polypeptide or protein domain, such as a polypeptide ligand. In some embodiments, the polypeptide ligand may be a K homology (KH) domain, an MS2 coat protein domain, a PP7 coat protein domain, an SfMu Com coat protein domain, a sterile α motif, a telomerase Ku-binding motif and Ku protein, a telomerase Sm7-binding motif and Sm7 protein, or an RNA recognition motif. In some embodiments, the polypeptide ligand may be part of a base editing fusion protein or a component of a base editor system. For example, a nucleobase editing component may comprise a deaminase domain and an RNA recognition motif. In some embodiments, the linker is an amino acid or amino acids (*e.g.*, peptide(s) or protein(s)). In some embodiments, the linker of the present disclosure is absent, or the linker comprises an amino acid sequence (GGGS)n (SEQ ID NO: 47), (GGGGS)n (SEQ ID NO: 48), (G)n, (EAAAK)n (SEQ ID NO: 49), (GGS)n, (SGGS)n (SEQ ID NO: 51), or SGSETPGTSESATPES (SEQ ID NO: 52), or an (XP)n motif, or a combination thereof, wherein n is independently an integer from 1 to 32, and wherein X is any amino acid.

In some embodiments, the linker comprises:
GGS,
GGSGGS (SEQ ID NO: 50),
GGSGGSGGSGGS (SEQ ID NO: 119),
GGSGGSGGSGGSGGS (SEQ ID NO: 120),
GGSGGSGGSGGSGGSGGS (SEQ ID NO: 121),
GGSGGSGGSGGSGGSGGSGG (SEQ ID NO: 2)
GGSGGSGGSGGSGGSGGSGGS (SEQ ID NO: 122),
GGSGGSGGSGGSGGSGGSGGSGGS (SEQ ID NO: 219),
SGGSSGGSSGSETPGTSESATPESSGGSSGGS (SEQ ID NO: 53),

SGGSSGGSSGSETPGTSESATPESSGGSSGGSSGGSSGGSSGSETPGTSESATPESSG
GSSGGS (SEQ ID NO: 146),

PGSPAGSPTSTEEGTSESATPESGPGTSTEPSEGSAPGSPAGSPTSTEEGTSTEPSEGS

APGTSTEPSEGSAPGTSESATPESGPGSEPATS (SEQ ID NO: 147).

In some embodiments, the domains of the base editing fusion protein are linked via an optional linker. In some embodiments, the linker is absent or has a length of 0, indicating that the domains of the fusion protein are directly linked. In some embodiments, the linker has a length of 0 to 500 or 1 to 500 amino acids. In some embodiments, the linker has a length of 0 to 100 amino acids. In some embodiments, the linker has a length of 1 to 100 amino acids. In some embodiments, the linker has a length of 3 to 100 amino acids, *e.g.*, a length of 3, 4, 5, 6, 7, 8, 9, 10, 11, 12, 13, 14, 15, 16, 17, 18, 19, 20, 20-30, 30-40, 40-50, 50-60, 60-70, 70-80, 80-90, or 90-100 amino acids. In some embodiments, the linker has a length of no less than 12 amino acids, *e.g.*, 12, 13, 14, 15, 16, 17, 18, 19, 20, 21, 24, 32, 64, 92, or 100.

In other embodiments, the linker has a length of 0 to 100 amino acids.

In some specific embodiments, the linker comprises an amino acid sequence (GGGS)n (SEQ ID NO: 47), (GGGGS)n (SEQ ID NO: 48), (G)n, (EAAAK)n (SEQ ID NO: 49), (GGS)n, (SGGS)n (SEQ ID NO: 51), or SGSETPGTSESATPES (SEQ ID NO: 52), or an (XP)n motif, or a combination thereof, wherein n is independently an integer from 1 to 32, and wherein X is any amino acid.

In some preferred embodiments, the linker has a length of 12 to 100 amino acids.

In some embodiments, the fusion protein may form a complex with a guide RNA and binds to a target DNA.

In some embodiments, the structure of the base editing fusion protein according to the present disclosure may be selected from:
NH2-[deaminase N-terminal linker domain]-[deaminase]-[deaminase C-terminal linker domain]-COOH;
NH2-[deaminase N-terminal linker domain]-[deaminase]-[deaminase C-terminal linker domain]-[NLS]-COOH;
NH2-[NLS]-[deaminase N-terminal linker domain]-[deaminase]-[deaminase C-terminal linker domain]-[NLS]-COOH;
NH2-[deaminase N-terminal linker domain]-[cytidine deaminase]-[deaminase C-terminal linker domain]-[UGI]-COOH;
NH2-[deaminase N-terminal linker domain]-[cytidine deaminase]-[deaminase C-terminal linker domain]-[UGI]-[NLS]-COOH;
NH2-[NLS]-[deaminase N-terminal linker domain]-[cytidine deaminase]-[deaminase C-terminal linker domain]-[UGI]-[NLS]-COOH;
NH2-[deaminase N-terminal linker domain]-[cytidine deaminase]-[deaminase C-terminal linker domain]-[UGI]-[UGI]-COOH;
NH2-[deaminase N-terminal linker domain]-[cytidine deaminase]-[deaminase C-terminal linker domain]-[UGI]-[UGI]-[NLS]-COOH; and
NH2-[NLS]-[deaminase N-terminal linker domain]-[cytidine deaminase]-[deaminase C-terminal linker domain]-[UGI]-[UGI]-[NLS]-COOH.

In some embodiments, the structure of the base editing fusion protein according to the present disclosure may be selected from:
NH2-[X₁]-[X₂]-[deaminase]-[X₃]-[X₄]-COOH;
NH2-[X₁]-[X₂]-[deaminase]-[X₃]-[X₄]-[NLS]-COOH;
NH2-[NLS]-[X₁]-[X₂]-[deaminase]-[X₃]-[X₄]-[NLS]-COOH;
NH2-[X₁]-[X₂]-[cytidine deaminase]-[X₃]-[X₄]-[UGI]-COOH;
NH2-[X₁]-[X₂]-[cytidine deaminase]-[X₃]-[X₄]-[UGI]-[NLS]-COOH;
NH2-[NLS]-[X₁]-[X₂]-[cytidine deaminase]-[X₃]-[X₄]-[UGI]-[NLS]-COOH;
NH2-[X₁]-[X₂]-[cytidine deaminase]-[X₃]-[X₄]-[UGI]-[UGI]-COOH;
NH2-[X₁]-[X₂]-[cytidine deaminase]-[X₃]-[X₄]-[UGI]-[UGI]-[NLS]-COOH; and
NH2-[NLS]-[X₁]-[X₂]-[cytidine deaminase]-[X₃]-[X₄]-[UGI]-[UGI]-[NLS]-COOH.

In some embodiments, the base editing fusion protein comprises one or more of the following sequences:
(i) an amino acid sequence set forth in any one of SEQ ID NOs: 89-92, 94, 96, 99, 101, 103, 105-115, 118, 179, and 201-206;
(ii) an amino acid sequence having at least 80%, 82%, 85%, 87%, 90%, 92%, 95%, 96%, 97%, 98% or 99% sequence identity to the amino acid sequence set forth in any one of SEQ ID NOs: 89-92, 94, 96, 99, 101, 103, 105-115, 118, 179, and 201-206;
(iii) an amino acid sequence obtained by adding, substituting, deleting or inserting one or more amino acid residues in the amino acid sequence set forth in any one of SEQ ID NOs: 89-92, 94, 96, 99, 101, 103, 105-115, 118, 179, and 201-206; or
(iv) an amino acid sequence encoded by a nucleotide sequence that hybridizes with a polynucleotide sequence encoding the amino acid sequence set forth in any one of SEQ ID NOs: 89-92, 94, 96, 99, 101, 103, 105-115, 118, 179, and 201-206 under stringency conditions, wherein the stringency conditions are moderate stringency conditions, moderate-high stringency conditions, high stringency conditions, or very high stringency conditions.

In some exemplary embodiments, the fusion protein comprises the following structure: [X₁]-[X₂]-[deaminase]-[X₃]-[X₄], and in some embodiments, X₁ is absent, X₂ is SEQ ID NO: 164, X₃ is SEQ ID NO: 165, and X₄ is SEQ ID NO: 166. In some embodiments, the deaminase is mini-Sdd9 or a variant thereof.

In some exemplary embodiments, the base editing fusion protein comprises one or more of the following sequences:
(i) an amino acid sequence set forth in SEQ ID NO: 111;
(ii) an amino acid sequence having at least 80%, 82%, 85%, 87%, 90%, 92%, 95%, 96%, 97%, 98% or 99% sequence identity to the amino acid sequence set forth in SEQ ID NO: 111;
(iii) an amino acid sequence obtained by adding, substituting, deleting or inserting one or more amino acid residues in the amino acid sequence set forth in SEQ ID NO: 111; or
(iv) an amino acid sequence encoded by a nucleotide sequence that hybridizes with a polynucleotide sequence encoding the amino acid sequence set forth in SEQ ID NO: 111 under stringency conditions, wherein the stringency conditions are moderate stringency conditions, moderate-high stringency conditions, high stringency conditions, or very high stringency conditions.

In the present disclosure, "moderate stringency conditions", "moderate-high stringency conditions", "high stringency conditions" or "very high stringency conditions" describe the conditions for hybridization and washing of nucleic acids. Guidance for conducting hybridization reactions can be found in John Wiley&Sons, "Current Protocols in Molecular Biology", N.Y. (1989), 6.3.1-6.3.6, which is incorporated herein by reference. This literature describes both aqueous and non-aqueous processes and either can be used. For example, specific hybridization conditions are as follows: (1) the low stringency hybridization conditions refer to hybridizing in 6× sodium chloride/sodium citrate (SSC) at about 45°C, and then washing twice in 0.2× SSC and 0.1% SDS at 50°C or higher (the washing temperature can be raised to 55°C in the case of the low stringency conditions); (2) the moderate stringency hybridization conditions refer to hybridizing in 6× SSC at about 45°C, and then washing one or more times in 0.2× SSC and 0.1% SDS at 60°C; (3) the high stringency hybridization conditions refer to hybridizing in 6× SSC at about 45°C, and then washing one or more times in 0.2× SSC and 0.1% SDS at 65°C, which is preferred; and (4) the very high stringency hybridization conditions refer to hybridizing in 0.5 M sodium phosphate and 7% SDS at 65°C, and then washing one or more times in 0.2× SSC and 1% SDS at 65°C.

### III. Base Editing System

In another aspect of the present disclosure, there is provided a base editing system, wherein the base editing system comprises at least one of the following components i) to v):
i) a base editing fusion protein, and a guide RNA;
ii) an expression construct comprising a nucleotide sequence encoding a base editing fusion protein, and a guide RNA;
iii) a base editing fusion protein, and an expression construct comprising a nucleotide sequence encoding a guide RNA;
iv) an expression construct comprising a nucleotide sequence encoding a base editing fusion protein, and an expression construct comprising a nucleotide sequence encoding a guide RNA; and
v) an expression construct comprising a nucleotide sequence encoding a base editing fusion protein and a nucleotide sequence encoding a guide RNA;

wherein the base editing fusion protein comprises a deaminase domain and paired DNA-binding protein domains having a DNA single-strand cleavage function; and
the paired DNA-binding protein domains having the DNA single-strand cleavage function comprise a deaminase N-terminal linker domain and a deaminase C-terminal linker domain that are linked via an optional linker to the N-terminus and the C-terminus of the deaminase domain, respectively.

In some embodiments, the base editing fusion protein as well as the deaminase, the DNA-binding protein, the single domain, the linker, the UGI, the nuclear localization sequence and the like are as described above in "**II. Base Editing Fusion Protein**".

In some embodiments, the deaminase N-terminal linker domain and the deaminase C-terminal linker domain are independently composed of 1 or 2 single domains, and the single domains are linked via an optional linker.

In some embodiments, the base editing fusion protein comprises the following structure: [X₁]-[X₂]-[deaminase]-[X₃]-[X₄], wherein X₁₋₄ are single domains, and "-" is an optional linker.

In some specific embodiments, the X₁₋₄ single domains are selected from the following combinations:
(1) X₁ is SEQ ID NO: 158, X₂ is SEQ ID NO: 159, X₃ is SEQ ID NO: 160, and X₄ is absent;
(2) X₁ is absent, X₂ is SEQ ID NO: 161, X₃ is SEQ ID NO: 162, and X₄ is SEQ ID NO: 163;
(3) X₁ is absent, X₂ is SEQ ID NO: 164, X₃ is SEQ ID NO: 165, and X₄ is SEQ ID NO: 166;
(4) X₁ is SEQ ID NO: 181, X₂ is SEQ ID NO: 182, X₃ is a sequence Q, and X₄ is absent;
(5) X₁ is absent, X₂ is SEQ ID NO: 183, X₃ is SEQ ID NO: 184, and X₄ is SEQ ID NO: 185;
(6) X₁ is absent, X₂ is SEQ ID NO: 186, X₃ is SEQ ID NO: 187, and X₄ is SEQ ID NO: 188;
(7) X₁ is SEQ ID NO: 189, X₂ is SEQ ID NO: 190, X₃ is SEQ ID NO: 191, and X₄ is absent;
(8) X₁ is SEQ ID NO: 192, X₂ is SEQ ID NO: 193, X₃ is SEQ ID NO: 194, and X₄ is absent;
(9) X₁ is SEQ ID NO: 195, X₂ is SEQ ID NO: 196, X₃ is SEQ ID NO: 197, and X₄ is absent; and
(10) X₁ is SEQ ID NO: 198, X₂ is SEQ ID NO: 199, X₃ is SEQ ID NO: 200, and X₄ is absent.

In some embodiments, the deaminase is a cytidine deaminase or an adenosine deaminase.

In some embodiments, the cytidine deaminase is an APOBEC family deaminase, an SCP1.201 family deaminase or a homolog thereof; preferably, the APOBEC family deaminase is an AID deaminase, an APOBEC1 deaminase, an APOBEC2 deaminase, an APOBEC3A deaminase, an APOBEC3B deaminase, an APOBEC3C deaminase, an APOBEC3D deaminase, an APOBEC3F deaminase, an APOBEC3G deaminase, an APOBEC3H deaminase, or a variant thereof. The SCP1.201 family deaminase is an Sdd2 deaminase, an Sdd3 deaminase, an Sdd4 deaminase, an Sdd6 deaminase, an Sdd7 deaminase, an Sdd9 deaminase, an Sdd10 deaminase, an Sdd59 deaminase, a mini-Sdd3 deaminase, a mini-Sdd6 deaminase, a mini-Sdd7 deaminase, a mini-Sdd9 deaminase, or a variant thereof.

In some embodiments, the adenosine deaminase is QB34, TadA8e, TadA7.10, a TadA dimer, or a variant thereof, and optionally, the TadA dimer comprises TadA7.10 and wild-type TadA.

In some specific embodiments, the homolog of the APOBEC family deaminase is TadA8e, TadA7.10 or their respective mutants derived from *Petromyzon marinus* cytidine deaminase 1 (pmCDA1).

In some embodiments, the linker has a length of 0 to 100 amino acids.

In some embodiments, the linker comprises an amino acid sequence (GGGS)n (SEQ ID NO: 47), (GGGGS)n (SEQ ID NO: 48), (G)n, (EAAAK)n (SEQ ID NO: 49), (GGS)n, (SGGS)n (SEQ ID NO: 51), or SGSETPGTSESATPES (SEQ ID NO: 52), or an (XP)n motif, or a combination thereof, wherein n is independently an integer from 1 to 32, and wherein X is any amino acid.

In some preferred embodiments, the linker has a length of 12 to 100 amino acids.

In some embodiments, the fusion protein may form a complex with a guide RNA and binds to a target DNA.

In some embodiments, the base editing system of the present disclosure further comprises a uracil DNA glycosylase inhibitor (UGI) or an expression construct expressing the uracil DNA glycosylase inhibitor (UGI). The uracil DNA glycosylase inhibitor/expression construct expressing the uracil DNA glycosylase inhibitor is present alone, or in combination with other components of the base editing system to form at least one fusion protein or expression construct of the fusion protein. In some embodiments, the fusion protein contains about 1, 2, 3, 4, 5, 6, 7, 8, 9, 10 or more UGIs.

In some embodiments, the fusion protein of the base editing system according to the present disclosure further comprises a nuclear localization sequence.

### IV. Polynucleotide

In another aspect of the present disclosure, there is provided a polynucleotide, wherein the polynucleotide encodes:
(a) the above-mentioned base editing fusion protein; or
(b) the above-mentioned base editing system.

The polynucleotide of the present disclosure may be in a DNA or RNA form. The DNA form includes cDNA, genomic DNA or synthetic DNA. DNA may be single-stranded or double-stranded. DNA may be a coding strand or a non-coding strand.

The polynucleotide encoding the base editing fusion protein or the base editing system according to the present disclosure includes: a coding sequence encoding only the base editing fusion protein or the base editing system; a coding sequence of the base editing fusion protein or the base editing system and various additional coding sequences; and a coding sequence (and optionally additional coding sequences) of the base editing fusion protein or the base editing system and a non-coding sequence.

"Polynucleotide encoding the base editing fusion protein or the base editing system" may be a polynucleotide containing the coding sequence encoding this base editing fusion protein or base editing system, or may be a polynucleotide further containing an additional coding sequence and/or a non-coding sequence.

### V. Expression Construct

In another aspect of the present disclosure, there is provided an expression construct, the expression construct comprises the above-mentioned polynucleotide.

In some embodiments, the expression construct is selected from expression constructs from viruses, bacteria, yeast, plants, and mammalian cells.

### VI. Host cell

In another aspect of the present disclosure, there is provided a host cell, comprising the above-mentioned base editing fusion protein, the above-mentioned base editing system, the above-mentioned polynucleotide, or the above-mentioned expression construct.

### VII. Kit

In another aspect of the present disclosure, there is provided a kit, comprising the above-mentioned base editing fusion protein, the above-mentioned base editing system, the above-mentioned polynucleotide, the above-mentioned expression construct, or the above-mentioned host cell.

### VIII. Pharmaceutical Composition

In another aspect of the present disclosure, there is provided a pharmaceutical composition, comprising the above-mentioned base editing fusion protein, the above-mentioned base editing system, the above-mentioned polynucleotide, the above-mentioned expression construct, or the above-mentioned host cell, and optionally, a pharmaceutically acceptable carrier.

The term "pharmaceutically acceptable carrier" refers to any inactive substance suitable for use in a formulation to deliver a base editing fusion protein, a base editing system, a polynucleotide, an expression construct, or a host cell. The carrier may be an anti-adhesion agent, a binder, a coating, a disintegrant, a filler or a diluent, a preservative (such as an antioxidant, an antibacterial agent or an antifungal agent), a sweetener, an absorption delayer, a wetting agent, an emulsifier, a buffering agent, and the like. Examples of suitable pharmaceutically acceptable carriers include water, ethanol, polyols (*e.g.*, glycerol, propylene glycol, and polyethylene glycol), dextrose, vegetable oils (*e.g.*, olive oil), saline, buffer solutions, buffered saline, and isotonic agents (such as sugars, polyols, sorbitol, and sodium chloride).

### IX. Method for Producing at Least One Genetically Modified Cell

In another aspect of the present disclosure, there is provided a method for producing at least one genetically modified cell, comprising introducing the base editing system of the present disclosure into the at least one cell to cause a substitution or insertion in a nucleic acid sequence within a target nucleotide region in the at least one cell.

In some embodiments, the nucleic acid sequence within the target nucleotide region is in the genome of an organism. In some embodiments, the organism is a prokaryote. In some embodiments, the prokaryote is bacteria. In some embodiments, the organism is a eukaryote. In some embodiments, the organism is a plant or a fungus. In some embodiments, the organism is a vertebrate. In some embodiments, the vertebrate is a mammal. In some embodiments, the mammal is a mouse, a rat, or human. In some embodiments, the organism is a cell. In some embodiments, the cell is a mouse cell, a rat cell, or a human cell. In some embodiments, the cell is a HEK-293 cell. In some embodiments, the cell is a HEK293T cell.

In some embodiments, the cell is derived from a prokaryote such as bacteria; or a eukaryote such as plants, fungi or vertebrates. In some embodiments, wherein the vertebrates are mammals such as human, mouse, rat, monkey, dog, pig, sheep, cattle, and cat; and the plants are crop plants, such as wheat, rice, corn, soybean, sunflower, sorghum, oilseed rape, alfalfa, cotton, barley, millet, sugar cane, tomato, tobacco, cassava or potato.

### X. Method for Producing at Least One Genetically Modified Plant

In another aspect, the present disclosure provides a method for producing a genetically modified plant, comprising introducing the base editing system of the present disclosure into at least one plant to cause one or more nucleotide substitutions in a nucleic acid sequence within a target nucleotide region in a genome of the at least one plant.

In some embodiments, the method further comprises screening the at least one plant for a plant with one or more desired nucleotide substitutions.

In the method of the present disclosure, the base editing composition may be introduced into a plant via various methods well-known to those skilled in the art. The methods usable to introduce the base editing system of the present disclosure into a plant include, but are not limited to: particle bombardment, PEG-mediated protoplast transformation, *Agrobacterium*-mediated transformation, plant virus-mediated transformation, pollen tube pathway, and ovary injection.

In the method of the present disclosure, the modification of the nucleic acid sequence within the target nucleotide region may be achieved by simply introducing or producing a complex of the base editing fusion protein and a guide RNA into a plant cell, and the modification may be stably inherited without the need of the stable transformation of the exogenous polynucleotide encoding the components of the base editing system into the plant. This avoids the potential off-target effects of the stably existing (continuously produced) base editing system, and also avoids the integration of the exogenous nucleotide sequence(s) in the plant genome, thereby exhibiting higher biological safety.

In some preferred embodiments, the introduction is performed in the absence of selection pressure, thereby avoiding the integration of the exogenous nucleotide sequence(s) in the plant genome.

In some embodiments, the introduction includes transforming the base editing system of the present disclosure into an isolated plant cell or tissue and then enabling the regeneration of the transformed plant cell or tissue into an intact plant. Preferably, the regeneration is performed in the absence of selection pressure, that is, any selective agent for the selector gene carried on the expression vector is not used during tissue culture. The regeneration efficiency of plant may be enhanced without the use of a selective agent, and a modified plant that does not comprise an exogenous nucleotide sequence is obtained.

In other embodiments, the base editing system of the present disclosure may be transformed into a specific part of an intact plant, such as leaf, stem tip, pollen tube, young ear, or hypocotyl. This is particularly suitable for the transformation of the plants that are difficult to regenerate by tissue culture.

In some embodiments of the present disclosure, the protein expressed *in vitro* and/or the RNA molecule transcribed *in vitro* (*e.g.* the expression construct is an RNA molecule transcribed *in vitro*) are directly transformed into the plant. The protein and/or RNA molecule are capable of achieving base editing in plant cells and subsequently degraded by cells, avoiding the integration of exogenous nucleotide sequence in the plant genome.

Therefore, in some embodiments, a plant whose genome is free of the integrated exogenous polynucleotide, *i.e.*, a transgene-free modified plant, may be obtained by conducting the genetic modification and breeding of plant using the method of the present disclosure.

In some embodiments of the present disclosure, the modified target nucleic acid region is associated with plant traits such as an agronomic trait. As a result, the one or more nucleotide substitutions result in the plant having altered (preferably, improved) traits such as an agronomic trait, as compared to the wild-type plant.

In some embodiments, the method further comprises a step of screening a plant having one or more nucleotide substitutions as desired and/or a desired trait such as an agronomic trait.

In some embodiments of the present disclosure, the method further comprises obtaining the progeny of the genetically modified plant. Preferably, the genetically modified plant or the progeny thereof has one or more nucleotide substitutions as desired and/or a desired trait such as an agronomic trait.

### XI. Genetically Modified Plants, Progeny Thereof or Parts Thereof

In another aspect, the present disclosure further provides a genetically modified plant, a progeny thereof or a part thereof, wherein the plant is obtained by the above-mentioned method for producing at least one genetically modified plant according to the present disclosure. In some embodiments, the genetically modified plant, the progeny thereof or the part thereof is non-transgenic. Preferably, the genetically modified plant or the progeny thereof has a desired genetic modification and/or a desired trait such as an agronomic trait.

In another aspect, the present disclosure further provides a method for plant breeding, comprising hybridizing a first genetically modified plant that comprises one or more nucleotide substitutions in the target nucleic acid region and is obtained by the method of the present disclosure with a second plant free of the one or more nucleotide substitutions, thereby introducing the one or more nucleotide substitutions into the second plant. Preferably, the first genetically modified plant has a desired trait such as an agronomic trait.

### XII. Use in Treatment of Diseases

The present disclosure further encompasses use of the base editing system of the present disclosure in treatment of a disease.

The up-regulation, down-regulation, inactivation, activation, mutation correction or the like of disease-related genes may be achieved by modifying the disease-related genes with the base editing system of the present disclosure, thereby realizing the prevention and/or treatment of diseases. For example, the target nucleotide region according to the present disclosure may be located in the protein coding region of a disease-related gene or, for example, may be located in a gene expression regulatory region such as a promoter region or an enhancer region, thereby enabling the functional modification of the disease-related gene or the modification of the expression of the disease-related gene. Therefore, the modifications of a disease-related gene described herein include not only the modifications of the disease-related gene itself (for example, the protein coding region), but also the modifications of its expression regulatory regions (such as a promoter, an enhancer, and an intron).

A "disease-related" gene refers to any gene that produces a transcription or translation product at an abnormal level or in an abnormal form in cells derived from a disease-affected tissue as compared to the non-disease control tissue or cell. In a case where the modified expression is associated with the occurrence and/or progression of a disease, it may be a gene expressed at an abnormally high level; and it may be a gene expressed at an abnormally low level. A disease-related gene also refers to a genetically mutated gene that has one or more mutations, or is directly responsible for the etiology of the disease or in linkage disequilibrium with one or more genes responsible for the etiology of the disease. The mutation or genetic variation is, for example, a single nucleotide variation (SNV). The transcription or translation product may be known or unknown, and may be at a normal or abnormal level.

Accordingly, the present disclosure further provides a method for treating a disease in a subject in need thereof, comprising delivering an effective amount of the base editing system of the present disclosure to the subject so as to modify a gene related to the disease (*e.g.*, deaminating the chondriosomal DNA with a fusion protein or fusion proteins). Provided is use of the base editing system in preparation of a pharmaceutical composition for treating a disease in a subject in need thereof, wherein the base editing system is used to modify a gene related to the disease. The present disclosure further provides a medicament for treating a disease in a subject in need thereof, comprising the base editing system of the present disclosure and optionally a pharmaceutically acceptable carrier, wherein the base editing system is used to modify a gene related to the disease.

In some embodiments, the fusion protein or base editing system described in the present disclosure is used to introduce a point mutation into a target nucleic acid by subjecting the target nucleobase (for example, C residue or A residue) in the target nucleic acid to deamination. In some embodiments, the mutation results in the correction of a genetic defect, for example, upon correcting a point mutation that leads to the loss of function in the genetic product. In some embodiments, the genetic defect is associated with a disease or condition (for example, lysosomal storage disease or a metabolic disease such as Type I diabetes). In some embodiments, the method provided herein may be used to introduce an inactivating point mutation into a gene or allele encoding a genetic product associated with the disease or condition.

In some embodiments, the embodiments described in the present disclosure are intended to restore the function of a dysfunctional gene via genome editing. The base editing fusion protein provided herein may be used for *in-vitro* gene editing of human cells, such as the correction of a disease-related mutation in a human cell culture.

In some embodiments, the embodiments described in the present disclosure are intended to treat a disease associated with or caused by a point mutation, and the point mutation may be corrected by the DNA base editing fusion protein provided herein. In some embodiments, the disease is a proliferative disease. In some embodiments, the disease is a genetic disease. In some embodiments, the disease is a *de novo* disease. In some embodiments, the disease is a metabolic disease. In some embodiments, the disease is lysosomal storage disease.

In some embodiments, the embodiments described in the present disclosure are intended to treat mitochondrial diseases or disorders. As used herein, a "mitochondrial disease" refers to a disease caused by abnormal mitochondria, for example, a mitochondrial gene mutation or a gene mutation in enzymatic pathway. Examples of the disease include, but are not limited to: neurological diseases, loss of motion control, muscle weakness and pain, gastrointestinal diseases and difficulty in swallowing, poor growth, heart diseases, liver diseases, diabetes, respiratory complications, epilepsy, vision/hearing problems, lactic acidosis, developmental retardation and susceptibility to infection.

Examples of the diseases according to the present disclosure include, but are not limited to, genetic diseases, circulatory system diseases, muscle diseases, diseases of brain and the central nervous and immune systems, Alzheimer's disease, secretase disorders, amyotrophic lateral sclerosis (ALS), autism, trinucleotide repeat expansion disorders, hearing diseases, gene-targeted therapy of non-dividing cells (neurons and muscle cells), liver and kidney diseases, diseases in epithelial cells and lung, cancer, Usher syndrome or retinitis pigmentosa, cystic fibrosis, HIV and AIDS, β-mediterranean anemia, sickle cell disease, herpes simplex virus, autistic, drug addiction, age-related macular degeneration, and schizophrenia. Other diseases that can be treated via the point mutation correction or the introduction of an inactivating mutation into a disease-related gene are also known to those skilled in the art, and therefore the present disclosure is not limited to the diseases listed herein. The strategies and the fusion proteins provided in the present disclosure may also be used for treatment of other related diseases, in addition to the diseases exemplarily described herein, and this application is apparent to those skilled in the art. Reference for diseases or targets applicable to the present disclosure can be found in the following documents, which describe the related diseases to which the base editing system is applicable: WO2015089465A1 (PCT/US2014/070135), WO2016205711A1 (PCT/US2016/038181), WO2018141835A1 (PCT/EP2018/052491), WO2020191234A1 (PCT/US2020/023713), WO2020191233A1 (PCT/US2020/023712), WO2019079347A1 (PCT/US2018/056146), and WO2021155065A1 (PCT/US2021/015580).

### Examples

### Example 1: Construction of QBE Base Editing System

### 1.1 Design of DNA-binding protein domain

In this example, the amino acid sequence of the DNA-binding protein was subjected to circular permutation. The circular permutation pattern of the DNA-binding protein was known in the art (Reference: Oakes, Benjamin L et al. "CRISPR-Cas9 Circular Permutants as Programmable Scaffolds for Genome Modification." Cell vol. 176, 1-2 (2019): 254-267.e16.). The linker for circularly permutation was selected as GGSGGSGGSGGSGGSGGSGG (SEQ ID NO: 2). Thus, a circularly permuted sequence set forth in SEQ ID NO: 29 having a length of 1388 amino acids was yielded (where the amino acid position of the circularly permuted sequence was defined in such a way that the methionine M encoded by a start codon was the first amino acid, and the glycine G directly linked to the first amino acid in the linker was the 1388^{th} amino acid).

The circularly permuted DNA-binding protein was cleaved at two sites to obtain a pair of separate polypeptides, which were the paired DNA-binding protein domains having a DNA single-strand cleavage function according to the present disclosure. The above paired DNA-binding protein domains having a DNA single-strand cleavage function were fused or linked to the N-terminus and C-terminus of the deaminase domain, respectively. Based on their positions at which they were fused to the deaminase, the domains linked to the N-terminus or C-terminus of the deaminase were defined as the deaminase N-terminal linker domain (QBE-N) and the deaminase C-terminal linker domain (QBE-C), respectively. As the paired deaminase terminal linker domains in this example, their amino acid sequences were shown in Table 2. In Table 2, the first cleavage site was defined as a deaminase linking site, where the lower-numbered amino acid was linked to the N-terminus of the deaminase and the higher-numbered amino acid was linked to the C-terminus of the deaminase; and the second cleavage site was defined as a non-deaminase linking site, where the lower-numbered amino acid was the C-terminus of the QBE-C domain and the higher-numbered amino acid was the N-terminus of the QBE-N domain.

**Table 1: Exemplary Paired Deaminase Terminal Linker Domains**

| Paired deaminase terminal linker domain | First cleavage site | Second cleavage site | Sequence of deaminase N-terminal linker domain (QBE-N) | Sequence of deaminase C-terminal linker domain (QBE-C) |
|---|---|---|---|---|
| QBE1 | 1026/1027 | 1040/1041 | SEQ ID NO: 3 | SEQ ID NO: 4 |
| QBE2 | 1057/1058 | 213/214 | SEQ ID NO: 17 | SEQ ID NO: 18 |
| QBE3 | 1244/1245 | 1030/1031 | SEQ ID NO: 7 | SEQ ID NO: 8 |
| QBE4 | 1026/1027 | 1027/1028 | SEQ ID NO: 1 | Sequence Q |
| QBE5 | 1244/1245 | 213/214 | SEQ ID NO: 5 | SEQ ID NO: 6 |
| QBE6 | 1244/1245 | 459/460 | SEQ ID NO: 9 | SEQ ID NO: 10 |
| QBE7 | 1244/1245 | 559/560 | SEQ ID NO: 11 | SEQ ID NO: 12 |
| QBE8 | 1244/1245 | 686/687 | SEQ ID NO: 13 | SEQ ID NO: 14 |
| QBE9 | 1244/1245 | 801/802 | SEQ ID NO: 15 | SEQ ID NO: 16 |
| QBE10 | 1057/1058 | 1030/1031 | SEQ ID NO: 19 | SEQ ID NO: 20 |
| QBE11 | 1057/1058 | 459/460 | SEQ ID NO: 21 | SEQ ID NO: 22 |
| QBE12 | 1057/1058 | 559/560 | SEQ ID NO: 23 | SEQ ID NO: 24 |
| QBE13 | 1057/1058 | 686/687 | SEQ ID NO: 25 | SEQ ID NO: 26 |
| QBE14 | 1057/1058 | 801/802 | SEQ ID NO: 27 | SEQ ID NO: 28 |

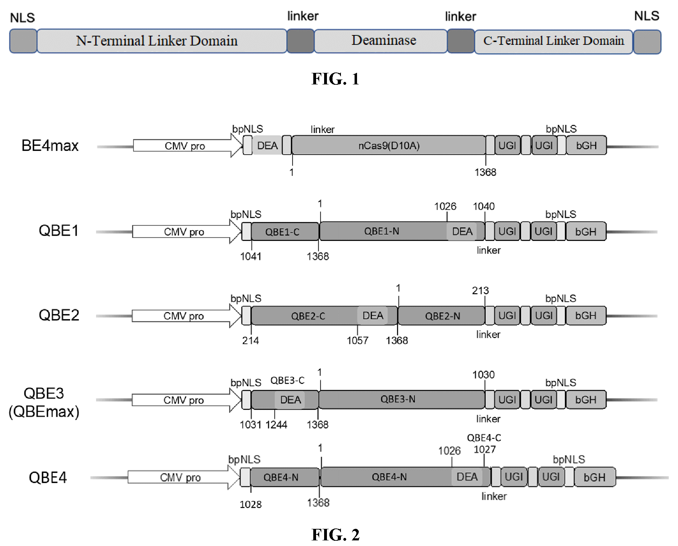

The base editing fusion protein formed by linking or fusing QBE-N, QBE-C to a deaminase domain comprised the following structure: [deaminase N-terminal linker domain]-[deaminase]-[deaminase C-terminal linker domain], where the domains might be linked directly or via a linker, and the linker was a linker sequence having a length of 0 to 32 amino acids. Alternatively, the fusion protein could also comprise a nuclear localization sequence (NLS), a uracil DNA glycosylase inhibitor (UGI), etc. FIG. 1 was a schematic diagram of an exemplary base editing fusion protein.

Furthermore, because QBE-N or QBE-C contained a circularly permuted linker of the DNA-binding protein and the domains containing a circularly permuted linker could also be represented as two single domains linked via a circularly permuted linker (as shown in FIG. 2), the structure of the base editing fusion protein could also be represented as follows: 1) when the circularly permuted linker was in the deaminase N-terminal linker domain, the resulting fusion protein comprised the following structure: [X₁]-[X₂]-[deaminase]-[X₃]; and 2) when the circularly permuted linker was in the deaminase C-terminal linker domain, the resulting fusion protein comprised the following structure: [X₂]-[deaminase]-[X₃]-[X₄]. In these structures, "[Xn]" represented a single domain that did not contain a linker, and "-" represented a linker. Therefore, according to the single domains, the paired deaminase terminal domains were as shown in Table 2.

**Table 2: Exemplary Paired Deaminase Terminal Linker Domains**

| Paired deaminase terminal linker domains | First cleavage site | Second cleavage site | [X₁] | [X₂] | [X₃] | [X₄] |
|---|---|---|---|---|---|---|
| QBE1 | 1026/1027 | 1040/1041 | SEQ ID NO: 158 | SEQ ID NO: 159 | SEQ ID NO: 160 | Absence |
| QBE2 | 1057/1058 | 213/214 | Absence | SEQ ID NO: 161 | SEQ ID NO: 162 | SEQ ID NO: 163 |
| QBE3 | 1244/1245 | 1030/1031 | Absence | SEQ ID NO: 164 | SEQ ID NO: 165 | SEQ ID NO: 166 |
| QBE4 | 1026/1027 | 1027/1028 | SEQ ID NO: 181 | SEQ ID NO: 182 | Sequence Q | Absence |

### 1.2 Construction of cytosine base editing system and adenine base editing system

Deaminases usable in the exemplary base editing systems according to the present disclosure included, but were not limited to, the deaminases listed as proteins set forth in SEQ ID NOs: 54-88. In this example, the cytidine deaminases rAPOBEC1, hAPOBEC3A (A3A), mini-Sdd3, mini-Sdd6, Sdd7, and mini-Sdd9 and the adenosine deaminase TadA-8e were exemplarily integrated into the sequences of the paired QBE deaminase terminal linker domains to obtain a series of novel cytosine base editors and adenine base editors. Exemplary base editing fusion proteins included, but were not limited to, those shown in Table 3. The base editing system was designated in the form of "deaminase-deaminase terminal linker domain" (*e.g.*, A3A-QBE1 was a base editing system composed of the A3A deaminase and the paired QBE1 deaminase terminal linker domains).

**Table 3: Exemplary Base editing fusion proteins**

| Base editing fusion protein | Deaminase terminal linker domain | Deaminase | Amino acid sequence of base editing system |
|---|---|---|---|
| rAPOBEC1-QBE1 | QBE1 | rAPOBEC1 | SEQ ID NO: 96 |
| A3A-QBE1 | QBE1 | A3A | SEQ ID NO: 103 |
| mini-Sdd3QBE1-QBE1 | QBE1 | mini-Sdd3 | SEQ ID NO: 99 |
| mini-Sdd6-QBE1 | QBE1 | mini-Sdd6 | SEQ ID NO: 101 |
| Sdd7-QBE1 | QBE1 | Sdd7 | SEQ ID NO: 94 |
| mini-Sdd9-QBE1 | QBE1 | mini-Sdd9 | SEQ ID NO: 105 |
| TadA-8e -QBE1 | QBE1 | TadA-8e | SEQ ID NO: 90 |
| rAPOBEC1-QBE2 | QBE2 | rAPOBEC1 | SEQ ID NO: 106 |
| A3A -QBE2 | QBE2 | A3A | SEQ ID NO: 112 |
| mini-Sdd3-QBE2 | QBE2 | mini-Sdd3 | SEQ ID NO: 108 |
| mini-Sdd6-QBE2 | QBE2 | mini-Sdd6 | SEQ ID NO: 114 |
| Sdd7-QBE2 | QBE2 | Sdd7 | SEQ ID NO: 118 |
| mini-Sdd9-QBE2 | QBE2 | mini-Sdd9 | SEQ ID NO: 110 |
| TadA-8e -QBE2 | QBE2 | TadA-8e | SEQ ID NO: 91 |
| rAPOBEC1-QBE3 | QBE3 | rAPOBEC1 | SEQ ID NO: 107 |
| A3A -QBE3 | QBE3 | A3A | SEQ ID NO: 113 |
| mini-Sdd3-QBE3 | QBE3 | mini-Sdd3 | SEQ ID NO: 109 |
| mini-Sdd6-QBE3 | QBE3 | mini-Sdd6 | SEQ ID NO: 115 |
| Sdd7-QBE3 | QBE3 | Sdd7 | SEQ ID NO: 179 |
| mini-Sdd9-QBE3 | QBE3 | mini-Sdd9 | SEQ ID NO: 111 |
| TadA-8e -QBE3 | QBE3 | TadA-8e | SEQ ID NO: 92 |
| TadA-8e -QBE4 | QBE4 | TadA-8e | SEQ ID NO: 89 |

### Example 2: Editing Efficiency of QBE Base Editing System

To detect the editing efficiency of the QBE base editing system according to the present disclosure, this example designed a sgRNA, in which the scaffold sequence was selected as:
guuuuagagcuagaaauagcaaguuaaaauaaggcuaguccguuaucaacuugaaaaaguggcaccgagucgg ugc (SEQ ID NO: 151). The spacer of this sgRNA corresponded to the protospacer (GACAAACCAGAAGCCGCTCC; SEQ ID NO: 129) at the target site1 of the *CTNNB1* gene and the protospacer (GAACACAAAGCATAGACTGC; SEQ ID NO: 131) at the target site3 of the VISTA enhancer hs267 in the HEK293T cell genome. The fourth-generation base editing system, BE4max (Reference: Komor, Alexis C et al. "Improved base excision repair inhibition and bacteriophage Mu Gam protein yields C:G-to-T:A base editors with higher efficiency and product purity." Science advances vol. 3,8 eaao4774. 30 Aug (2017)) fused with a deaminase shown in Table 3 was selected as a control (SEQ ID NOs: 125-126, SEQ ID NOs: 152-153, SEQ ID NOs: 155-156, SEQ ID NOs: 180). The schematic diagram of the structure of the base editing fusion protein used in this example was as shown in FIG. 2. The coding plasmids encoding the base editing fusion protein and sgRNA were co-transfected into HEK293T cells. The genomic DNA was extracted from the cells 72 hours after transfection. The target fragment was amplified by two rounds of PCR and the products were purified. The base editing efficiency at the target sites was detected by next-generation sequencing (NGS).

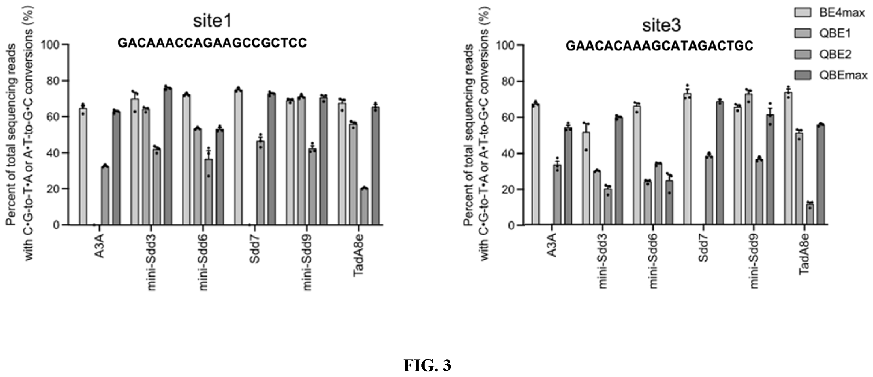

FIG. 3 showed the statistics on the editing capabilities and editing efficiency of different base editing systems at target sites. As shown in FIG. 3, when QBE1 was linked to mini-Sdd3, mini-Sdd6, mini-Sdd9, and TadA-8e deaminases, editing of specific bases could occur at both site1 and site3, and when QBE1 was linked to the mini-Sdd9 cytidine deaminase, the resulting base editing system exhibited slightly higher editing efficiency as compared to mini-Sdd9-BE4max as a control, and its editing efficiency at site3 reached 74%. All the base editing systems obtained by linking QBE2 to all the six deaminases enabled editing of specific bases at both target site1 and site3, and the highest editing efficiency (37%) was achieved when QBE2 was linked to the mini-Sdd9 cytidine deaminase. All the base editing systems obtained by linking QBE3 to the deaminases exhibited a better base editing capacity, and comparable or slightly higher editing efficiency compared to the control BE4max, and the highest efficiency (70%) was achieved when QBE3 was linked to the Sdd7 cytidine deaminase.

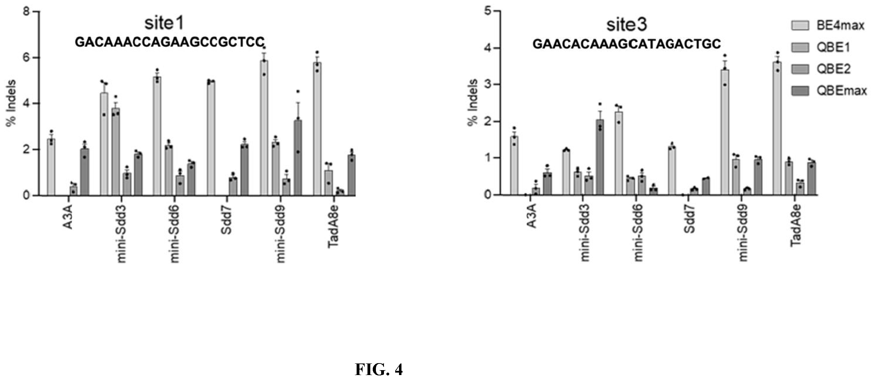

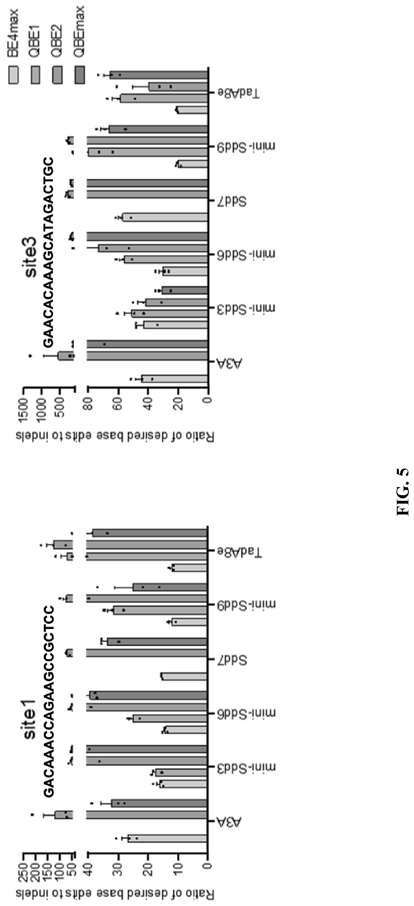

When directed mutations were performed on specific bases (such as A-T and C-G), indel was undesirable as it reduced the accuracy of base editing. In this example, the random insertion/deletion (indel) efficiency of each base editing system was analyzed. FIG. 4 showed the indel efficiency of each base editing system, and FIG. 5 showed the editing efficiency/indel ratio. The higher this ratio was, the higher editing efficiency and the lower indel efficiency this base editing system achieved, that is, a better base editing effect was achieved. The indel efficiency results in FIG. 4 indicated that the indel efficiency of the QBE base editing systems was generally lower than that of the BE4max system. As shown in FIG. 5, the editing efficiency/indel ratio of BE4max was about 10 to 60, and the editing efficiency/indel ratios of the QBE base editing systems were significantly improved as compared to the BE4max control group.

In summary, the above-mentioned QBE base editing systems linked to different types of deaminases enabled editing of specific bases (C-to-T; A-to-G). Their base editing efficiency was similar to or significantly improved as compared to the control group BE4max, for example, mini-Sdd9-QBE1 and mini-Sdd3-QBE3. The base editing systems exhibited significantly reduced indel and significantly improved editing efficiency/indel ratio, indicating that their editing accuracy was significantly improved and the overall effect of the base editing systems was significantly improved. Among them, the QBE3 deaminase terminal linker domain exhibited a better editing effect. Hereinbelow, a base editing fusion protein having the QBE3 fusion protein structure was selected as a representative for further comparison and analysis, which was designated as QBEmax.

### Example 3: Purity of Base Editing Product

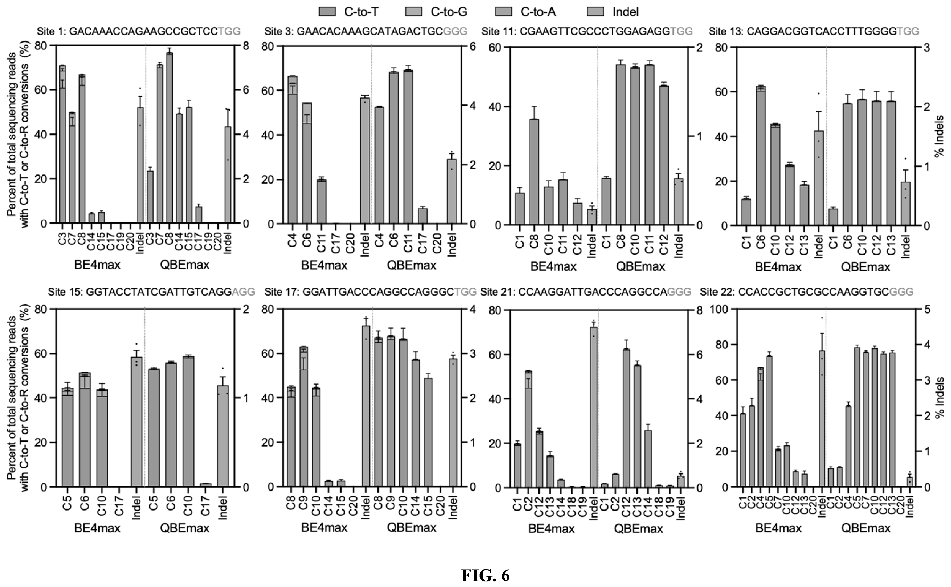

Studies had shown that the product purity of base editing systems was not high. For example, in addition to C-to-T (the intended editing type), CBE might also yield C-to-R (impure, non-intended editing types, including C-to-A and C-to-G), resulting in gene editing byproducts. The BE4 system developed by the researchers not only exhibited high editing efficiency but also reduced the occurrence of C-to-R by 2.3-fold compared to BE3 (Reference: Komor, Alexis C et al. "Improved base excision repair inhibition and bacteriophage Mu Gam protein yields C:G-to-T:A base editors with higher efficiency and product purity." Science advances vol. 3, 8eaao4774. 30 Aug (2017).). To verify the product purity of the cytosine base editing yielded by the QBE base editing systems of the present disclosure, the base editing results of mini-Sdd9-QBEmax and mini-Sdd9-BE4max within the editing windows at eight target sites were statistically analyzed in this example, and the proportions of C-to-T, C-to-A, and C-to-G occurring within each editing window were calculated to work out the product purity of the base editing systems. The results were shown in FIG. 6. The mini-Sdd9-QBEmax exhibited editing purity of 99.4% ± 0.4% at the cytosine base positions within the editing windows, which was significantly higher than the editing purity of 95.5% ± 2.8% exhibited by mini-Sdd9-BE4max.

Therefore, the QBE base editing system of the present disclosure could effectively enhance the product purity of gene editing and reduce gene editing byproducts.

### Example 4: Base Editing Window

The editing windows varied slightly among different types of base editing systems. Wider editing windows could cover more potential editing sites to thereby expand the applications of base editors. In this example, the editing windows of QBE base editing systems of different types were analyzed.

### 4.1 Editing Window of Adenine Base Editor

In this example, sgRNAs targeting adenosine- and cytidine-rich editing sites-site1 and site3 (protospacer sequences set forth in SEQ ID NOs: 129-131) in the HEK293T cell genome were designed. ABE8e fused to Cas9, TadA-8e was selected as a control (SEQ ID NO: 123). Coding plasmids encoding novel base editing systems TadA8e-QBE1, TadA8e-QBE2, TadA8e-QBE3, and TadA8e-QBE4, in which TadA-8e was used as a deaminase, were constructed according to the method described in Example 2.

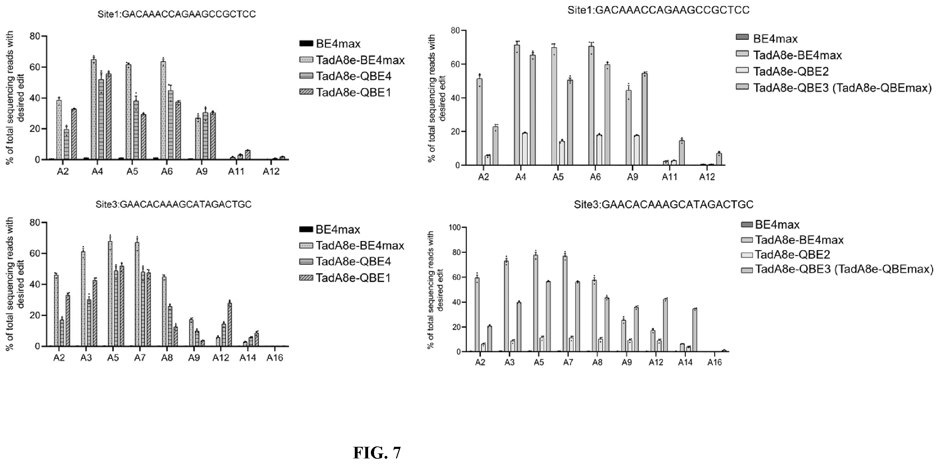

The editing windows of different adenine base editing systems were analyzed, and the first base distal to the PAM in the target sequence was defined as 1. The results showed that for the target site1, the editing window of ABE8e, TadA8e-QBE4, TadA8e-QBE1 or TadA8e-QBE3 included A2, A4, A5, A6, and A9, and the base editing systems of the present disclosure also exhibited an editing capability at A11 and A12 distal to the PAM. For the target site3, the editing windows of ABE8e, TadA8e-QBE4, and TadA8e-QBE1 all included A2, A3, A5, A7, A8, A9, A12, and A14, and the highest editing efficiency occurred at A5. Of note, TadA8e-QBE4, TadA8e-QBE1 or TadA8e-QBE3 exhibited higher editing efficiency at sites A12 and A14 distal to the PAM, and editing was still detectable at site A16 for TadA8e-QBE3 (as shown in FIG. 7). Thus, the base editing systems of the present disclosure further expanded the base editing windows of the adenine base editing systems.

### 4.2 Editing Window of Cytosine Base Editor (CBE)

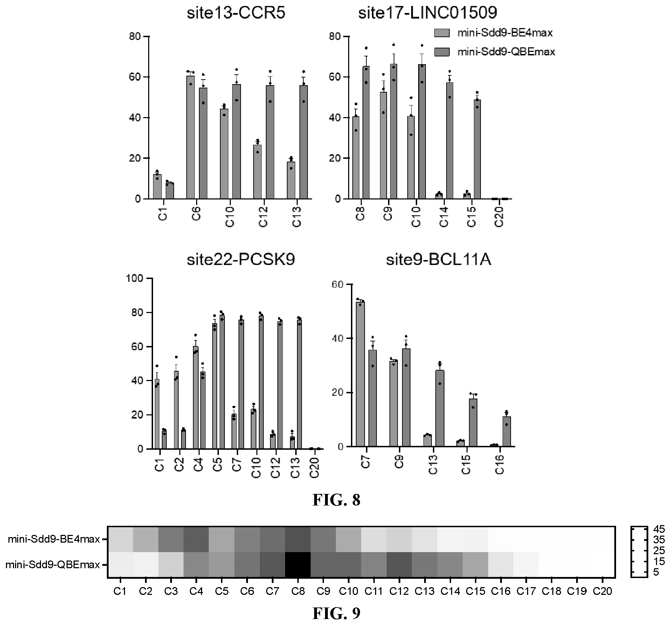

In this example, the mini-Sdd9 cytidine deaminase was exemplarily linked to QBEmax to construct a mini-Sdd9-QBEmax cytosine base editor. The mini-Sdd9-QBEmax was targeted to 17 endogenous target sites (the protospacer sequences corresponding to sgRNAs were set forth in SEQ ID NOs: 129-145) in the HEK293T cell line, and the mini-Sdd9-BE4max was used as a control for comparison of editing windows. FIG. 6 and FIG. 8 were diagrams showing comparisons of the editing windows for some endogenous target sites. FIG. 9 was a grayscale diagram of the distribution of editing windows for all 17 endogenous target sites, where the darker the grayscale was, the higher the editing frequency occurred at that site.

The editing results of four endogenous target sites shown in FIG. 8 were taken as an example. For the target site13, the editing efficiency of mini-Sdd9-BE4max at positions C10, C12, and C13 gradually decreased, demonstrating that these positions had been at the edge of the editing window of mini-Sdd9-BE4max, whereas mini-Sdd9-QBEmax exhibited high editing levels at all of positions C6 to C13. For the target site22, it could be more clearly observed that while mini-Sdd9-BE4max exhibited high editing efficiency at C1 to C5, mini-Sdd9-QBEmax showed stable and high-level editing efficiency at positions C5 to C13 distal to the PAM, whereas the editing efficiency of mini-Sdd9-BE4max at these positions gradually decreased, and even decreased to about 10% at positions C12 and C13. FIG. 9 showed the distribution of editing windows for the 17 endogenous target sites. It could be clearly observed that mini-Sdd9-BE4max exhibited better editing capabilities at C3 to C8, namely, its editing window was located between C3 and C8. In contrast, mini-Sdd9-QBEmax exhibited better editing capabilities at C6 to C15, and its editing window was shifted toward the region distal to the PAM, while the editing window was expanded, covering more editable sites.

In summary, the editing window of mini-Sdd9-QBEmax was expanded and also shifted toward the region distal to the PAM. This not only broadened the editing range but also provided greater flexibility for the editing window, freeing it from the constraint of existing cytosine editors that could only edit cytosines near the PAM.

### Example 5: Structure-Based Mechanism Analysis

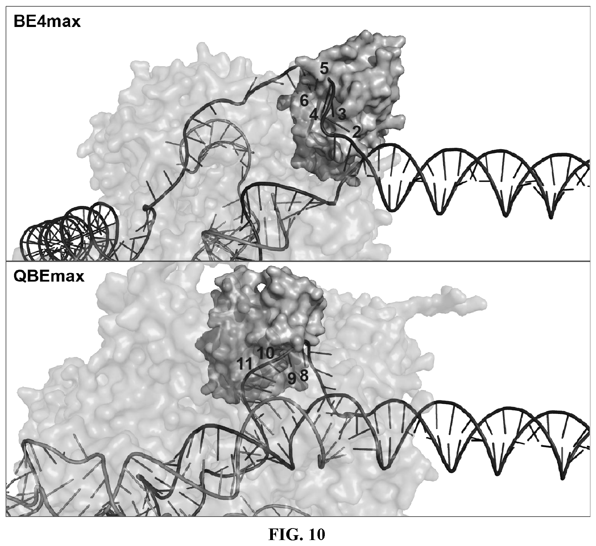

In this example, AlphaFold3 was used to predict the structure of the ternary complex of a base editor (BE4max and QBEmax), sgRNA, and DNA (Abramson, Josh et al. "Accurate structure prediction of biomolecular interactions with AlphaFold 3." Nature vol. 630, 8016 (2024): 493-500.). The structure prediction results (FIG. 10) showed that the deaminase domain of QBEmax of the present disclosure exhibited closer binding to nucleotides proximal to the PAM nick, whereas the deaminase domain of BE4max was more inclined to approach nucleotides distal to the PAM nick. This structural prediction was consistent with the experimental results.

### Example 6: Effects of Circular Permutation and Deaminase Terminal Fusion on QBE Base Editing System

**To** verify the effects of circular permutation of the DNA-binding protein and fusion of the paired DNA-binding protein domains to both termini of the deaminase on the editing effect of the final base editing system, two control groups were designed in this example. Control Group 1): An inlaid base editing system in which the deaminase was directly inlaid into a DNA-binding protein that had not been circularly permutated. Specifically, in the above-mentioned inlaid base editing fusion protein, the deaminase domain was directly inlaid into the DNA-binding protein, which was not circularly permutated, at the position 1244/1245 of the DNA-binding protein, where the amino acid at position 1244 was linked to the N-terminus of the deaminase, and the amino acid at position 1245 was linked to the C-terminus of the deaminase. This fusion protein was designated as CBE-Inlaid1244.

Control Group 2): A base editing system in which circularly permuted and single-nicked polypeptide was used as a new DNA-binding protein domain and fused to the terminus of the deaminase. Specifically, the DNA-binding protein was subjected to circular permutation according to the method described in Example 1, and the circularly permuted sequence was cleaved at 1030/1031. The amino acid at position 1031 was used as the N-terminus of the permuted DNA-binding protein domain and the amino acid at position 1030 was used as its C-terminus. The DNA-binding protein domain of BE4max was replaced with the permuted DNA-binding protein domain to obtain a new fusion protein, which was designated as CBE-CP1031.

In this example, mini-Sdd9 was selected as the deaminase, and the editing effects of mini-Sdd9-BE4max, mini-Sdd9-QBEmax, mini-Sdd9-CBE-CP1031, and mini-Sdd9-CBE-Inlaid1244 at site1 and site3 were determined. The experimental and detection methods were the same as those in Example 2.

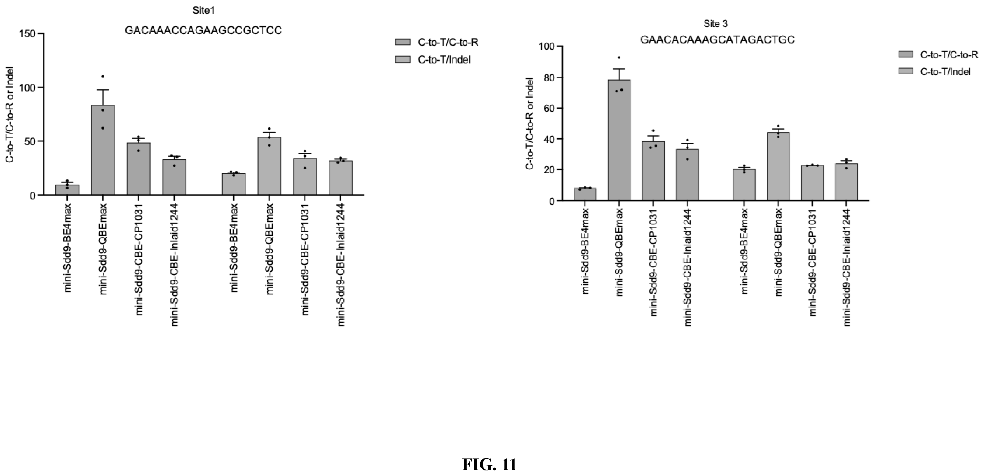

FIG. 11 showed the results of the ratios of C-to-T (the intended editing type) to C-to-R (impure, non-intended editing types, including C-to-A and C-to-G) and the ratios of C-to-T to Indel. The results showed that both the C-to-T/C-to-R ratio and the C-to-T/Indel ratio of QBEmax were higher than those of the other three base editors.

The above results indicated that QBEmax yielded fewer Indel byproducts and achieved higher product purity of C-to-T, while realizing efficient target base editing. Therefore, QBEmax provided higher accuracy and safety, and was of significant value in applications of base editing.

### Example 7: Effect of Circular Linker on Base Editing Efficiency

To verify the effect of linker length on the QBE base editing system, the circularly permuted linker GGSGGSGGSGGSGGSGGSGG (SEQ ID NO: 2) in QBE was replaced with circularly permuted linkers (CP linkers) of different lengths for experiment in this example. The same target sites as those used in Example 2 were tested in this example. The editing system was selected as QBEmax, and the deaminase used was mini-Sdd9. The sequences of the CP linkers for replacement were as shown in the table below.

**Table 4:**

| **Length** | **Sequence** | Linker SEQ ID NO: | Base editing system SEQ ID NO: |
|---|---|---|---|
| 3aa | GGS | / | 168 |
| 6aa | GGSGGS | 50 | 169 |
| 12aa | GGSGGSGGSGGS | 119 | 170 |
| 15aa | GGSGGSGGSGGSGGS | 120 | 171 |
| 18aa | GGSGGSGGSGGSGGSGGS | 121 | 172 |
| 20aa | GGSGGSGGSGGSGGSGGSGG | 2 | 111 |
| 21aa | GGSGGSGGSGGSGGSGGSGGS | 122 | 173 |
| 32aa | SGGSSGGSSGSETPGTSESATPESSGGSSGGS | 53 | 174 |
| 64aa | SGGSSGGSSGSETPGTSESATPESSGGSSGGSSGG SSGGSSGSETPGTSESATPESSGGSSGGS | 146 | 175 |

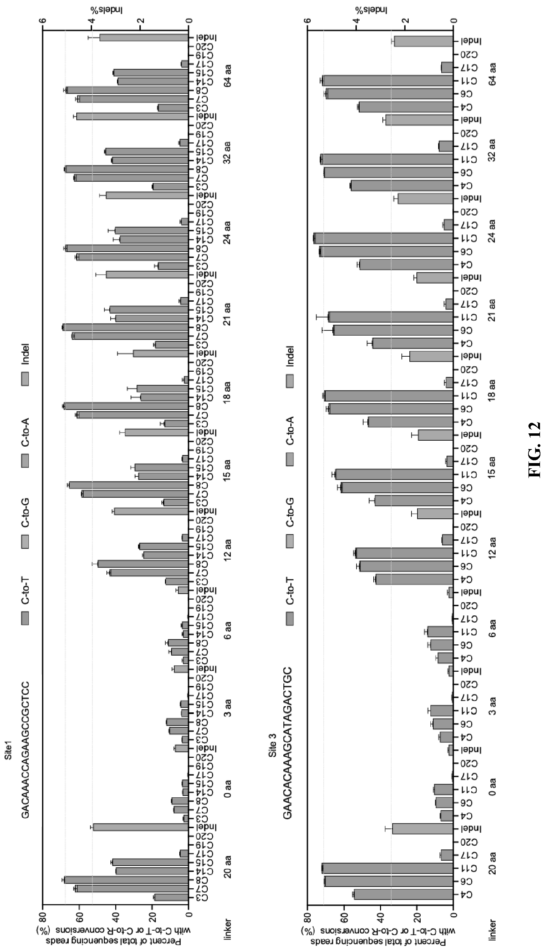

The results suggested that as shown in FIG. 12, the effects of different CP linker lengths on QBEmax were tested at two endogenous targets, site 1 and site 3. The results showed that the editing efficiency of the QBE base editing system increased in an S-shaped curve over the length of the CP linker. When the length of the CP linker was no less than 12 aa, high editing efficiency was consistently achieved. When the length of the CP linker was between 15 aa and 64 aa, there were no significant changes in editing efficiency, editing product purity, Indel frequency, etc. as compared to QBEmax containing the original CP linker of 20 aa.

In summary, when the length of the circularly permuted linker was less than 6 aa, this might lead to low stability of the QBE fusion protein and thus relatively low efficiency of the base editor comprising such a linker. However, when the linker length was no less than 12 aa, the stability of the fusion protein was less affected, and the base editor comprising such a linker could maintain a relatively high level of editing efficiency.

### Example 8: Applications of QBE Base Editor in Plant Cells

To detect the editing effect of the QBE base editing system of the present disclosure in plant cells, a sgRNA was designed in this example. This sgRNA was targeted to the target rice site1 (GACCTCCACGCCGCCGGCAATGG; SEQ ID NO: 148, where the underlined part represented the PAM sequence and the remaining represented the protospacer sequence) of the OsEPSPS promoter, the target rice site2 (GAGTGCGCGGCCATGGCGAGAGG; SEQ ID NO: 149, where the underlined part represented the PAM sequence and the remaining represented the protospacer sequence) of the OsTKL promoter, and the target rice site 3 (TCAGCAGGAATCTACCTTTGTGG; SEQ ID NO: 150, where the underlined part represented the PAM sequence and the remaining represented the protospacer sequence) of the OsEPSPS promoter in rice protoplast cells. The fourth-generation base editing system BE4max was selected as a control. 5 µg of QBEmax or BE4max and 5 µg of the corresponding sgRNA were mixed and the plasmid mixture was transformed into rice protoplast cells using PEG4000. The genomic DNA was extracted from the cells 48 hours after transformation. The base editing effects at the target sites were detected by next-generation sequencing (NGS).

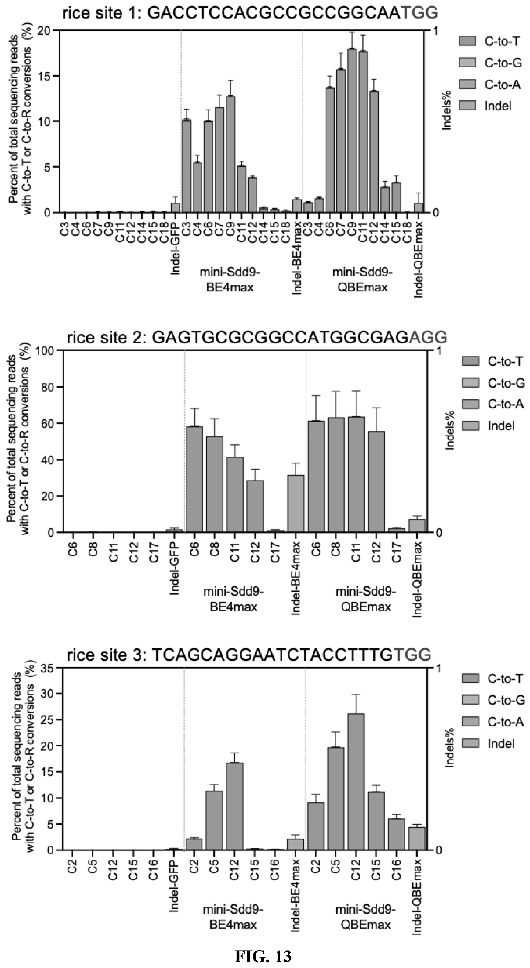

The experimental results in FIG. 13 showed that the QBEmax system also exhibited a good gene editing effect in rice protoplasts, and had a widened editing window, improved product purity, and a lower indel frequency as compared to BE4max.

### Example 9: Other QBE Base Editing Systems

To further expand the types of QBE editing system, multiple QBE base editing systems were constructed and validated in this example. Construction of the deaminase terminal linker domains of the base editing systems and the sequences of their single domains were shown in Table 5. BE4max and QBEmax were used as the control groups, and mini-Sdd9 was selected as an exemplary deaminase to form cytosine base editing systems (SEQ ID NOs: 201-206). The editing effects of the above-mentioned base editing systems at site1 and site3 were detected. The experimental methods and detection procedures were the same as those described in Examples 2 to 4.

**Table 5:**

| Paired deaminase terminal linker domain | First cleavage site | Second cleavage site | [X₁] | [X₂] | [X₃] | [X₄] |
|---|---|---|---|---|---|---|
| QBE84 | 1026/1027 | 213/214 | Absence | SEQ ID NO: 183 | SEQ ID NO: 184 | SEQ ID NO: 185 |
| QBE85 | 1244/1245 | 231/232 | Absence | SEQ ID NO: 186 | SEQ ID NO: 187 | SEQ ID NO: 188 |
| QBE87 | 202/203 | 1030/1031 | SEQ ID NO: 189 | SEQ ID NO: 190 | SEQ ID NO: 191 | Absence |
| QBE88 | 202/203 | 1149/1150 | SEQ ID NO: 192 | SEQ ID NO: 193 | SEQ ID NO: 194 | Absence |
| QBE89 | 468/469 | 1030/1031 | SEQ ID NO: 195 | SEQ ID NO: 196 | SEQ ID NO: 197 | Absence |
| QBE90 | 468/469 | 1149/1150 | SEQ ID NO: 198 | SEQ ID NO: 199 | SEQ ID NO: 200 | Absence |

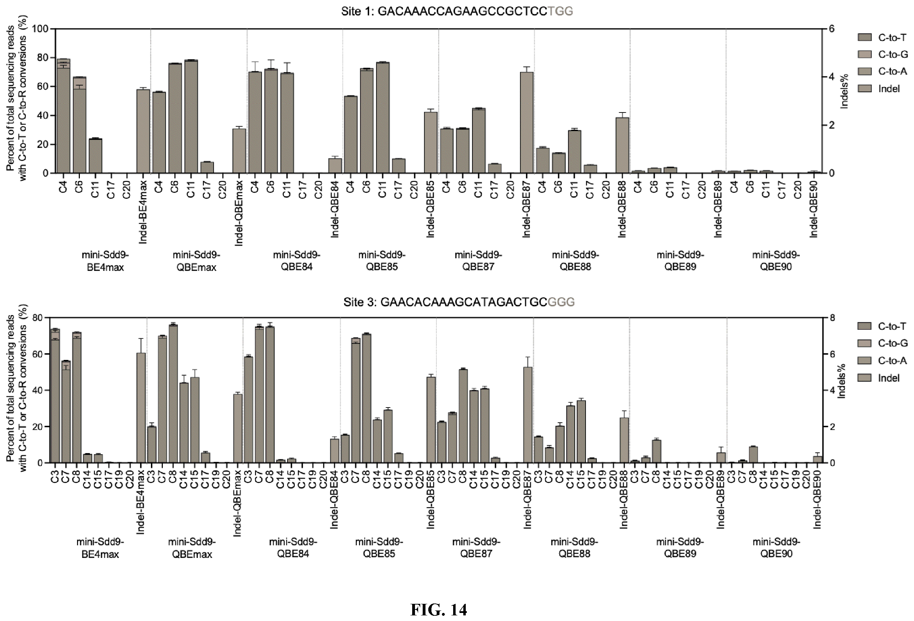

The experimental results were as shown in FIG. 14. Compared with BE4max, the product purity of some new QBE base editing systems was significantly improved. Among them, QBE84 and QBE85 achieved improved product purity and decreased Indel, while maintaining high editing efficiency. In terms of editing windows, the editing window of QBE84 was similar to that of BE4max, while the editing windows of QBE85, QBE87, and QBE88 were somewhat broadened. As a result, a series of new QBE base editing systems were yielded in this example, and the types and application scenarios of base editing tools were broadened.

While the present disclosure has been described with reference to specific embodiments herein, those skilled in the art will appreciate that various variations and equivalent substitutions may be made without departing from the true spirits and scopes of the present disclosure. In addition, various modifications may be made to adapt a specific circumstance, material, composition of substance, process, process step or steps to the purposes, spirits and scopes of the present disclosure. All such modifications are intended to be within the scope of the appended claims.

The nucleotide or amino acid sequences involved in the present disclosure are as follows:
>SEQ ID NO:1 QBE4-N 
EIGKATAKYFFYSNIMNFFKTEITLANGEIRKRPLIETNGETGEIVWDKGRDFATVR
KVLSMPQVNIVKKTEVQTGGFSKESILPKRNSDKLIARKKDWDPKKYGGFDSPTVAYS
VLVVAKVEKGKSKKLKSVKELLGITIMERSSFEKNPIDFLEAKGYKEVKKDLIIKLPKYS
LFELENGRKRMLASAGELQKGNELALPSKYVNFLYLASHYEKLKGSPEDNEQKQLFV
EQHKHYLDEIIEQISEFSKRVILADANLDKVLSAYNKHRDKPIREQAENIIHLFTLTNLGA
PAAFKYFDTTIDRKRYTSTKEVLDATLIHQSITGLYETRIDLSQLGGDGGSGGSGGS
GGSGGSGGMDKKYSIGLAIGTNSVGWAVITDEYKVPSKKFKVLGNTDRHSIKKNLIGA
LLFDSGETAEATRLKRTARRRYTRRKNRICYLQEIFSNEMAKVDDSFFHRLEESFLVEED
KKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQL
PGEKKNGLFGNLIALSLGLTPNFKSNFDLAEDAKLQLSKDTYDDDLDNLLAQIGDQYA
DLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTF
DNGSIPHQIHLGELHAILRRQEDFYPFLKDNREKIEKILTFRIPYYVGPLARGNSRFAWM
TRKSEETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHL
FDDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKPE
NIVIEMARENQTTQKGQKNSRERMKRIEEGIKELGSQILKEHPVENTQLQNEKLYLYYL
QNGRDMYVDQELDINRLSDYDVDHIVPQSFLKDDSIDNKVLTRSDKNRGKSDNVPSEE
VVKKMKNYWRQLLNAKLITQRKFDNLTKAERGGLSELDKAGFIKRQLVETRQITKHV
AQILDSRMNTKYDENDKLIREVKVITLKSKLVSDFRKDFQFYKVREINNYHHAHDAYL
NAVVGTALIKKYPKLESEFVYGDYKVYDVRKMIAKSE
> QBE4-C
   Q
> SEQ ID NO:2 Linker
   GGSGGSGGSGGSGGSGGSGG
>SEQ ID NO:3 QBE1-N 
NIMNFFKTEITLANGEIRKRPLIETNGETGEIVWDKGRDFATVRKVLSMPQVNIVK
KTEVQTGGFSKESILPKRNSDKLIARKKDWDPKKYGGFDSPTVAYSVLVVAKVEKGKS
KKLKSVKELLGITIMERSSFEKNPIDFLEAKGYKEVKKDLIIKLPKYSLFELENGRKRML
ASAGELQKGNELALPSKYVNFLYLASHYEKLKGSPEDNEQKQLFVEQHKHYLDEIIEQI
SEFSKRVILADANLDKVLSAYNKHRDKPIREQAENIIHLFTLTNLGAPAAFKYFDTTIDR
KRYTSTKEVLDATLIHQSITGLYETRIDLSQLGGDGGSGGSGGSGGSGGSGGSGGMDK
KYSIGLAIGTNSVGWAVITDEYKVPSKKFKVLGNTDRHSIKKNLIGALLFDSGETAEAT
RLKRTARRRYTRRKNRICYLQEIFSNEMAKVDDSFFHRLEESFLVEEDKKHERHPIFGNI
VDEVAYHEKYPTIYHLRKKLVDSTDKADLRLIYLALAHMIKFRGHFLIEGDLNPDNSD
VDKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNLS
DAILLSDILRVNTEITKAPLSASMIKRYDEHHQDLTLLKALVRQQLPEKYKEIFFDQSKN
GYAGYIDGGASQEEFYKFIKPILEKMDGTEELLVKLNREDLLRKQRTFDNGSIPHQIHL
GELHAILRRQEDFYPFLKDNREKIEKILTFRIPYYVGPLARGNSRFAWMTRKSEETITPW
NFEEVVDKGASAQSFIERMTNFDKNLPNEKVLPKHSLLYEYFTVYNELTKVKYVTEG
MRKPAFLSGEQKKAIVDLLFKTNRKVTVKQLKEDYFKKIECFDSVEISGVEDRFNASL
GTYHDLLKIIKDKDFLDNEENEDILEDIVLTLTLFEDREMIEERLKTYAHLFDDKVMKQ
LKRRRYTGWGRLSRKLINGIRDKQSGKTILDFLKSDGFANRNFMQLIHDDSLTFKEDIQ
KAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYV
DQELDINRLSDYDVDHIVPQSFLKDDSIDNKVLTRSDKNRGKSDNVPSEEVVKKMKNY
WRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALI
KKYPKLESEFVYGDYKVYDVRKMIAKSE
>SEQ ID NO:4 QBE1-C
   QEIGKATAKYFFYS
>SEQ ID NO:5 QBE5-N 
ARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDEH
HQDLTLLKALVRQQLPEKYKEIFFDQSKNGYAGYIDGGASQEEFYKFIKPILEKMDGTE
ELLVKLNREDLLRKQRTFDNGSIPHQIHLGELHAILRRQEDFYPFLKDNREKIEKILTFRI
PYYVGPLARGNSRFAWMTRKSEETITPWNFEEVVDKGASAQSFIERMTNFDKNLPNEK
 
VLPKHSLLYEYFTVYNELTKVKYVTEGMRKPAFLSGEQKKAIVDLLFKTNRKVTVKQL
KEDYFKKIECFDSVEISGVEDRFNASLGTYHDLLKIIKDKDFLDNEENEDILEDIVLTLTL
FEDREMIEERLKTYAHLFDDKVMKQLKRRRYTGWGRLSRKLINGIRDKQSGKTILDFL
KSDGFANRNFMQLIHDDSLTFKEDIQKAQVSGQGDSLHEHIANLAGSPAIKKGILQTVK
VVDELVKVMGRHKPENIVIEMARENQTTQKGQKNSRERMKRIEEGIKELGSQILKEHP
VENTQLQNEKLYLYYLQNGRDMYVDQELDINRLSDYDVDHIVPQSFLKDDSIDNKVLT
RSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFY
KVREINNYHHAHDAYLNAVVGTALIKKYPKLESEFVYGDYKVYDVRKMIAKSEQEIG
KATAKYFFYSNIMNFFKTEITLANGEIRKRPLIETNGETGEIVWDKGRDFATVRKVLSM
PQVNIVKKTEVQTGGFSKESILPKRNSDKLIARKKDWDPKKYGGFDSPTVAYSVLVVA
KVEKGKSKKLKSVKELLGITIMERSSFEKNPIDFLEAKGYKEVKKDLIIKLPKYSLFELE
NGRKRMLASAGELQKGNELALPSKYVNFLYLASHYEK
>SEQ ID NO:6 QBE5-C 
LKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDGGSGGSGGSGGSGGSGGSGGMDKKYSIGLAIGTNSVGWAVITDEYKVPSKK
FKVLGNTDRHSIKKNLIGALLFDSGETAEATRLKRTARRRYTRRKNRICYLQEIFSNEM
AKVDDSFFHRLEESFLVEEDKKHERHPIFGNIVDEVAYHEKYPTIYHLRKKLVDSTDKA
DLRLIYLALAHMIKFRGHFLIEGDLNPDNSDVDKLFIQLVQTYNQLFEENPINASGVDA
KAILS
>SEQ ID NO:7 QBE3-N 
KATAKYFFYSNIMNFFKTEITLANGEIRKRPLIETNGETGEIVWDKGRDFATVRKV
LSMPQVNIVKKTEVQTGGFSKESILPKRNSDKLIARKKDWDPKKYGGFDSPTVAYSVL
VVAKVEKGKSKKLKSVKELLGITIMERSSFEKNPIDFLEAKGYKEVKKDLIIKLPKYSLF
ELENGRKRMLASAGELQKGNELALPSKYVNFLYLASHYEK
>SEQ ID NO:8 QBE3-C 
LKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDGGSGGSGGSGGSGGSGGSGGMDKKYSIGLAIGTNSVGWAVITDEYKVPSKK
FKVLGNTDRHSIKKNLIGALLFDSGETAEATRLKRTARRRYTRRKNRICYLQEIFSNEM
AKVDDSFFHRLEESFLVEEDKKHERHPIFGNIVDEVAYHEKYPTIYHLRKKLVDSTDKA
 
DLRLIYLALAHMIKFRGHFLIEGDLNPDNSDVDKLFIQLVQTYNQLFEENPINASGVDA
KAILSARLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMDGT
EELLVKLNREDLLRKQRTFDNGSIPHQIHLGELHAILRRQEDFYPFLKDNREKIEKILTFR
IPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSIDNK
VLTRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSEQEI
G
>SEQ ID NO:9 QBE6-N 
SRFAWMTRKSEETITPWNFEEVVDKGASAQSFIERMTNFDKNLPNEKVLPKHSLL
YEYFTVYNELTKVKYVTEGMRKPAFLSGEQKKAIVDLLFKTNRKVTVKQLKEDYFKK
IECFDSVEISGVEDRFNASLGTYHDLLKIIKDKDFLDNEENEDILEDIVLTLTLFEDREMI
EERLKTYAHLFDDKVMKQLKRRRYTGWGRLSRKLINGIRDKQSGKTILDFLKSDGFAN
RNFMQLIHDDSLTFKEDIQKAQVSGQGDSLHEHIANLAGSPAIKKGILQTVKVVDELVK
VMGRHKPENIVIEMARENQTTQKGQKNSRERMKRIEEGIKELGSQILKEHPVENTQLQ
NEKLYLYYLQNGRDMYVDQELDINRLSDYDVDHIVPQSFLKDDSIDNKVLTRSDKNRG
KSDNVPSEEVVKKMKNYWRQLLNAKLITQRKFDNLTKAERGGLSELDKAGFIKRQLV
ETRQITKHVAQILDSRMNTKYDENDKLIREVKVITLKSKLVSDFRKDFQFYKVREINNY
HHAHDAYLNAVVGTALIKKYPKLESEFVYGDYKVYDVRKMIAKSEQEIGKATAKYFF
YSNIMNFFKTEITLANGEIRKRPLIETNGETGEIVWDKGRDFATVRKVLSMPQVNIVKK
TEVQTGGFSKESILPKRNSDKLIARKKDWDPKKYGGFDSPTVAYSVLVVAKVEKGKSK
KLKSVKELLGITIMERSSFEKNPIDFLEAKGYKEVKKDLIIKLPKYSLFELENGRKRMLA
SAGELQKGNELALPSKYVNFLYLASHYEK
>SEQ ID NO:10 QBE6-C LKGSPEDNEQKQLFVEQHKHYLDEIIEQISEFSKRVILADANLDKVLSAYNKHRDK 
PIREQAENIIHLFTLTNLGAPAAFKYFDTTIDRKRYTSTKEVLDATLIHQSITGLYETRIDL
SQLGGDGGSGGSGGSGGSGGSGGSGGMDKKYSIGLAIGTNSVGWAVITDEYKVPSKK
FKVLGNTDRHSIKKNLIGALLFDSGETAEATRLKRTARRRYTRRKNRICYLQEIFSNEM
AKVDDSFFHRLEESFLVEEDKKHERHPIFGNIVDEVAYHEKYPTIYHLRKKLVDSTDKA
DLRLIYLALAHMIKFRGHFLIEGDLNPDNSDVDKLFIQLVQTYNQLFEENPINASGVDA
KAILSARLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMDGT
EELLVKLNREDLLRKQRTFDNGSIPHQIHLGELHAILRRQEDFYPFLKDNREKIEKILTFR
IPYYVGPLARGN
>SEQ ID NO:11 QBE7-N 
TVKQLKEDYFKKIECFDSVEISGVEDRFNASLGTYHDLLKIIKDKDFLDNEENEDI
LEDIVLTLTLFEDREMIEERLKTYAHLFDDKVMKQLKRRRYTGWGRLSRKLINGIRDK
QSGKTILDFLKSDGFANRNFMQLIHDDSLTFKEDIQKAQVSGQGDSLHEHIANLAGSPA
IKKGILQTVKVVDELVKVMGRHKPENIVIEMARENQTTQKGQKNSRERMKRIEEGIKE
LGSQILKEHPVENTQLQNEKLYLYYLQNGRDMYVDQELDINRLSDYDVDHIVPQSFLK
DDSIDNKVLTRSDKNRGKSDNVPSEEVVKKMKNYWRQLLNAKLITQRKFDNLTKAER
GGLSELDKAGFIKRQLVETRQITKHVAQILDSRMNTKYDENDKLIREVKVITLKSKLVS
DFRKDFQFYKVREINNYHHAHDAYLNAVVGTALIKKYPKLESEFVYGDYKVYDVRK
MIAKSEQEIGKATAKYFFYSNIMNFFKTEITLANGEIRKRPLIETNGETGEIVWDKGRDF
ATVRKVLSMPQVNIVKKTEVQTGGFSKESILPKRNSDKLIARKKDWDPKKYGGFDSPT
VAYSVLVVAKVEKGKSKKLKSVKELLGITIMERSSFEKNPIDFLEAKGYKEVKKDLIIKL
PKYSLFELENGRKRMLASAGELQKGNELALPSKYVNFLYLASHYEK
>SEQ ID NO:12 QBE7-C 
LKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDGGSGGSGGSGGSGGSGGMDKKYSIGLAIGTNSVGWAVITDEYKVPSKK
FKVLGNTDRHSIKKNLIGALLFDSGETAEATRLKRTARRRYTRRKNRICYLQEIFSNEM
AKVDDSFFHRLEESFLVEEDKKHERHPIFGNIVDEVAYHEKYPTIYHLRKKLVDSTDKA
DLRLIYLALAHMIKFRGHFLIEGDLNPDNSDVDKLFIQLVQTYNQLFEENPINASGVDA
KAILSARLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMDGT
 
EELLVKLNREDLLRKQRTFDNGSIPHQIHLGELHAILRRQEDFYPFLKDNREKIEKILTFR
IPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKV
>SEQ ID NO:13 QBE8-N 
GFANRNFMQLIHDDSLTFKEDIQKAQVSGQGDSLHEHIANLAGSPAIKKGILQTVK
VVDELVKVMGRHKPENIVIEMARENQTTQKGQKNSRERMKRIEEGIKELGSQILKEHP
VENTQLQNEKLYLYYLQNGRDMYVDQELDINRLSDYDVDHIVPQSFLKDDSIDNKVLT
RSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFY
KVREINNYHHAHDAYLNAVVGTALIKKYPKLESEFVYGDYKVYDVRKMIAKSEQEIG
KATAKYFFYSNIMNFFKTEITLANGEIRKRPLIETNGETGEIVWDKGRDFATVRKVLSM
PQVNIVKKTEVQTGGFSKESILPKRNSDKLIARKKDWDPKKYGGFDSPTVAYSVLVVA
KVEKGKSKKLKSVKELLGITIMERSSFEKNPIDFLEAKGYKEVKKDLIIKLPKYSLFELE
NGRKRMLASAGELQKGNELALPSKYVNFLYLASHYEK
>SEQ ID NO:14 QBE8-C 
LKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDGGSGGSGGSGGSGGSGGSGGMDKKYSIGLAIGTNSVGWAVITDEYKVPSKK
FKVLGNTDRHSIKKNLIGALLFDSGETAEATRLKRTARRRYTRRKNRICYLQEIFSNEM
AKVDDSFFHRLEESFLVEEDKKHERHPIFGNIVDEVAYHEKYPTIYHLRKKLVDSTDKA
DLRLIYLALAHMIKFRGHFLIEGDLNPDNSDVDKLFIQLVQTYNQLFEENPINASGVDA
KAILSARLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMDGT
EELLVKLNREDLLRKQRTFDNGSIPHQIHLGELHAILRRQEDFYPFLKDNREKIEKILTFR
IPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSD
>SEQ ID NO:15 QBE9-N ENTQLQNEKLYLYYLQNGRDMYVDQELDINRLSDYDVDHIVPQSFLKDDSIDNK 
VLTRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSEQEI
GKATAKYFFYSNIMNFFKTEITLANGEIRKRPLIETNGETGEIVWDKGRDFATVRKVLS
MPQVNIVKKTEVQTGGFSKESILPKRNSDKLIARKKDWDPKKYGGFDSPTVAYSVLVV
AKVEKGKSKKLKSVKELLGITIMERSSFEKNPIDFLEAKGYKEVKKDLIIKLPKYSLFEL
ENGRKRMLASAGELQKGNELALPSKYVNFLYLASHYEK
>SEQ ID NO:16 QBE9-C 
LKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDGGSGGSGGSGGSGGSGGSGGMDKKYSIGLAIGTNSVGWAVITDEYKVPSKK
FKVLGNTDRHSIKKNLIGALLFDSGETAEATRLKRTARRRYTRRKNRICYLQEIFSNEM
AKVDDSFFHRLEESFLVEEDKKHERHPIFGNIVDEVAYHEKYPTIYHLRKKLVDSTDKA
DLRLIYLALAHMIKFRGHFLIEGDLNPDNSDVDKLFIQLVQTYNQLFEENPINASGVDA
KAILSARLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMDGT
EELLVKLNREDLLRKQRTFDNGSIPHQIHLGELHAILRRQEDFYPFLKDNREKIEKILTFR
IPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILKE
HPV
>SEQ ID NO:17 QBE2-N 
ARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDEH
HQDLTLLKALVRQQLPEKYKEIFFDQSKNGYAGYIDGGASQEEFYKFIKPILEKMDGTE
ELLVKLNREDLLRKQRTFDNGSIPHQIHLGELHAILRRQEDFYPFLKDNREKIEKILTFRI
PYYVGPLARGNSRFAWMTRKSEETITPWNFEEVVDKGASAQSFIERMTNFDKNLPNEK
VLPKHSLLYEYFTVYNELTKVKYVTEGMRKPAFLSGEQKKAIVDLLFKTNRKVTVKQL
KEDYFKKIECFDSVEISGVEDRFNASLGTYHDLLKIIKDKDFLDNEENEDILEDIVLTLTL
 
FEDREMIEERLKTYAHLFDDKVMKQLKRRRYTGWGRLSRKLINGIRDKQSGKTILDFL
KSDGFANRNFMQLIHDDSLTFKEDIQKAQVSGQGDSLHEHIANLAGSPAIKKGILQTVK
VVDELVKVMGRHKPENIVIEMARENQTTQKGQKNSRERMKRIEEGIKELGSQILKEHP
VENTQLQNEKLYLYYLQNGRDMYVDQELDINRLSDYDVDHIVPQSFLKDDSIDNKVLT
RSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFY
KVREINNYHHAHDAYLNAVVGTALIKKYPKLESEFVYGDYKVYDVRKMIAKSEQEIG
KATAKYFFYSNIMNFFKTEITLANGEI
>SEQ ID NO:18 QBE2-C 
RKRPLIETNGETGEIVWDKGRDFATVRKVLSMPQVNIVKKTEVQTGGFSKESILPK
RNSDKLIARKKDWDPKKYGGFDSPTVAYSVLVVAKVEKGKSKKLKSVKELLGITIMER
SSFEKNPIDFLEAKGYKEVKKDLIIKLPKYSLFELENGRKRMLASAGELQKGNELALPS
KYVNFLYLASHYEKLKGSPEDNEQKQLFVEQHKHYLDEIIEQISEFSKRVILADANLDK
VLSAYNKHRDKPIREQAENIIHLFTLTNLGAPAAFKYFDTTIDRKRYTSTKEVLDATLIH
QSITGLYETRIDLSQLGGDGGSGGSGGSGGSGGSGGSGGMDKKYSIGLAIGTNSVGWA
VITDEYKVPSKKFKVLGNTDRHSIKKNLIGALLFDSGETAEATRLKRTARRRYTRRKNR
ICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFE
ENPINASGVDAKAILS
>SEQ ID NO:19 QBE10-N
   KATAKYFFYSNIMNFFKTEITLANGEI
>SEQ ID NO:20 QBE10-C 
RKRPLIETNGETGEIVWDKGRDFATVRKVLSMPQVNIVKKTEVQTGGFSKESILPK
RNSDKLIARKKDWDPKKYGGFDSPTVAYSVLVVAKVEKGKSKKLKSVKELLGITIMER
SSFEKNPIDFLEAKGYKEVKKDLIIKLPKYSLFELENGRKRMLASAGELQKGNELALPS
KYVNFLYLASHYEKLKGSPEDNEQKQLFVEQHKHYLDEIIEQISEFSKRVILADANLDK
VLSAYNKHRDKPIREQAENIIHLFTLTNLGAPAAFKYFDTTIDRKRYTSTKEVLDATLIH
QSITGLYETRIDLSQLGGDGGSGGSGGSGGSGGSGGMDKKYSIGLAIGTNSVGWA
VITDEYKVPSKKFKVLGNTDRHSIKKNLIGALLFDSGETAEATRLKRTARRRYTRRKNR
ICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFE
ENPINASGVDAKAILSARLSKSRRLENLIAQLPGEKKNGLFGNLIALSLGLTPNFKSNFD
 
LAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYK
FIKPILEKMDGTEELLVKLNREDLLRKQRTFDNGSIPHQIHLGELHAILRRQEDFYPFLK
DNREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLING
IRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVPQ
SFLKDDSIDNKVLTRSDKNRGKSDNVPSEEVVKKMKNYWRQLLNAKLITQRKFDNLT
KAERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVYD
VRKMIAKSEQEIG
>SEQ ID NO:21 QBE11-N 
SRFAWMTRKSEETITPWNFEEVVDKGASAQSFIERMTNFDKNLPNEKVLPKHSLL
YEYFTVYNELTKVKYVTEGMRKPAFLSGEQKKAIVDLLFKTNRKVTVKQLKEDYFKK
IECFDSVEISGVEDRFNASLGTYHDLLKIIKDKDFLDNEENEDILEDIVLTLTLFEDREMI
EERLKTYAHLFDDKVMKQLKRRRYTGWGRLSRKLINGIRDKQSGKTILDFLKSDGFAN
RNFMQLIHDDSLTFKEDIQKAQVSGQGDSLHEHIANLAGSPAIKKGILQTVKVVDELVK
VMGRHKPENIVIEMARENQTTQKGQKNSRERMKRIEEGIKELGSQILKEHPVENTQLQ
NEKLYLYYLQNGRDMYVDQELDINRLSDYDVDHIVPQSFLKDDSIDNKVLTRSDKNRG
KSDNVPSEEVVKKMKNYWRQLLNAKLITQRKFDNLTKAERGGLSELDKAGFIKRQLV
ETRQITKHVAQILDSRMNTKYDENDKLIREVKVITLKSKLVSDFRKDFQFYKVREINNY
HHAHDAYLNAVVGTALIKKYPKLESEFVYGDYKVYDVRKMIAKSEQEIGKATAKYFF
YSNIMNFFKTEITLANGEI
>SEQ ID NO:22 QBE11-C 
RKRPLIETNGETGEIVWDKGRDFATVRKVLSMPQVNIVKKTEVQTGGFSKESILPK
RNSDKLIARKKDWDPKKYGGFDSPTVAYSVLVVAKVEKGKSKKLKSVKELLGITIMER
SSFEKNPIDFLEAKGYKEVKKDLIIKLPKYSLFELENGRKRMLASAGELQKGNELALPS
KYVNFLYLASHYEKLKGSPEDNEQKQLFVEQHKHYLDEIIEQISEFSKRVILADANLDK
VLSAYNKHRDKPIREQAENIIHLFTLTNLGAPAAFKYFDTTIDRKRYTSTKEVLDATLIH
QSITGLYETRIDLSQLGGDGGSGGSGGSGGSGGSGGSGGMDKKYSIGLAIGTNSVGWA
 
VITDEYKVPSKKFKVLGNTDRHSIKKNLIGALLFDSGETAEATRLKRTARRRYTRRKNR
ICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFE
ENPINASGVDAKAILSARLSKSRRLENLIAQLPGEKKNGLFGNLIALSLGLTPNFKSNFD
LAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYK
FIKPILEKMDGTEELLVKLNREDLLRKQRTFDNGSIPHQIHLGELHAILRRQEDFYPFLK
DNREKIEKILTFRIPYYVGPLARGN
>SEQ ID NO:23 QBE12-N 
TVKQLKEDYFKKIECFDSVEISGVEDRFNASLGTYHDLLKIIKDKDFLDNEENEDI
LEDIVLTLTLFEDREMIEERLKTYAHLFDDKVMKQLKRRRYTGWGRLSRKLINGIRDK
QSGKTILDFLKSDGFANRNFMQLIHDDSLTFKEDIQKAQVSGQGDSLHEHIANLAGSPA
IKKGILQTVKVVDELVKVMGRHKPENIVIEMARENQTTQKGQKNSRERMKRIEEGIKE
LGSQILKEHPVENTQLQNEKLYLYYLQNGRDMYVDQELDINRLSDYDVDHIVPQSFLK
DDSIDNKVLTRSDKNRGKSDNVPSEEVVKKMKNYWRQLLNAKLITQRKFDNLTKAER
GGLSELDKAGFIKRQLVETRQITKHVAQILDSRMNTKYDENDKLIREVKVITLKSKLVS
DFRKDFQFYKVREINNYHHAHDAYLNAVVGTALIKKYPKLESEFVYGDYKVYDVRK
MIAKSEQEIGKATAKYFFYSNIMNFFKTEITLANGEI
>SEQ ID NO:24 QBE12-C 
RKRPLIETNGETGEIVWDKGRDFATVRKVLSMPQVNIVKKTEVQTGGFSKESILPK
RNSDKLIARKKDWDPKKYGGFDSPTVAYSVLVVAKVEKGKSKKLKSVKELLGITIMER
SSFEKNPIDFLEAKGYKEVKKDLIIKLPKYSLFELENGRKRMLASAGELQKGNELALPS
KYVNFLYLASHYEKLKGSPEDNEQKQLFVEQHKHYLDEIIEQISEFSKRVILADANLDK
VLSAYNKHRDKPIREQAENIIHLFTLTNLGAPAAFKYFDTTIDRKRYTSTKEVLDATLIH
QSITGLYETRIDLSQLGGDGGSGGSGGSGGSGGSGGSGGMDKKYSIGLAIGTNSVGWA
VITDEYKVPSKKFKVLGNTDRHSIKKNLIGALLFDSGETAEATRLKRTARRRYTRRKNR
ICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFE
ENPINASGVDAKAILSARLSKSRRLENLIAQLPGEKKNGLFGNLIALSLGLTPNFKSNFD
LAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYK
FIKPILEKMDGTEELLVKLNREDLLRKQRTFDNGSIPHQIHLGELHAILRRQEDFYPFLK
DNREKIEKILTFRIPYYVGPLARGNSRFAWMTRKSEETITPWNFEEVVDKGASAQSFIER
 
MTNFDKNLPNEKVLPKHSLLYEYFTVYNELTKVKYVTEGMRKPAFLSGEQKKAIVDL
LFKTNRKV
>SEQ ID NO:25 QBE13-N 
GFANRNFMQLIHDDSLTFKEDIQKAQVSGQGDSLHEHIANLAGSPAIKKGILQTVK
VVDELVKVMGRHKPENIVIEMARENQTTQKGQKNSRERMKRIEEGIKELGSQILKEHP
VENTQLQNEKLYLYYLQNGRDMYVDQELDINRLSDYDVDHIVPQSFLKDDSIDNKVLT
RSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFY
KVREINNYHHAHDAYLNAVVGTALIKKYPKLESEFVYGDYKVYDVRKMIAKSEQEIG
KATAKYFFYSNIMNFFKTEITLANGEI
>SEQ ID NO:26 QBE13-C 
RKRPLIETNGETGEIVWDKGRDFATVRKVLSMPQVNIVKKTEVQTGGFSKESILPK
RNSDKLIARKKDWDPKKYGGFDSPTVAYSVLVVAKVEKGKSKKLKSVKELLGITIMER
SSFEKNPIDFLEAKGYKEVKKDLIIKLPKYSLFELENGRKRMLASAGELQKGNELALPS
KYVNFLYLASHYEKLKGSPEDNEQKQLFVEQHKHYLDEIIEQISEFSKRVILADANLDK
VLSAYNKHRDKPIREQAENIIHLFTLTNLGAPAAFKYFDTTIDRKRYTSTKEVLDATLIH
QSITGLYETRIDLSQLGGDGGSGGSGGSGGSGGSGGSGGMDKKYSIGLAIGTNSVGWA
VITDEYKVPSKKFKVLGNTDRHSIKKNLIGALLFDSGETAEATRLKRTARRRYTRRKNR
ICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFE
ENPINASGVDAKAILSARLSKSRRLENLIAQLPGEKKNGLFGNLIALSLGLTPNFKSNFD
LAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYK
FIKPILEKMDGTEELLVKLNREDLLRKQRTFDNGSIPHQIHLGELHAILRRQEDFYPFLK
DNREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLING
IRDKQSGKTILDFLKSD
>SEQ ID NO:27 QBE14-N 
ENTQLQNEKLYLYYLQNGRDMYVDQELDINRLSDYDVDHIVPQSFLKDDSIDNK
VLTRSDKNRGKSDNVPSEEVVKKMKNYWRQLLNAKLITQRKFDNLTKAERGGLSELD
 
KAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSEQEI
GKATAKYFFYSNIMNFFKTEITLANGEI
>SEQ ID NO:28 QBE14-C 
RKRPLIETNGETGEIVWDKGRDFATVRKVLSMPQVNIVKKTEVQTGGFSKESILPK
RNSDKLIARKKDWDPKKYGGFDSPTVAYSVLVVAKVEKGKSKKLKSVKELLGITIMER
SSFEKNPIDFLEAKGYKEVKKDLIIKLPKYSLFELENGRKRMLASAGELQKGNELALPS
KYVNFLYLASHYEKLKGSPEDNEQKQLFVEQHKHYLDEIIEQISEFSKRVILADANLDK
VLSAYNKHRDKPIREQAENIIHLFTLTNLGAPAAFKYFDTTIDRKRYTSTKEVLDATLIH
QSITGLYETRIDLSQLGGDGGSGGSGGSGGSGGSGGSGGMDKKYSIGLAIGTNSVGWA
VITDEYKVPSKKFKVLGNTDRHSIKKNLIGALLFDSGETAEATRLKRTARRRYTRRKNR
ICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFE
ENPINASGVDAKAILSARLSKSRRLENLIAQLPGEKKNGLFGNLIALSLGLTPNFKSNFD
LAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYK
FIKPILEKMDGTEELLVKLNREDLLRKQRTFDNGSIPHQIHLGELHAILRRQEDFYPFLK
DNREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLING
IRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEE
GIKELGSQILKEHPV
>SEQ ID NO:29 Circularly Permuted Sequence 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILSARLSKSRRLENLIAQLPGEKK
NGLFGNLIALSLGLTPNFKSNFDLAEDAKLQLSKDTYDDDLDNLLAQIGDQYADLFLA
AKNLSDAILLSDILRVNTEITKAPLSASMIKRYDEHHQDLTLLKALVRQQLPEKYKEIFF
DQSKNGYAGYIDGGASQEEFYKFIKPILEKMDGTEELLVKLNREDLLRKQRTFDNGSIP
HQIHLGELHAILRRQEDFYPFLKDNREKIEKILTFRIPYYVGPLARGNSRFAWMTRKSEE
 
TITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRF
NASLGTYHDLLKIIKDKDFLDNEENEDILEDIVLTLTLFEDREMIEERLKTYAHLFDDKV
MKQLKRRRYTGWGRLSRKLINGIRDKQSGKTILDFLKSDGFANRNFMQLIHDDSLTFK
EDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRD
MYVDQELDINRLSDYDVDHIVPQSFLKDDSIDNKVLTRSDKNRGKSDNVPSEEVVKK
MKNYWRQLLNAKLITQRKFDNLTKAERGGLSELDKAGFIKRQLVETRQITKHVAQILD
SRMNTKYDENDKLIREVKVITLKSKLVSDFRKDFQFYKVREINNYHHAHDAYLNAVV
GTALIKKYPKLESEFVYGDYKVYDVRKMIAKSEQEIGKATAKYFFYSNIMNFFKTEITL
ANGEIRKRPLIETNGETGEIVWDKGRDFATVRKVLSMPQVNIVKKTEVQTGGFSKESIL
PKRNSDKLIARKKDWDPKKYGGFDSPTVAYSVLVVAKVEKGKSKKLKSVKELLGITIM
ERSSFEKNPIDFLEAKGYKEVKKDLIIKLPKYSLFELENGRKRMLASAGELQKGNELAL
PSKYVNFLYLASHYEKLKGSPEDNEQKQLFVEQHKHYLDEIIEQISEFSKRVILADANL
DKVLSAYNKHRDKPIREQAENIIHLFTLTNLGAPAAFKYFDTTIDRKRYTSTKEVLDATL
IHQSITGLYETRIDLSQLGGDGGSGGSGGSGGSGGSGGSGG
>SEQ ID NO:30 NLS
   KKRKV
>SEQ ID NO:31 NLS
   PKKKRKV
>SEQ ID NO:32 NLS
   KRPAATKKAGQAKKKK
>SEQ ID NO:33 NLS
   MDSLLMNRRKFLYQFKNVRWAKGRRETYLC
>SEQ ID NO:34 NLS
   KRTADGSEFEPKKKRKV
>SEQ ID NO:35 NLS
   PAAKRVKLD
>SEQ ID NO:36 NLS
   RQRRNELKRSP
>SEQ ID NO:37 NLS
   NQSSNFGPMKGGNFGGRSSGPYGGGGQYFAKPRNQGGY
>SEQ ID NO:38 NLS
   RMRIZFKNKGKDTAELRRRRVEVSVELRKAKKDEQILKRRNV
>SEQ ID NO:39 NLS
   VSRKRPRP
>SEQ ID NO:40 NLS
   PPKKARED
>SEQ ID NO:41 NLS
   POPKKKPL
>SEQ ID NO:42 NLS
   SALIKKKKKMAP
>SEQ ID NO:43 NLS
   RKLKKKIKKL
>SEQ ID NO:44 NLS
   REKKKFLKRR
>SEQ ID NO:45 NLS
   KRKGDEVDGVDEVAKKKSKK
>SEQ ID NO:46 NLS
   RKCLQAGMNLEARKTKK
>SEQ ID NO: 47 Linker
   GGGS
>SEQ ID NO: 48 Linker
   GGGGS
>SEQ ID NO: 49 Linker
   EAAAK
> Linker
   GGS
>SEQ ID NO: 50 Linker
   GGSGGS
>SEQ ID NO: 51 Linker
   SGGS
>SEQ ID NO: 52 Linker
   SGSETPGTSESATPES
>SEQ ID NO:53 Linker
   SGGSSGGSSGSETPGTSESATPESSGGSSGGS
>SEQ ID NO:54 hAID 
DSLLMNRRKFLYQFKNVRWAKGRRETYLCYVVKRRDSATSFSLDFGYLRNKNGC
HVELLFLRYISDWDLDPGRCYRVTWFTSWSPCYDCARHVADFLRGNPNLSLRIFTARL
YFCEDRKAEPEGLRRLHRAGVQIAIMTFKDYFYCWNTFVENHERTFKAWEGLHENSV
RLSRQLRRILLPLYEVDDLRDAFRTLGL
>SEQ ID NO:55 Deaminase rAPOBEC1: 
SSETGPVAVDPTLRRRIEPHEFEVFFDPRELRKETCLLYEINWGGRHSIWRHTSQNT
NKHVEVNFIEKFTTERYFCPNTRCSITWFLSWSPCGECSRAITEFLSRYPHVTLFIYIARL
YHHADPRNRQGLRDLISSGVTIQIMTEQESGYCWRNFVNYSPSNEAHWPRYPHLWVR
LYVLELYCIILGLPPCLNILRRKQPQLTFFTIALQSCHYQRLPPHILWATGLK
>SEQ ID NO:56 hAPOBEC1 
TSEKGPSTGDPTLRRRIEPWEFDVFYDPRELRKEACLLYEIKWGMSRKIWRSSGK
NTTNHVEVNFIKKFTSERDFHPSMSCSITWFLSWSPCWECSQAIREFLSRHPGVTLVIYV
ARLFWHMDQQNRQGLRDLVNSGVTIQIMRASEYYHCWRNFVNYPPGDEAHWPQYPP
LWMMLYALELHCIILSLPPCLKISRRWQNHLTFFRLHLQNCHYQTIPPHILLATGLIHPSV
AWR
>SEQ ID NO:57 hAPOBEC3A: 
EASPASGPRHLMDPHIFTSNFNNGIGRHKTYLCYEVERLDNGTSVKMDQHRGFLH
NQAKNLLCGFYGRHAELRFLDLVPSLQLDPAQIYRVTWFISWSPCFSWGCAGEVRAFL
QENTHVRLRIFAARIYDYDPLYKEALQMLRDAGAQVSIMTYDEFKHCWDTFVDHQGC
PFQPWDGLDEHSQALSGRLRAILQNQGN
>SEQ ID NO:58 hAPOBEC3G-CTD: 
DPPTFTFNFNNEPWVRGRHETYLCYEVERMHNDTWVLLNQRRGFLCNQAPHKH
GFLEGRHAELCFLDVIPFWKLDLDQDYRVTCFTSWSPCFSCAQEMAKFISKNKHVSLCI
FTARIYDDQGRCQEGLRTLAEAGAKISIMTYSEFKHCWDTFVDHQGCPFQPWDGLDE
HSQDLSGRLRAILQNQEN
>SEQ ID NO:59 PmCDA1: 
TDAEYVRIHEKLDIYTFKKQFFNNKKSVSHRCYVLFELKRRGERRACFWGYAVN
KPQSGTERGIHAEIFSIRKVEEYLRDNPGQFTINWYSSWSPCADCAEKILEWYNQELRG
NGHTLKIWACKLYYEKNARNQIGLWNLRDNGVGLNVMVSEHYQCCRKIFIQSSHNQL
NENRWLEKTLKRAEKRRSELSIMIQVKILHTTKSPAVSRGSG
>SEQ ID NO:60 PpAPOBEC1 
TSEKGPSTGDPTLRRRIESWEFDVFYDPRELRKETCLLYEIKWGMSRKIWRSSGKN
TTNHVEVNFIKKFTSERRFHSSISCSITWFLSWSPCWECSQAIREFLSQHPGVTLVIYVAR
LFWHMDQRNRQGLRDLVNSGVTIQIMRASEYYHCWRNFVNYPPGDEAHWPQYPPLW
MMLYALELHCIILSLPPCLKISRRWQNHLAFFRLHLQNCHYQTIPPHILLATGLIHPSVT
WR
>SEQ ID NO:61 tCDA1EQ: 
SHRCYVLFELKRRGERRACFWGYAVNKPQSGTERGIHAEIFSIRKVEEYLRDNPGQ
FTINWYSSWSPCADCAEKILEWYNQELRGNGHTLKIEACKLYYEKNARNQIGLQNLR
DNGVGLNV
>SEQ ID NO:62 RrAPOBEC3F 
KPQIRDHRPNPMEAMYPHIFYFHFENLEKAYGRNETWLCFTVEIIKQYLPVPWKK
GVFRNQVDPETHCHAEKCFLSWFCNNTLSPKKNYQVTWYTSWSPCPECAGEVAEFLA
EHSNVKLTIYTARLYYFWDTDYQEGLRSLSEEGASVEIMDYEDFQYCWENFVYDDGE
PFKRWKGLKYNFQSLTRRLREILQ
>SEQ ID NO:63 AmAPOBEC1: 
ADSSEKMRGQYISRDTFEKNYKPIDGTKEAHLLCEIKWGKYGKPWLHWCQNQR
MNIHAEDYFMNNIFKAKKHPVHCYVTWYLSWSPCADCASKIVKFLEERPYLKLTIYVA
QLYYHTEEENRKGLRLLRSKKVIIRVMDISDYNYCWKVFVSNQNGNEDYWPLQFDPW
VKENYSRLLDIFWESKCRSPNPW
>SEQ ID NO:64 SsAPOBEC3B: 
DPQRLRQWPGPGPASRGGYGQRPRIRNPEEWFHELSPRTFSFHFRNLRFASGRNRS
YICCQVEGKNCFFQGIFQNQVPPDPPCHAELCFLSWFQSWGLSPDEHYYVTWFISWSP
CCECAAKVAQFLEENRNVSLSLSAARLYYFWKSESREGLRRLSDLGAQVGIMSFQDFQ
HCWNNFVHNLGMPFQPWKKLHKNYQRLVTELKQILREEPATYGSPQAQGKVRIGSTA
AGLRHSHSHTRSEAHLRPNHSSRQHRILNPPREARARTCVLVDASWICYR
>SEQ ID NO:65 hAPOBEC3B 
NPQIRNPMERMYRDTFYDNFENEPILYGRSYTWLCYEVKIKRGRSNLLWDTGVFR
GQVYFKPQYHAEMCFLSWFCGNQLPAYKCFQITWFVSWTPCPDCVAKLAEFLSEHPN
VTLTISAARLYYYWERDYRRALCRLSQAGARVKIMDYEEFAYCWENFVYNEGQQFMP
WYKFDENYAFLHRTLKEILRYLMDPDTFTFNFNNDPLVLRRRQTYLCYEVERLDNGT
WVLMDQHMGFLCNEAKNLLCGFYGRHAELRFLDLVPSLQLDPAQIYRVTWFISWSPC
FSWGCAGEVRAFLQENTHVRLRIFAARIYDYDPLYKEALQMLRDAGAQVSIMTYDEF
EYCWDTFVYRQGCPFQPWDGLEEHSQALSGRLRAILQNQGN
>SEQ ID NO:66 hAPOBEC3C 
NPQIRNPMKAMYPGTFYFQFKNLWEANDRNETWLCFTVEGIKRRSVVSWKTGVF
RNQVDSETHCHAERCFLSWFCDDILSPNTKYQVTWYTSWSPCPDCAGEVAEFLARHS
NVNLTIFTARLYYFQYPCYQEGLRSLSQEGVAVEIMDYEDFKYCWENFVYNDNEPFKP
WKGLKTNFRLLKRRLRESLQ
>SEQ ID NO:67 hAPOBEC3D 
NPQIRNPMERMYRDTFYDNFENEPILYGRSYTWLCYEVKIKRGRSNLLWDTGVFR
GPVLPKRQSNHRQEVYFRFENHAEMCFLSWFCGNRLPANRRFQITWFVSWNPCLPCV
VKVTKFLAEHPNVTLTISAARLYYYRDRDWRWVLLRLHKAGARVKIMDYEDFAYCW
ENFVCNEGQPFMPWYKFDDNYASLHRTLKEILRNPMEAMYPHIFYFHFKNLLKACGR
NESWLCFTMEVTKHHSAVFRKRGVFRNQVDPETHCHAERCFLSWFCDDILSPNTNYE
VTWYTSWSPCPECAGEVAEFLARHSNVNLTIFTARLCYFWDTDYQEGLCSLSQEGASV
KIMGYKDFVSCWKNFVYSDDEPFKPWKGLQTNFRLLKRRLREILQ
>SEQ ID NO:68 hAPOBEC3F 
KPHFRNTVERMYRDTFSYNFYNRPILSRRNTVWLCYEVKTKGPSRPRLDAKIFRG
QVYSQPEHHAEMCFLSWFCGNQLPAYKCFQITWFVSWTPCPDCVAKLAEFLAEHPNV
TLTISAARLYYYWERDYRRALCRLSQAGARVKIMDDEEFAYCWENFVYSEGQPFMPW
YKFDDNYAFLHRTLKEILRNPMEAMYPHIFYFHFKNLRKAYGRNESWLCFTMEVVKH
HSPVSWKRGVFRNQVDPETHCHAERCFLSWFCDDILSPNTNYEVTWYTSWSPCPECA
GEVAEFLARHSNVNLTIFTARLYYFWDTDYQEGLRSLSQEGASVEIMGYKDFKYCWEN
FVYNDDEPFKPWKGLKYNFLFLDSKLQEILE
>SEQ ID NO:69 hAPOBEC3G 
KPHFRNTVERMYRDTFSYNFYNRPILSRRNTVWLCYEVKTKGPSRPPLDAKIFRG
QVYSELKYHPEMRFFHWFSKWRKLHRDQEYEVTWYISWSPCTKCTRDMATFLAEDP
KVTLTIFVARLYYFWDPDYQEALRSLCQKRDGPRATMKIMNYDEFQHCWSKFVYSQR
ELFEPWNNLPKYYILLHIMLGEILRHSMDPPTFTFNFNNEPWVRGRHETYLCYEVERM
HNDTWVLLNQRRGFLCNQAPHKHGFLEGRHAELCFLDVIPFWKLDLDQDYRVTCFTS
WSPCFSCAQEMAKFISKNKHVSLCIFTARIYDDQGRCQEGLRTLAEAGAKISIMTYSEF
KHCWDTFVDHQGCPFQPWDGLDEHSQDLSGRLRAILQNQEN
>SEQ ID NO:70 hAPOBEC3H 
ALLTAETFRLQFNNKRRLRRPYYPRKALLCYQLTPQNGSTPTRGYFENKKKCHAE
ICFINEIKSMGLDETQCYQVTCYLTWSPCSSCAWELVDFIKAHDHLNLRIFASRLYYHW
CKPQQDGLRLLCGSQVPVEVMGFPEFADCWENFVDHEKPLSFNPYKMLEELDKNSRA
IKRRLDRIKS
>SEQ ID NO:71 APOBEC3B-CTD EILRYLMDPDTFTFNFNNDPLVLRRRQTYLCYEVERLDNGTWVLMDQHMGFLCN 
EAKNLLCGFYGRHAELRFLDLVPSLQLDPAQIYRVTWFISWSPCFSWGCAGEVRAFLQ
ENTHVRLRIFAARIYDYDPLYKEALQMLRDAGAQVSIMTYDEFEYCWDTFVYRQGCP
FQPWDGLEEHSQALSGRLRAILQNQGN
>SEQ ID NO:72 evoFERNY 
FERNYDPRELRKETYLLYEIKWGKSGKLWRHWCQNNRTQHAEVYFLENIFNARR
FNPSTHCSITWYLSWSPCAECSQKIVDFLKEHPNVNLEIYVARLYYHEDERNRQGLRDL
VNSGVTIRIMDLPDYNYCWKTFVSDQGGDEDYWPGHFAPWIKQYSLKL
>SEQ ID NO:73 Sdd2 
APDSLVWFDPLGLIVLQQVPYNDHPLFGAVSEFIQGKSRSDLRGRNVAAVLLDDG
TVIVRASEGGGNHAERVLMGLSEVDPAKVVAVYTERSPCTGRINCHDLLDSSLGADVP
VYYTHEMIRGQEGKTAQQIEADRNQFCRGG
>SEQ ID NO:74 Sdd3 
SASAQLNTYLAAIGNSTTTVEAQPEAAPPPAAAESLDSTPRLPDGGIDFHALAKRL
GLLEARPTEQPPFDPRRFNPACWQGLKPYDQAGTAEGNLFIAPGKRWNTRPMQASKLE
VGPQSDLHPQWRSRKAPWHIEGKIAAYMRQKGFTDGCVYLNARPCSGPDGCARNLPD
LLPVGSTLHVHARYIDRTGETRFYYREYRGTGKALT
>SEQ ID NO:75 Sdd4 
LDAMDAYLSEIAGGNAPARAGPKAPEPKQPGGSSSPRARDGRIDFRALLERLKAQ
GVVGLEGRSDDPIPDFDPKKQNPACYQGLAPRQKGKPVRGNLFFPDGRRWNDVALES
SRGEPAFDLNIIKPEYRSLSPARGHLEGNVAAWMRSTFHQEMVLYINESPCRKHGKGCL
YTLEHFLPRGYVLHVWSRNDRGEWRGNTFRGSGEAFTEGA
>SEQ ID NO:76 Sdd6 
VETRDKIIAAKSRSDAGLLAFQQATNGSIDSRPAEAIANLQRAKTHLDEAQRLVAN
SDAAVDNYINAILGGASAATAQPSAVIPASKPSRFKPMRTDPAKADEIRPHVGKDRAVAT
LWDADGNRVLGLHSADDDGPAATAAWKPPWRDYVRLRRHVEAHAAARMHQDGHKT
MVMYINLPPCKYFDGCKLNLEDILPKGSTLWMHRVFQNGGTKIYQFNGTGRAYV
>SEQ ID NO:77 Sdd7 
LEAVRARLIGEGGGPGAVPEGGDGPPAVPAEEVERLRGELPPPVVPGTGQKTHGR
WIGPDGRVRAIVSGRDEDAALVHAQLAAKGIPDEPTRNSDVEQKLAAHMVANGIRHV
 TLVINHRPCRGFDDSCDTLVPIILPEGCTLTVHGQTDKGMRVRVRYTGGARPWWS
>SEQ ID NO:78 Sdd9 
LDVDDPHTFYVLAGKTPVLVHNSECPIWVKNALQELVGRKETSGKVFDVDGKPI
GPDIQSGYKDRELRRGVYETLRKSPFFQKHFPSTATWYVSLHVEAQYAVWMLRNRIKH
ATVVINNTYVCSDMNRLHDNCMTAVPHILPEGYTMTVWKADRTEVTLRGKAPKE
>SEQ ID NO:79 Sdd10 
LDAALGAVRRIIAALGTSGAERASPGANGSERVDELAERLPPTVVPNTSAKTHGW
WFTGQGAAQELISGEGPDARAAYEALREEGYPRPGMPFVAMHVEIKLAAHMRRNDIE
HATVVINNIPCPLVWGCENLIGVVLPEGSSLTVHGSNGYERTFTGGRKPPWPR
>SEQ ID NO:80 Sdd59 
LLTPPPRPAAPPTTRPKPLVARTGDAYPPGTEWALPLIVQPHPPVGGTVPVEGHVRA
LRPESQISHVFHPGGGHWTEQARARLRVLPGFGWAVNLGHHVELQIAAWMTACGIHH
AELVLNRPPCGERYGLGCHQALPVLLPRGYRLTVSSTRGGPQPYQHHYEGKA
>SEQ ID NO:81 mini-Sdd3 
PRRFNPACWQGLKPYDQAGTAEGNLFIAPGKRWNTRPMQASKLEVGPQSDLHPQ
WRSRKAPWHIEGKIAAYMRQKGFTDGCVYLNARPCSGPDGCARNLPDLLPVGSTLHV
HARYIDRTGETRFYYREYRGTGKALT
>SEQ ID NO:82 mini-Sdd6 
PASKPSRFKPMRTDPAKADEIRPHVGKDRAVATLWDADGNRVLGLHSADDDGPAA
TAAWKPPWRDYVRLRRHVEAHAAARMHQDGHKTMVMYINLPPCKYFDGCKLNLED
ILPKGSTLWMHRVFQNGGTKIYQFNGTGRAYV
>SEQ ID NO:83 ecTadA 
MSEVEFSHEYWMRHALTLAKRAWDEREVPVGAVLVHNNRVIGEGWNRPIGRHD
PTAHAEIMALRQGGLVMQNYRLIDATLYVTLEPCVMCAGAMIHSRIGRVVFGARDAKT
GAAGSLMDVLHHPGMNHRVEITEGILADECAALLSDFFRMRRQEIKAQKKAQSSTD
>SEQ ID NO:84 mADA 
AQTPAFNKPKVELHVHLDGAIKPETILYFGKKRGIALPADTVEELRNIIGMDKPLSL
PGFLAKFDYYMPVIAGCREAIKRIAYEFVEMKAKEGVVYVEVRYSPHLLANSKVDPM
 
PWNQTEGDVTPDDVVDLVNQGLQEGEQAFGIKVRSILCCMRHQPSWSLEVLELCKKY
NQKTVVAMDLAGDETIEGSSLFPGHVEAYEGAVKNGIHRTVHAGEVGSPEVVREAVDI
LKTERVGHGYHTIEDEALYNRLLKENMHFEVCPWSSYLTGAWDPKTTHAVVRFKNDK
ANYSLNTDDPLIFKSTLDTDYQMTKKDMGFTEEEFKRLNINAAKSSFLPEEEKKELLER
LYREYQ
>SEQ ID NO:85 hADAR2 
HLDQTPSRQPIPSEGLQLHLPQVLADAVSRLVLGKFGDLTDNFSSPHARRKVLAGV
VMTTGTDVKDAKVISVSTGTKCINGEYMSDRGLALNDCHAEIISRRSLLRFLYTQLELY
LNNKDDQKRSIFQKSERGGFRLKENVQFHLYISTSPCGDARIFSPHEPILEEPADRHPNR
KARGQLRTKIESGEGTIPVRSNASIQTWDGVLQGERLLTMSCSDKIARWNVVGIQGSLL
SIFVEPIYFSSIILGSLYHGDHLSRAMYQRISNIEDLPPLYTLNKPLLSGISNAEARQPGKA
PNFSVNWTVGDSAIEVINATTGKDELGRASRLCKHALYCRWMRVHGKVPSHLLRSKIT
KPNVYHESKLAAKEYQAAKARLFTAFIKAGLGAWVEKPTEQDQFSLTP
>SEQ ID NO:86 hADAT2 
EAKAAPKPAASGACSVSAEETEKWMEEAMHMAKEALENTEVPVGCLMVYNNE
VVGKGRNEVNQTKNATRHAEMVAIDQVLDWCRQSGKSPSEVFEHTVLYVTVEPCIMC
AAALRLMKIPLVVYGCQNERFGGCGSVLNIASADLPNTGRPFQCIPGYRAEEAVEMLK
TFYKQENPNAPKSKVRKKECQKS
>SEQ ID NO:87 ecTadA*(TadA-7.10) 
SEVEFSHEYWMRHALTLAKRARDEREVPVGAVLVLNNRVIGEGWNRAIGLHDPT
AHAEIMALRQGGLVMQNYRLIDATLYVTFEPCVMCAGAMIHSRIGRVVFGVRNAKTG
AAGSLMDVLHYPGMNHRVEITEGILADECAALLCYFFRMPRQVFNAQKKAQSSTD
>SEQ ID NO:88 TadA*ABE8e (TadA-8e) 
SEVEFSHEYWMRHALTLAKRARDEREVPVGAVLVLNNRVIGEGWNRAIGLHDPT
AHAEIMALRQGGLVMQNYRLIDATLYVTFEPCVMCAGAMIHSRIGRVVFGVRNSKRG
AAGSLMNVLNYPGMNHRVEITEGILADECAALLCDFYRMPRQVFNAQKKAQSSIN
>SEQ ID NO:89 TadA-8e-QBE4 
MKRTADGSEFESPKKKRKVEIGKATAKYFFYSNIMNFFKTEITLANGEIRKRPLIET
NGETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPIDFL
 
EAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDGGSGGSGGSGGSGGSGGSGGMDKKYSIGLAIGTNSVGWAVITDEYKVPSK
KFKVLGNTDRHSIKKNLIGALLFDSGETAEATRLKRTARRRYTRRKNRICYLQEIFSNE
MAKVDDSFFHRLEESFLVEEDKKHERHPIFGNIVDEVAYHEKYPTIYHLRKKLVDSTDK
ADLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQLS
KDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTF
RIPYYVGPLARGNSRFAWMTRKSEETITPWNFEEVVDKGASAQSFIERMTNFDKNLPN
EKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSIDNK
VLTRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSESG
GSSGGSSGSETPGTSESATPESSGGSSGGSSEVEFSHEYWMRHALTLAKRARDEREVPV
GAVLVLNNRVIGEGWNRAIGLHDPTAHAEIMALRQGGLVMQNYRLIDATLYVTFEPCV
MCAGAMIHSRIGRVVFGVRNSKRGAAGSLMNVLNYPGMNHRVEITEGILADECAALL
CDFYRMPRQVFNAQKKAQSSINSGGSSGGSSGSETPGTSESATPESSGGSSGGSQSGGS
KRTADGSEFEPKKKRKV
>SEQ ID NO:90 TadA-8e-QBE1 
MKRTADGSEFESPKKKRKVNIMNFFKTEITLANGEIRKRPLIETNGETGEIVWDKG
RDFATVRKVLSMPQVNIVKKTEVQTGGFSKESILPKRNSDKLIARKKDWDPKKYGGFD
SPTVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGDGGSGG
SGGSGGSGGSGGSGGMDKKYSIGLAIGTNSVGWAVITDEYKVPSKKFKVLGNTDRHSI
 
KKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHM
IKFRGHFLIEGDLNPDNSDVDKLFIQLVQTYNQLFEENPINASGVDAKAILSARLSKSRR
LENLIAQLPGEKKNGLFGNLIALSLGLTPNFKSNFDLAEDAKLQLSKDTYDDDLDNLL
AQIGDQYADLFLAAKNLSDAILLSDILRVNTEITKAPLSASMIKRYDEHHQDLTLLKALV
RQQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLARGN
SRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECF
DSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVM
GRHKPENIVIEMARENQTTQKGQKNSRERMKRIEEGIKELGSQILKEHPVENTQLQNE
KLYLYYLQNGRDMYVDQELDINRLSDYDVDHIVPQSFLKDDSIDNKVLTRSDKNRGKS
DNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSESGGSSGGSSGSETPG
TSESATPESSGGSSGGSSEVEFSHEYWMRHALTLAKRARDEREVPVGAVLVLNNRVIGE
GWNRAIGLHDPTAHAEIMALRQGGLVMQNYRLIDATLYVTFEPCVMCAGAMIHSRIGR
VVFGVRNSKRGAAGSLMNVLNYPGMNHRVEITEGILADECAALLCDFYRMPRQVFNA
QKKAQSSINSGGSSGGSSGSETPGTSESATPESSGGSSGGSQEIGKATAKYFFYSSGGSK
RTADGSEFEPKKKRKV
>SEQ ID NO:91 TadA-8e-QBE2 
MKRTADGSEFESPKKKRKVARLSKSRRLENLIAQLPGEKKNGLFGNLIALSLGLTP
NFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQE
DFYPFLKDNREKIEKILTFRIPYYVGPLARGNSRFAWMTRKSEETITPWNFEEVVDKGA
SAQSFIERMTNFDKNLPNEKVLPKHSLLYEYFTVYNELTKVKYVTEGMRKPAFLSGEQ
KKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSL
HEHIANLAGSPAIKKGILQTVKVVDELVKVMGRHKPENIVIEMARENQTTQKGQKNSR
ERMKRIEEGIKELGSQILKEHPVENTQLQNEKLYLYYLQNGRDMYVDQELDINRLSDY
 
DVDHIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVY
GDYKVYDVRKMIAKSEQEIGKATAKYFFYSNIMNFFKTEITLANGEISGGSSGGSSGSE
TPGTSESATPESSGGSSGGSSEVEFSHEYWMRHALTLAKRARDEREVPVGAVLVLNNR
VIGEGWNRAIGLHDPTAHAEIMALRQGGLVMQNYRLIDATLYVTFEPCVMCAGAMIH
SRIGRVVFGVRNSKRGAAGSLMNVLNYPGMNHRVEITEGILADECAALLCDFYRMPR
QVFNAQKKAQSSINSGGSSGGSSGSETPGTSESATPESSGGSSGGSRKRPLIETNGETGEI
VWDKGRDFATVRKVLSMPQVNIVKKTEVQTGGFSKESILPKRNSDKLIARKKDWDPK
KYGGFDSPTVAYSVLVVAKVEKGKSKKLKSVKELLGITIMERSSFEKNPIDFLEAKGYK
EVKKDLIIKLPKYSLFELENGRKRMLASAGELQKGNELALPSKYVNFLYLASHYEKLK
GSPEDNEQKQLFVEQHKHYLDEIIEQISEFSKRVILADANLDKVLSAYNKHRDKPIREQ
AENIIHLFTLTNLGAPAAFKYFDTTIDRKRYTSTKEVLDATLIHQSITGLYETRIDLSQLG
GDGGSGGSGGSGGSGGSGGSGGMDKKYSIGLAIGTNSVGWAVITDEYKVPSKKFKVL
GNTDRHSIKKNLIGALLFDSGETAEATRLKRTARRRYTRRKNRICYLQEIFSNEMAKVD
DSFFHRLEESFLVEEDKKHERHPIFGNIVDEVAYHEKYPTIYHLRKKLVDSTDKADLRLI
YLALAHMIKFRGHFLIEGDLNPDNSDVDKLFIQLVQTYNQLFEENPINASGVDAKAILS
SGGSKRTADGSEFEPKKKRKV
>SEQ ID NO:92 TadA-8e-QBE3 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSSEVEFSHEYWMRHALTLAKRARDER
EVPVGAVLVLNNRVIGEGWNRAIGLHDPTAHAEIMALRQGGLVMQNYRLIDATLYVTF
EPCVMCAGAMIHSRIGRVVFGVRNSKRGAAGSLMNVLNYPGMNHRVEITEGILADEC
AALLCDFYRMPRQVFNAQKKAQSSINSGGSSGGSSGSETPGTSESATPESSGGSSGGSL
KGSPEDNEQKQLFVEQHKHYLDEIIEQISEFSKRVILADANLDKVLSAYNKHRDKPIRE
QAENIIHLFTLTNLGAPAAFKYFDTTIDRKRYTSTKEVLDATLIHQSITGLYETRIDLSQL
GGDGGSGGSGGSGGSGGSGGSGGMDKKYSIGLAIGTNSVGWAVITDEYKVPSKKFKV
LGNTDRHSIKKNLIGALLFDSGETAEATRLKRTARRRYTRRKNRICYLQEIFSNEMAKV
DDSFFHRLEESFLVEEDKKHERHPIFGNIVDEVAYHEKYPTIYHLRKKLVDSTDKADLR
LIYLALAHMIKFRGHFLIEGDLNPDNSDVDKLFIQLVQTYNQLFEENPINASGVDAKAIL
SARLSKSRRLENLIAQLPGEKKNGLFGNLIALSLGLTPNFKSNFDLAEDAKLQLSKDTY
 
DDDLDNLLAQIGDQYADLFLAAKNLSDAILLSDILRVNTEITKAPLSASMIKRYDEHHQ
DLTLLKALVRQQLPEKYKEIFFDQSKNGYAGYIDGGASQEEFYKFIKPILEKMDGTEEL
LVKLNREDLLRKQRTFDNGSIPHQIHLGELHAILRRQEDFYPFLKDNREKIEKILTFRIPY
YVGPLARGNSRFAWMTRKSEETITPWNFEEVVDKGASAQSFIERMTNFDKNLPNEKVL
PKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFE
DREMIEERLKTYAHLFDDKVMKQLKRRRYTGWGRLSRKLINGIRDKQSGKTILDFLKS
DGFANRNFMQLIHDDSLTFKEDIQKAQVSGQGDSLHEHIANLAGSPAIKKGILQTVKVV
DELVKVMGRHKPENIVIEMARENQTTQKGQKNSRERMKRIEEGIKELGSQILKEHPVE
NTQLQNEKLYLYYLQNGRDMYVDQELDINRLSDYDVDHIVPQSFLKDDSIDNKVLTRS
DKNRGKSDNVPSEEVVKKMKNYWRQLLNAKLITQRKFDNLTKAERGGLSELDKAGFI
KRQLVETRQITKHVAQILDSRMNTKYDENDKLIREVKVITLKSKLVSDFRKDFQFYKVR
EINNYHHAHDAYLNAVVGTALIKKYPKLESEFVYGDYKVYDVRKMIAKSEQEIGSGGS
KRTADGSEFEPKKKRKV
>SEQ ID NO.93 Sdd7-QBE4 
MKRTADGSEFESPKKKRKVEIGKATAKYFFYSNIMNFFKTEITLANGEIRKRPLIET
NGETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDGGSGGSGGSGGSGGSGGSGGMDKKYSIGLAIGTNSVGWAVITDEYKVPSK
KFKVLGNTDRHSIKKNLIGALLFDSGETAEATRLKRTARRRYTRRKNRICYLQEIFSNE
MAKVDDSFFHRLEESFLVEEDKKHERHPIFGNIVDEVAYHEKYPTIYHLRKKLVDSTDK
ADLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQLS
KDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTF
RIPYYVGPLARGNSRFAWMTRKSEETITPWNFEEVVDKGASAQSFIERMTNFDKNLPN
EKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSDGFANRNFMQLIHDDSLTFKEDIQKAQVSGQGDSLHEHIANLAGSPAIKKGILQT
 
VKVVDELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSIDNK
VLTRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSESG
GSSGGSSGSETPGTSESATPESSGGSSGGSLEAVRARLIGEGGGPGAVPEGGDGPPAVPA
EEVERLRGELPPPVVPGTGQKTHGRWIGPDGRVRAIVSGRDEDAALVHAQLAAKGIPD
EPTRNSDVEQKLAAHMVANGIRHVTLVINHRPCRGFDDSCDTLVPIILPEGCTLTVHGQ
TDKGMRVRVRYTGGARPWWSSGGSSGGSSGSETPGTSESATPESSGGSSGGSQSGGSG
GSGGSTNLSDIIEKETGKQLVIQESILMLPEEVEEVIGNKPESDILVHTAYDESTDENVML
LTSDAPEYKPWALVIQDSNGENKIKMLSGGSGGSGGSTNLSDIIEKETGKQLVIQESILM
LPEEVEEVIGNKPESDILVHTAYDESTDENVMLLTSDAPEYKPWALVIQDSNGENKIKM
LSGGSKRTADGSEFEPKKKRKV
>SEQ ID NO.94 Sdd7-QBE1 
MKRTADGSEFESPKKKRKVNIMNFFKTEITLANGEIRKRPLIETNGETGEIVWDKG
RDFATVRKVLSMPQVNIVKKTEVQTGGFSKESILPKRNSDKLIARKKDWDPKKYGGFD
SPTVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGDGGSGG
SGGSGGSGGSGGSGGMDKKYSIGLAIGTNSVGWAVITDEYKVPSKKFKVLGNTDRHSI
KKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHM
IKFRGHFLIEGDLNPDNSDVDKLFIQLVQTYNQLFEENPINASGVDAKAILSARLSKSRR
LENLIAQLPGEKKNGLFGNLIALSLGLTPNFKSNFDLAEDAKLQLSKDTYDDDLDNLL
AQIGDQYADLFLAAKNLSDAILLSDILRVNTEITKAPLSASMIKRYDEHHQDLTLLKALV
RQQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLARGN
SRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECF
DSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVM
GRHKPENIVIEMARENQTTQKGQKNSRERMKRIEEGIKELGSQILKEHPVENTQLQNE
 
KLYLYYLQNGRDMYVDQELDINRLSDYDVDHIVPQSFLKDDSIDNKVLTRSDKNRGKS
DNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSESGGSSGGSSGSETPG
TSESATPESSGGSSGGSLEAVRARLIGEGGGPGAVPEGGDGPPAVPAEEVERLRGELPPP
VVPGTGQKTHGRWIGPDGRVRAIVSGRDEDAALVHAQLAAKGIPDEPTRNSDVEQKL
AAHMVANGIRHVTLVINHRPCRGFDDSCDTLVPIILPEGCTLTVHGQTDKGMRVRVRYT
GGARPWWSSGGSSGGSSGSETPGTSESATPESSGGSSGGSQEIGKATAKYFFYSSGGSG
GSGGSTNLSDIIEKETGKQLVIQESILMLPEEVEEVIGNKPESDILVHTAYDESTDENVML
LTSDAPEYKPWALVIQDSNGENKIKMLSGGSGGSGGSTNLSDIIEKETGKQLVIQESILM
LPEEVEEVIGNKPESDILVHTAYDESTDENVMLLTSDAPEYKPWALVIQDSNGENKIKM
LSGGSKRTADGSEFEPKKKRKV
>SEQ ID NO:95 rAPOBEC1-QBE4 
MKRTADGSEFESPKKKRKVEIGKATAKYFFYSNIMNFFKTEITLANGEIRKRPLIET
NGETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDGGSGGSGGSGGSGGSGGSGGMDKKYSIGLAIGTNSVGWAVITDEYKVPSK
KFKVLGNTDRHSIKKNLIGALLFDSGETAEATRLKRTARRRYTRRKNRICYLQEIFSNE
MAKVDDSFFHRLEESFLVEEDKKHERHPIFGNIVDEVAYHEKYPTIYHLRKKLVDSTDK
ADLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQLS
KDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTF
RIPYYVGPLARGNSRFAWMTRKSEETITPWNFEEVVDKGASAQSFIERMTNFDKNLPN
EKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSIDNK
 
VLTRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSESG
GSSGGSSGSETPGTSESATPESSGGSSGGSSSETGPVAVDPTLRRRIEPHEFEVFFDPRELR
KETCLLYEINWGGRHSIWRHTSQNTNKHVEVNFIEKFTTERYFCPNTRCSITWFLSWSP
CGECSRAITEFLSRYPHVTLFIYIARLYHHADPRNRQGLRDLISSGVTIQIMTEQESGYC
WRNFVNYSPSNEAHWPRYPHLWVRLYVLELYCIILGLPPCLNILRRKQPQLTFFTIALQS
CHYQRLPPHILWATGLKSGGSSGGSSGSETPGTSESATPESSGGSSGGSQSGGSGGSGGS
TNLSDIIEKETGKQLVIQESILMLPEEVEEVIGNKPESDILVHTAYDESTDENVMLLTSDA
PEYKPWALVIQDSNGENKIKMLSGGSGGSGGSTNLSDIIEKETGKQLVIQESILMLPEEV
EEVIGNKPESDILVHTAYDESTDENVMLLTSDAPEYKPWALVIQDSNGENKIKMLSGGS
KRTADGSEFEPKKKRKV
>SEQ ID NO:96 rAPOBEC1-QBE1 
MKRTADGSEFESPKKKRKVNIMNFFKTEITLANGEIRKRPLIETNGETGEIVWDKG
RDFATVRKVLSMPQVNIVKKTEVQTGGFSKESILPKRNSDKLIARKKDWDPKKYGGFD
SPTVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGDGGSGG
SGGSGGSGGSGGSGGMDKKYSIGLAIGTNSVGWAVITDEYKVPSKKFKVLGNTDRHSI
KKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHM
IKFRGHFLIEGDLNPDNSDVDKLFIQLVQTYNQLFEENPINASGVDAKAILSARLSKSRR
LENLIAQLPGEKKNGLFGNLIALSLGLTPNFKSNFDLAEDAKLQLSKDTYDDDLDNLL
AQIGDQYADLFLAAKNLSDAILLSDILRVNTEITKAPLSASMIKRYDEHHQDLTLLKALV
RQQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLARGN
SRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECF
DSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVM
GRHKPENIVIEMARENQTTQKGQKNSRERMKRIEEGIKELGSQILKEHPVENTQLQNE
KLYLYYLQNGRDMYVDQELDINRLSDYDVDHIVPQSFLKDDSIDNKVLTRSDKNRGKS
 
DNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSESGGSSGGSSGSETPG
TSESATPESSGGSSGGSSSETGPVAVDPTLRRRIEPHEFEVFFDPRELRKETCLLYEINWG
GRHSIWRHTSQNTNKHVEVNFIEKFTTERYFCPNTRCSITWFLSWSPCGECSRAITEFLS
RYPHVTLFIYIARLYHHADPRNRQGLRDLISSGVTIQIMTEQESGYCWRNFVNYSPSNE
AHWPRYPHLWVRLYVLELYCIILGLPPCLNILRRKQPQLTFFTIALQSCHYQRLPPHILW
ATGLKSGGSSGGSSGSETPGTSESATPESSGGSSGGSQEIGKATAKYFFYSSGGSGGSGG
STNLSDIIEKETGKQLVIQESILMLPEEVEEVIGNKPESDILVHTAYDESTDENVMLLTSD
APEYKPWALVIQDSNGENKIKMLSGGSGGSGGSTNLSDIIEKETGKQLVIQESILMLPEE
VEEVIGNKPESDILVHTAYDESTDENVMLLTSDAPEYKPWALVIQDSNGENKIKMLSGG
SKRTADGSEFEPKKKRKV
>SEQ ID NO:97 mini-Sdd3-QBE4 
MKRTADGSEFESPKKKRKVEIGKATAKYFFYSNIMNFFKTEITLANGEIRKRPLIET
NGETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDGGSGGSGGSGGSGGSGGSGGMDKKYSIGLAIGTNSVGWAVITDEYKVPSK
KFKVLGNTDRHSIKKNLIGALLFDSGETAEATRLKRTARRRYTRRKNRICYLQEIFSNE
MAKVDDSFFHRLEESFLVEEDKKHERHPIFGNIVDEVAYHEKYPTIYHLRKKLVDSTDK
ADLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQLS
KDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTF
RIPYYVGPLARGNSRFAWMTRKSEETITPWNFEEVVDKGASAQSFIERMTNFDKNLPN
EKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSIDNK
 
VLTRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSESG
GSSGGSSGSETPGTSESATPESSGGSSGGSPRRFNPACWQGLKPYDQAGTAEGNLFIAP
GKRWNTRPMQASKLEVGPQSDLHPQWRSRKAPWHIEGKIAAYMRQKGFTDGCVYLN
ARPCSGPDGCARNLPDLLPVGSTLHVHARYIDRTGETRFYYREYRGTGKALTSGGSSG
GSSGSETPGTSESATPESSGGSSGGSQSGGSGGSGGSTNLSDIIEKETGKQLVIQESILML
PEEVEEVIGNKPESDILVHTAYDESTDENVMLLTSDAPEYKPWALVIQDSNGENKIKML
SGGSGGSGGSTNLSDIIEKETGKQLVIQESILMLPEEVEEVIGNKPESDILVHTAYDESTD
ENVMLLTSDAPEYKPWALVIQDSNGENKIKMLSGGSKRTADGSEFEPKKKRKV
>SEQ ID NO:98 mini-Sdd3(G133R) -QBE4 
MKRTADGSEFESPKKKRKVEIGKATAKYFFYSNIMNFFKTEITLANGEIRKRPLIET
NGETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDGGSGGSGGSGGSGGSGGSGGMDKKYSIGLAIGTNSVGWAVITDEYKVPSK
KFKVLGNTDRHSIKKNLIGALLFDSGETAEATRLKRTARRRYTRRKNRICYLQEIFSNE
MAKVDDSFFHRLEESFLVEEDKKHERHPIFGNIVDEVAYHEKYPTIYHLRKKLVDSTDK
ADLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQLS
KDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTF
RIPYYVGPLARGNSRFAWMTRKSEETITPWNFEEVVDKGASAQSFIERMTNFDKNLPN
EKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSIDNK
VLTRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDFQ
 
FYKVREINNYHHAHDAYLNAVVGTALIKKYPKLESEFVYGDYKVYDVRKMIAKSESG
GSSGGSSGSETPGTSESATPESSGGSSGGSPRRFNPACWQGLKPYDQAGTAEGNLFIAP
GKRWNTRPMQASKLEVGPQSDLHPQWRSRKAPWHIEGKIAAYMRQKGFTDGCVYLN
ARPCSGPDGCARNLPDLLPVGSTLHVHARYIDRTGETRFYYREYRGTRKALTSGGSSG
GSSGSETPGTSESATPESSGGSSGGSQSGGSGGSGGSTNLSDIIEKETGKQLVIQESILML
PEEVEEVIGNKPESDILVHTAYDESTDENVMLLTSDAPEYKPWALVIQDSNGENKIKML
SGGSGGSGGSTNLSDIIEKETGKQLVIQESILMLPEEVEEVIGNKPESDILVHTAYDESTD
ENVMLLTSDAPEYKPWALVIQDSNGENKIKMLSGGSKRTADGSEFEPKKKRKV
>SEQ ID NO:99 mini-Sdd3-QBE1 
MKRTADGSEFESPKKKRKVNIMNFFKTEITLANGEIRKRPLIETNGETGEIVWDKG
RDFATVRKVLSMPQVNIVKKTEVQTGGFSKESILPKRNSDKLIARKKDWDPKKYGGFD
SPTVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGDGGSGG
SGGSGGSGGSGGSGGMDKKYSIGLAIGTNSVGWAVITDEYKVPSKKFKVLGNTDRHSI
KKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHM
IKFRGHFLIEGDLNPDNSDVDKLFIQLVQTYNQLFEENPINASGVDAKAILSARLSKSRR
LENLIAQLPGEKKNGLFGNLIALSLGLTPNFKSNFDLAEDAKLQLSKDTYDDDLDNLL
AQIGDQYADLFLAAKNLSDAILLSDILRVNTEITKAPLSASMIKRYDEHHQDLTLLKALV
RQQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLARGN
SRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECF
DSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVM
GRHKPENIVIEMARENQTTQKGQKNSRERMKRIEEGIKELGSQILKEHPVENTQLQNE
KLYLYYLQNGRDMYVDQELDINRLSDYDVDHIVPQSFLKDDSIDNKVLTRSDKNRGKS
DNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSESGGSSGGSSGSETPG
TSESATPESSGGSSGGSPRRFNPACWQGLKPYDQAGTAEGNLFIAPGKRWNTRPMQAS
 
KLEVGPQSDLHPQWRSRKAPWHIEGKIAAYMRQKGFTDGCVYLNARPCSGPDGCAR
NLPDLLPVGSTLHVHARYIDRTGETRFYYREYRGTGKALTSGGSSGGSSGSETPGTSES
ATPESSGGSSGGSQEIGKATAKYFFYSSGGSGGSGGSTNLSDIIEKETGKQLVIQESILML
PEEVEEVIGNKPESDILVHTAYDESTDENVMLLTSDAPEYKPWALVIQDSNGENKIKML
SGGSGGSGGSTNLSDIIEKETGKQLVIQESILMLPEEVEEVIGNKPESDILVHTAYDESTD
ENVMLLTSDAPEYKPWALVIQDSNGENKIKMLSGGSKRTADGSEFEPKKKRKV
>SEQ ID NO:100 mini-Sdd6-QBE4 
MKRTADGSEFESPKKKRKVEIGKATAKYFFYSNIMNFFKTEITLANGEIRKRPLIET
NGETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDGGSGGSGGSGGSGGSGGSGGMDKKYSIGLAIGTNSVGWAVITDEYKVPSK
KFKVLGNTDRHSIKKNLIGALLFDSGETAEATRLKRTARRRYTRRKNRICYLQEIFSNE
MAKVDDSFFHRLEESFLVEEDKKHERHPIFGNIVDEVAYHEKYPTIYHLRKKLVDSTDK
ADLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQLS
KDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTF
RIPYYVGPLARGNSRFAWMTRKSEETITPWNFEEVVDKGASAQSFIERMTNFDKNLPN
EKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSIDNK
VLTRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSESG
GSSGGSSGSETPGTSESATPESSGGSSGGSPASKPSRFKPMRTDPAKADEIRPHVGKDRA
VATLWDADGNRVLGLHSADDDGPAATAAWKPPWRDYVRLRRHVEAHAAARMHQDG
HKTMVMYINLPPCKYFDGCKLNLEDILPKGSTLWMHRVFQNGGTKIYQFNGTGRAYV
 
SGGSSGGSSGSETPGTSESATPESSGGSSGGSQSGGSGGSGGSTNLSDIIEKETGKQLVIQ
ESILMLPEEVEEVIGNKPESDILVHTAYDESTDENVMLLTSDAPEYKPWALVIQDSNGEN
KIKMLSGGSGGSGGSTNLSDIIEKETGKQLVIQESILMLPEEVEEVIGNKPESDILVHTAY
DESTDENVMLLTSDAPEYKPWALVIQDSNGENKIKMLSGGSKRTADGSEFEPKKKRKV
>SEQ ID NO:101 mini-Sdd6-QBE1 
MKRTADGSEFESPKKKRKVNIMNFFKTEITLANGEIRKRPLIETNGETGEIVWDKG
RDFATVRKVLSMPQVNIVKKTEVQTGGFSKESILPKRNSDKLIARKKDWDPKKYGGFD
SPTVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGDGGSGG
SGGSGGSGGSGGSGGMDKKYSIGLAIGTNSVGWAVITDEYKVPSKKFKVLGNTDRHSI
KKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHM
IKFRGHFLIEGDLNPDNSDVDKLFIQLVQTYNQLFEENPINASGVDAKAILSARLSKSRR
LENLIAQLPGEKKNGLFGNLIALSLGLTPNFKSNFDLAEDAKLQLSKDTYDDDLDNLL
AQIGDQYADLFLAAKNLSDAILLSDILRVNTEITKAPLSASMIKRYDEHHQDLTLLKALV
RQQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLARGN
SRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECF
DSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVM
GRHKPENIVIEMARENQTTQKGQKNSRERMKRIEEGIKELGSQILKEHPVENTQLQNE
KLYLYYLQNGRDMYVDQELDINRLSDYDVDHIVPQSFLKDDSIDNKVLTRSDKNRGKS
DNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSESGGSSGGSSGSETPG
TSESATPESSGGSSGGSPASKPSRFKPMRTDPAKADEIRPHVGKDRAVATLWDADGNRV
LGLHSADDDGPAATAAWKPPWRDYVRLRRHVEAHAAARMHQDGHKTMVMYINLPP
CKYFDGCKLNLEDILPKGSTLWMHRVFQNGGTKIYQFNGTGRAYVSGGSSGGSSGSET
PGTSESATPESSGGSSGGSQEIGKATAKYFFYSSGGSGGSGGSTNLSDIIEKETGKQLVIQ
ESILMLPEEVEEVIGNKPESDILVHTAYDESTDENVMLLTSDAPEYKPWALVIQDSNGEN
 
KIKMLSGGSGGSGGSTNLSDIIEKETGKQLVIQESILMLPEEVEEVIGNKPESDILVHTAY
DESTDENVMLLTSDAPEYKPWALVIQDSNGENKIKMLSGGSKRTADGSEFEPKKKRKV
>SEQ ID NO:102 hAPOBEC3A-QBE4 
MKRTADGSEFESPKKKRKVEIGKATAKYFFYSNIMNFFKTEITLANGEIRKRPLIET
NGETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDGGSGGSGGSGGSGGSGGSGGMDKKYSIGLAIGTNSVGWAVITDEYKVPSK
KFKVLGNTDRHSIKKNLIGALLFDSGETAEATRLKRTARRRYTRRKNRICYLQEIFSNE
MAKVDDSFFHRLEESFLVEEDKKHERHPIFGNIVDEVAYHEKYPTIYHLRKKLVDSTDK
ADLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQLS
KDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTF
RIPYYVGPLARGNSRFAWMTRKSEETITPWNFEEVVDKGASAQSFIERMTNFDKNLPN
EKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSIDNK
VLTRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSESG
GSSGGSSGSETPGTSESATPESSGGSSGGSEASPASGPRHLMDPHIFTSNFNNGIGRHKT
YLCYEVERLDNGTSVKMDQHRGFLHNQAKNLLCGFYGRHAELRFLDLVPSLQLDPAQ
IYRVTWFISWSPCFSWGCAGEVRAFLQENTHVRLRIFAARIYDYDPLYKEALQMLRDA
GAQVSIMTYDEFKHCWDTFVDHQGCPFQPWDGLDEHSQALSGRLRAILQNQGNSGGS
SGGSSGSETPGTSESATPESSGGSSGGSQSGGSGGSGGSTNLSDIIEKETGKQLVIQESIL
MLPEEVEEVIGNKPESDILVHTAYDESTDENVMLLTSDAPEYKPWALVIQDSNGENKIK
MLSGGSGGSGGSTNLSDIIEKETGKQLVIQESILMLPEEVEEVIGNKPESDILVHTAYDES
 TDENVMLLTSDAPEYKPWALVIQDSNGENKIKMLSGGSKRTADGSEFEPKKKRKV
>SEQ ID NO:103 hAPOBEC3A-QBE1 
MKRTADGSEFESPKKKRKVNIMNFFKTEITLANGEIRKRPLIETNGETGEIVWDKG
RDFATVRKVLSMPQVNIVKKTEVQTGGFSKESILPKRNSDKLIARKKDWDPKKYGGFD
SPTVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGDGGSGG
SGGSGGSGGSGGSGGMDKKYSIGLAIGTNSVGWAVITDEYKVPSKKFKVLGNTDRHSI
KKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHM
IKFRGHFLIEGDLNPDNSDVDKLFIQLVQTYNQLFEENPINASGVDAKAILSARLSKSRR
LENLIAQLPGEKKNGLFGNLIALSLGLTPNFKSNFDLAEDAKLQLSKDTYDDDLDNLL
AQIGDQYADLFLAAKNLSDAILLSDILRVNTEITKAPLSASMIKRYDEHHQDLTLLKALV
RQQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLARGN
SRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECF
DSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVM
GRHKPENIVIEMARENQTTQKGQKNSRERMKRIEEGIKELGSQILKEHPVENTQLQNE
KLYLYYLQNGRDMYVDQELDINRLSDYDVDHIVPQSFLKDDSIDNKVLTRSDKNRGKS
DNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSESGGSSGGSSGSETPG
TSESATPESSGGSSGGSEASPASGPRHLMDPHIFTSNFNNGIGRHKTYLCYEVERLDNGT
SVKMDQHRGFLHNQAKNLLCGFYGRHAELRFLDLVPSLQLDPAQIYRVTWFISWSPCF
SWGCAGEVRAFLQENTHVRLRIFAARIYDYDPLYKEALQMLRDAGAQVSIMTYDEFK
HCWDTFVDHQGCPFQPWDGLDEHSQALSGRLRAILQNQGNSGGSSGGSSGSETPGTS
ESATPESSGGSSGGSQEIGKATAKYFFYSSGGSGGSGGSTNLSDIIEKETGKQLVIQESIL
MLPEEVEEVIGNKPESDILVHTAYDESTDENVMLLTSDAPEYKPWALVIQDSNGENKIK
MLSGGSGGSGGSTNLSDIIEKETGKQLVIQESILMLPEEVEEVIGNKPESDILVHTAYDES
TDENVMLLTSDAPEYKPWALVIQDSNGENKIKMLSGGSKRTADGSEFEPKKKRKV
>SEQ ID NO:104 mini-Sdd9-QBE4 
MKRTADGSEFESPKKKRKVEIGKATAKYFFYSNIMNFFKTEITLANGEIRKRPLIET
NGETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDGGSGGSGGSGGSGGSGGSGGMDKKYSIGLAIGTNSVGWAVITDEYKVPSK
KFKVLGNTDRHSIKKNLIGALLFDSGETAEATRLKRTARRRYTRRKNRICYLQEIFSNE
MAKVDDSFFHRLEESFLVEEDKKHERHPIFGNIVDEVAYHEKYPTIYHLRKKLVDSTDK
ADLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQLS
KDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTF
RIPYYVGPLARGNSRFAWMTRKSEETITPWNFEEVVDKGASAQSFIERMTNFDKNLPN
EKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSIDNK
VLTRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSESG
GSSGGSSGSETPGTSESATPESSGGSSGGSCPIWVKNALQELVGRKETSGKVFDVDGKPI
GPDIQSGYKDRELRRGVYETLRKSPFFQKHFPSTATWYVSLHVEAQYAVWMLRNRIKH
ATVVINNTYVCSDMNRLHDNCMTAVPHILPEGYTMTVWKADRTEVTLRGKAPKESGG
SSGGSSGSETPGTSESATPESSGGSSGGSQSGGSGGSGGSTNLSDIIEKETGKQLVIQESIL
MLPEEVEEVIGNKPESDILVHTAYDESTDENVMLLTSDAPEYKPWALVIQDSNGENKIK
MLSGGSGGSGGSTNLSDIIEKETGKQLVIQESILMLPEEVEEVIGNKPESDILVHTAYDES
TDENVMLLTSDAPEYKPWALVIQDSNGENKIKMLSGGSKRTADGSEFEPKKKRKV
>SEQ ID NO:105 mini-Sdd9-QBE1 
MKRTADGSEFESPKKKRKVNIMNFFKTEITLANGEIRKRPLIETNGETGEIVWDKG
RDFATVRKVLSMPQVNIVKKTEVQTGGFSKESILPKRNSDKLIARKKDWDPKKYGGFD
SPTVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGDGGSGG
SGGSGGSGGSGGSGGMDKKYSIGLAIGTNSVGWAVITDEYKVPSKKFKVLGNTDRHSI
KKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHM
IKFRGHFLIEGDLNPDNSDVDKLFIQLVQTYNQLFEENPINASGVDAKAILSARLSKSRR
LENLIAQLPGEKKNGLFGNLIALSLGLTPNFKSNFDLAEDAKLQLSKDTYDDDLDNLL
AQIGDQYADLFLAAKNLSDAILLSDILRVNTEITKAPLSASMIKRYDEHHQDLTLLKALV
RQQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLARGN
SRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECF
DSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVM
GRHKPENIVIEMARENQTTQKGQKNSRERMKRIEEGIKELGSQILKEHPVENTQLQNE
KLYLYYLQNGRDMYVDQELDINRLSDYDVDHIVPQSFLKDDSIDNKVLTRSDKNRGKS
DNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSESGGSSGGSSGSETPG
TSESATPESSGGSSGGSCPIWVKNALQELVGRKETSGKVFDVDGKPIGPDIQSGYKDRE
LRRGVYETLRKSPFFQKHFPSTATWYVSLHVEAQYAVWMLRNRIKHATVVINNTYVCS
DMNRLHDNCMTAVPHILPEGYTMTVWKADRTEVTLRGKAPKESGGSSGGSSGSETPG
TSESATPESSGGSSGGSQEIGKATAKYFFYSSGGSGGSGGSTNLSDIIEKETGKQLVIQESI
LMLPEEVEEVIGNKPESDILVHTAYDESTDENVMLLTSDAPEYKPWALVIQDSNGENKI
KMLSGGSGGSGGSTNLSDIIEKETGKQLVIQESILMLPEEVEEVIGNKPESDILVHTAYDE
STDENVMLLTSDAPEYKPWALVIQDSNGENKIKMLSGGSKRTADGSEFEPKKKRKV
>SEQ ID NO:106 rAPOBEC1-QBE2 
MKRTADGSEFESPKKKRKVARLSKSRRLENLIAQLPGEKKNGLFGNLIALSLGLTP
NFKSNFDLAEDAKLQLSKDTYDDDLDNLLAQIGDQYADLFLAAKNLSDAILLSDILRV
 
NTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQE
DFYPFLKDNREKIEKILTFRIPYYVGPLARGNSRFAWMTRKSEETITPWNFEEVVDKGA
SAQSFIERMTNFDKNLPNEKVLPKHSLLYEYFTVYNELTKVKYVTEGMRKPAFLSGEQ
KKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSL
HEHIANLAGSPAIKKGILQTVKVVDELVKVMGRHKPENIVIEMARENQTTQKGQKNSR
ERMKRIEEGIKELGSQILKEHPVENTQLQNEKLYLYYLQNGRDMYVDQELDINRLSDY
DVDHIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVY
GDYKVYDVRKMIAKSEQEIGKATAKYFFYSNIMNFFKTEITLANGEISGGSSGGSSGSE
TPGTSESATPESSGGSSGGSSSETGPVAVDPTLRRRIEPHEFEVFFDPRELRKETCLLYEIN
WGGRHSIWRHTSQNTNKHVEVNFIEKFTTERYFCPNTRCSITWFLSWSPCGECSRAITE
FLSRYPHVTLFIYIARLYHHADPRNRQGLRDLISSGVTIQIMTEQESGYCWRNFVNYSPS
NEAHWPRYPHLWVRLYVLELYCIILGLPPCLNILRRKQPQLTFFTIALQSCHYQRLPPHIL
WATGLKSGGSSGGSSGSETPGTSESATPESSGGSSGGSRKRPLIETNGETGEIVWDKGR
DFATVRKVLSMPQVNIVKKTEVQTGGFSKESILPKRNSDKLIARKKDWDPKKYGGFDS
PTVAYSVLVVAKVEKGKSKKLKSVKELLGITIMERSSFEKNPIDFLEAKGYKEVKKDLII
KLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGDGGSGG
SGGSGGSGGSGGSGGMDKKYSIGLAIGTNSVGWAVITDEYKVPSKKFKVLGNTDRHSI
KKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHM
IKFRGHFLIEGDLNPDNSDVDKLFIQLVQTYNQLFEENPINASGVDAKAILSSGGSGGSG
GSTNLSDIIEKETGKQLVIQESILMLPEEVEEVIGNKPESDILVHTAYDESTDENVMLLTS
DAPEYKPWALVIQDSNGENKIKMLSGGSGGSGGSTNLSDIIEKETGKQLVIQESILMLPE
EVEEVIGNKPESDILVHTAYDESTDENVMLLTSDAPEYKPWALVIQDSNGENKIKMLSG
GSKRTADGSEFEPKKKRKV
>SEQ ID NO:107 rAPOBEC1-QBE3 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
 
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSSSETGPVAVDPTLRRRIEPHEFEVFFD
PRELRKETCLLYEINWGGRHSIWRHTSQNTNKHVEVNFIEKFTTERYFCPNTRCSITWF
LSWSPCGECSRAITEFLSRYPHVTLFIYIARLYHHADPRNRQGLRDLISSGVTIQIMTEQE
SGYCWRNFVNYSPSNEAHWPRYPHLWVRLYVLELYCIILGLPPCLNILRRKQPQLTFFTI
ALQSCHYQRLPPHILWATGLKSGGSSGGSSGSETPGTSESATPESSGGSSGGSLKGSPED
NEQKQLFVEQHKHYLDEIIEQISEFSKRVILADANLDKVLSAYNKHRDKPIREQAENIIH
LFTLTNLGAPAAFKYFDTTIDRKRYTSTKEVLDATLIHQSITGLYETRIDLSQLGGDGGS
GGSGGSGGSGGSGGSGGMDKKYSIGLAIGTNSVGWAVITDEYKVPSKKFKVLGNTDR
HSIKKNLIGALLFDSGETAEATRLKRTARRRYTRRKNRICYLQEIFSNEMAKVDDSFFHR
LEESFLVEEDKKHERHPIFGNIVDEVAYHEKYPTIYHLRKKLVDSTDKADLRLIYLALA
HMIKFRGHFLIEGDLNPDNSDVDKLFIQLVQTYNQLFEENPINASGVDAKAILSARLSK
SRRLENLIAQLPGEKKNGLFGNLIALSLGLTPNFKSNFDLAEDAKLQLSKDTYDDDLDN
LLAQIGDQYADLFLAAKNLSDAILLSDILRVNTEITKAPLSASMIKRYDEHHQDLTLLKA
LVRQQLPEKYKEIFFDQSKNGYAGYIDGGASQEEFYKFIKPILEKMDGTEELLVKLNRE
DLLRKQRTFDNGSIPHQIHLGELHAILRRQEDFYPFLKDNREKIEKILTFRIPYYVGPLAR
GNSRFAWMTRKSEETITPWNFEEVVDKGASAQSFIERMTNFDKNLPNEKVLPKHSLLY
EYFTVYNELTKVKYVTEGMRKPAFLSGEQKKAIVDLLFKTNRKVTVKQLKEDYFKKIE
CFDSVEISGVEDRFNASLGTYHDLLKIIKDKDFLDNEENEDILEDIVLTLTLFEDREMIEE
RLKTYAHLFDDKVMKQLKRRRYTGWGRLSRKLINGIRDKQSGKTILDFLKSDGFANR
NFMQLIHDDSLTFKEDIQKAQVSGQGDSLHEHIANLAGSPAIKKGILQTVKVVDELVKV
MGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGK
SDNVPSEEVVKKMKNYWRQLLNAKLITQRKFDNLTKAERGGLSELDKAGFIKRQLVE
TRQITKHVAQILDSRMNTKYDENDKLIREVKVITLKSKLVSDFRKDFQFYKVREINNYH
HAHDAYLNAVVGTALIKKYPKLESEFVYGDYKVYDVRKMIAKSEQEIGSGGSGGSGG
STNLSDIIEKETGKQLVIQESILMLPEEVEEVIGNKPESDILVHTAYDESTDENVMLLTSD
APEYKPWALVIQDSNGENKIKMLSGGSGGSGGSTNLSDIIEKETGKQLVIQESILMLPEE
VEEVIGNKPESDILVHTAYDESTDENVMLLTSDAPEYKPWALVIQDSNGENKIKMLSGG
SKRTADGSEFEPKKKRKV
>SEQ ID NO:108 mini-Sdd3-QBE2 
MKRTADGSEFESPKKKRKVARLSKSRRLENLIAQLPGEKKNGLFGNLIALSLGLTP
NFKSNFDLAEDAKLQLSKDTYDDDLDNLLAQIGDQYADLFLAAKNLSDAILLSDILRV
 
NTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQE
DFYPFLKDNREKIEKILTFRIPYYVGPLARGNSRFAWMTRKSEETITPWNFEEVVDKGA
SAQSFIERMTNFDKNLPNEKVLPKHSLLYEYFTVYNELTKVKYVTEGMRKPAFLSGEQ
KKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSL
HEHIANLAGSPAIKKGILQTVKVVDELVKVMGRHKPENIVIEMARENQTTQKGQKNSR
ERMKRIEEGIKELGSQILKEHPVENTQLQNEKLYLYYLQNGRDMYVDQELDINRLSDY
DVDHIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVY
GDYKVYDVRKMIAKSEQEIGKATAKYFFYSNIMNFFKTEITLANGEISGGSSGGSSGSE
TPGTSESATPESSGGSSGGSPRRFNPACWQGLKPYDQAGTAEGNLFIAPGKRWNTRPM
QASKLEVGPQSDLHPQWRSRKAPWHIEGKIAAYMRQKGFTDGCVYLNARPCSGPDGC
ARNLPDLLPVGSTLHVHARYIDRTGETRFYYREYRGTGKALTSGGSSGGSSGSETPGTS
ESATPESSGGSSGGSRKRPLIETNGETGEIVWDKGRDFATVRKVLSMPQVNIVKKTEVQ
TGGFSKESILPKRNSDKLIARKKDWDPKKYGGFDSPTVAYSVLVVAKVEKGKSKKLKS
VKELLGITIMERSSFEKNPIDFLEAKGYKEVKKDLIIKLPKYSLFELENGRKRMLASAGE
LQKGNELALPSKYVNFLYLASHYEKLKGSPEDNEQKQLFVEQHKHYLDEIIEQISEFSK
RVILADANLDKVLSAYNKHRDKPIREQAENIIHLFTLTNLGAPAAFKYFDTTIDRKRYTS
TKEVLDATLIHQSITGLYETRIDLSQLGGDGGSGGSGGSGGSGGSGGSGGMDKKYSIGL
AIGTNSVGWAVITDEYKVPSKKFKVLGNTDRHSIKKNLIGALLFDSGETAEATRLKRTA
RRRYTRRKNRICYLQEIFSNEMAKVDDSFFHRLEESFLVEEDKKHERHPIFGNIVDEVAY
HEKYPTIYHLRKKLVDSTDKADLRLIYLALAHMIKFRGHFLIEGDLNPDNSDVDKLFIQ
LVQTYNQLFEENPINASGVDAKAILSSGGSGGSGGSTNLSDIIEKETGKQLVIQESILMLP
EEVEEVIGNKPESDILVHTAYDESTDENVMLLTSDAPEYKPWALVIQDSNGENKIKMLS
GGSGGSGGSTNLSDIIEKETGKQLVIQESILMLPEEVEEVIGNKPESDILVHTAYDESTDE
NVMLLTSDAPEYKPWALVIQDSNGENKIKMLSGGSKRTADGSEFEPKKKRKV
>SEQ ID NO:109 mini-Sdd3-QBE3 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
 
EKSGGSSGGSSGSETPGTSESATPESSGGSSGGSPRRFNPACWQGLKPYDQAGTAEGNL
FIAPGKRWNTRPMQASKLEVGPQSDLHPQWRSRKAPWHIEGKIAAYMRQKGFTDGCV
YLNARPCSGPDGCARNLPDLLPVGSTLHVHARYIDRTGETRFYYREYRGTGKALTSGG
SSGGSSGSETPGTSESATPESSGGSSGGSLKGSPEDNEQKQLFVEQHKHYLDEIIEQISEF
SKRVILADANLDKVLSAYNKHRDKPIREQAENIIHLFTLTNLGAPAAFKYFDTTIDRKRY
TSTKEVLDATLIHQSITGLYETRIDLSQLGGDGGSGGSGGSGGSGGSGGSGGMDKKYSI
GLAIGTNSVGWAVITDEYKVPSKKFKVLGNTDRHSIKKNLIGALLFDSGETAEATRLKR
TARRRYTRRKNRICYLQEIFSNEMAKVDDSFFHRLEESFLVEEDKKHERHPIFGNIVDEV
AYHEKYPTIYHLRKKLVDSTDKADLRLIYLALAHMIKFRGHFLIEGDLNPDNSDVDKLF
IQLVQTYNQLFEENPINASGVDAKAILSARLSKSRRLENLIAQLPGEKKNGLFGNLIALS
LGLTPNFKSNFDLAEDAKLQLSKDTYDDDLDNLLAQIGDQYADLFLAAKNLSDAILLS
DILRVNTEITKAPLSASMIKRYDEHHQDLTLLKALVRQQLPEKYKEIFFDQSKNGYAGYI
DGGASQEEFYKFIKPILEKMDGTEELLVKLNREDLLRKQRTFDNGSIPHQIHLGELHAIL
RRQEDFYPFLKDNREKIEKILTFRIPYYVGPLARGNSRFAWMTRKSEETITPWNFEEVV
DKGASAQSFIERMTNFDKNLPNEKVLPKHSLLYEYFTVYNELTKVKYVTEGMRKPAFL
SGEQKKAIVDLLFKTNRKVTVKQLKEDYFKKIECFDSVEISGVEDRFNASLGTYHDLL
KIIKDKDFLDNEENEDILEDIVLTLTLFEDREMIEERLKTYAHLFDDKVMKQLKRRRYT
GWGRLSRKLINGIRDKQSGKTILDFLKSDGFANRNFMQLIHDDSLTFKEDIQKAQVSGQ
GDSLHEHIANLAGSPAIKKGILQTVKVVDELVKVMGRHKPENIVIEMARENQTTQKGQ
KNSRERMKRIEEGIKELGSQILKEHPVENTQLQNEKLYLYYLQNGRDMYVDQELDINR
LSDYDVDHIVPQSFLKDDSIDNKVLTRSDKNRGKSDNVPSEEVVKKMKNYWRQLLNA
KLITQRKFDNLTKAERGGLSELDKAGFIKRQLVETRQITKHVAQILDSRMNTKYDEND
KLIREVKVITLKSKLVSDFRKDFQFYKVREINNYHHAHDAYLNAVVGTALIKKYPKLES
EFVYGDYKVYDVRKMIAKSEQEIGSGGSGGSGGSTNLSDIIEKETGKQLVIQESILMLPE
EVEEVIGNKPESDILVHTAYDESTDENVMLLTSDAPEYKPWALVIQDSNGENKIKMLSG
GSGGSGGSTNLSDIIEKETGKQLVIQESILMLPEEVEEVIGNKPESDILVHTAYDESTDEN
VMLLTSDAPEYKPWALVIQDSNGENKIKMLSGGSKRTADGSEFEPKKKRKV
>SEQ ID NO:110 mini-Sdd9-QBE2 
MKRTADGSEFESPKKKRKVARLSKSRRLENLIAQLPGEKKNGLFGNLIALSLGLTP
NFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQE
DFYPFLKDNREKIEKILTFRIPYYVGPLARGNSRFAWMTRKSEETITPWNFEEVVDKGA
SAQSFIERMTNFDKNLPNEKVLPKHSLLYEYFTVYNELTKVKYVTEGMRKPAFLSGEQ
 
KKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSL
HEHIANLAGSPAIKKGILQTVKVVDELVKVMGRHKPENIVIEMARENQTTQKGQKNSR
ERMKRIEEGIKELGSQILKEHPVENTQLQNEKLYLYYLQNGRDMYVDQELDINRLSDY
DVDHIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVY
GDYKVYDVRKMIAKSEQEIGKATAKYFFYSNIMNFFKTEITLANGEISGGSSGGSSGSE
TPGTSESATPESSGGSSGGSCPIWVKNALQELVGRKETSGKVFDVDGKPIGPDIQSGYK
DRELRRGVYETLRKSPFFQKHFPSTATWYVSLHVEAQYAVWMLRNRIKHATVVINNTY
VCSDMNRLHDNCMTAVPHILPEGYTMTVWKADRTEVTLRGKAPKESGGSSGGSSGSE
TPGTSESATPESSGGSSGGSRKRPLIETNGETGEIVWDKGRDFATVRKVLSMPQVNIVK
KTEVQTGGFSKESILPKRNSDKLIARKKDWDPKKYGGFDSPTVAYSVLVVAKVEKGKS
KKLKSVKELLGITIMERSSFEKNPIDFLEAKGYKEVKKDLIIKLPKYSLFELENGRKRML
ASAGELQKGNELALPSKYVNFLYLASHYEKLKGSPEDNEQKQLFVEQHKHYLDEIIEQI
SEFSKRVILADANLDKVLSAYNKHRDKPIREQAENIIHLFTLTNLGAPAAFKYFDTTIDR
KRYTSTKEVLDATLIHQSITGLYETRIDLSQLGGDGGSGGSGGSGGSGGSGGSGGMDK
KYSIGLAIGTNSVGWAVITDEYKVPSKKFKVLGNTDRHSIKKNLIGALLFDSGETAEAT
RLKRTARRRYTRRKNRICYLQEIFSNEMAKVDDSFFHRLEESFLVEEDKKHERHPIFGNI
VDEVAYHEKYPTIYHLRKKLVDSTDKADLRLIYLALAHMIKFRGHFLIEGDLNPDNSD
VDKLFIQLVQTYNQLFEENPINASGVDAKAILSSGGSGGSGGSTNLSDIIEKETGKQLVI
QESILMLPEEVEEVIGNKPESDILVHTAYDESTDENVMLLTSDAPEYKPWALVIQDSNGE
NKIKMLSGGSGGSGGSTNLSDIIEKETGKQLVIQESILMLPEEVEEVIGNKPESDILVHTA
YDESTDENVMLLTSDAPEYKPWALVIQDSNGENKIKMLSGGSKRTADGSEFEPKKKRK
V
>SEQ ID NO:111 mini-Sdd9-QBE3 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSCPIWVKNALQELVGRKETSGKVFDV
DGKPIGPDIQSGYKDRELRRGVYETLRKSPFFQKHFPSTATWYVSLHVEAQYAVWMLR
NRIKHATVVINNTYVCSDMNRLHDNCMTAVPHILPEGYTMTVWKADRTEVTLRGKAP
 
KESGGSSGGSSGSETPGTSESATPESSGGSSGGSLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDGGSGGSGGSGGSGGSGGSGGM
DKKYSIGLAIGTNSVGWAVITDEYKVPSKKFKVLGNTDRHSIKKNLIGALLFDSGETAE
ATRLKRTARRRYTRRKNRICYLQEIFSNEMAKVDDSFFHRLEESFLVEEDKKHERHPIF
GNIVDEVAYHEKYPTIYHLRKKLVDSTDKADLRLIYLALAHMIKFRGHFLIEGDLNPDN
SDVDKLFIQLVQTYNQLFEENPINASGVDAKAILSARLSKSRRLENLIAQLPGEKKNGL
FGNLIALSLGLTPNFKSNFDLAEDAKLQLSKDTYDDDLDNLLAQIGDQYADLFLAAKN
LSDAILLSDILRVNTEITKAPLSASMIKRYDEHHQDLTLLKALVRQQLPEKYKEIFFDQS
KNGYAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKSEETIT
PWNFEEVVDKGASAQSFIERMTNFDKNLPNEKVLPKHSLLYEYFTVYNELTKVKYVTE
GMRKPAFLSGEQKKAIVDLLFKTNRKVTVKQLKEDYFKKIECFDSVEISGVEDRFNAS
LGTYHDLLKIIKDKDFLDNEENEDILEDIVLTLTLFEDREMIEERLKTYAHLFDDKVMK
QLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMARE
NQTTQKGQKNSRERMKRIEEGIKELGSQILKEHPVENTQLQNEKLYLYYLQNGRDMY
VDQELDINRLSDYDVDHIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTAL
IKKYPKLESEFVYGDYKVYDVRKMIAKSEQEIGSGGSGGSGGSTNLSDIIEKETGKQLV
IQESILMLPEEVEEVIGNKPESDILVHTAYDESTDENVMLLTSDAPEYKPWALVIQDSNG
ENKIKMLSGGSGGSGGSTNLSDIIEKETGKQLVIQESILMLPEEVEEVIGNKPESDILVHT
AYDESTDENVMLLTSDAPEYKPWALVIQDSNGENKIKMLSGGSKRTADGSEFEPKKKR
KV
>SEQ ID NO:112 hAPOBEC3A-QBE2 
MKRTADGSEFESPKKKRKVARLSKSRRLENLIAQLPGEKKNGLFGNLIALSLGLTP
NFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQE
DFYPFLKDNREKIEKILTFRIPYYVGPLARGNSRFAWMTRKSEETITPWNFEEVVDKGA
SAQSFIERMTNFDKNLPNEKVLPKHSLLYEYFTVYNELTKVKYVTEGMRKPAFLSGEQ
KKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGR
 
LSRKLINGIRDKQSGKTILDFLKSDGFANRNFMQLIHDDSLTFKEDIQKAQVSGQGDSL
HEHIANLAGSPAIKKGILQTVKVVDELVKVMGRHKPENIVIEMARENQTTQKGQKNSR
ERMKRIEEGIKELGSQILKEHPVENTQLQNEKLYLYYLQNGRDMYVDQELDINRLSDY
DVDHIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVY
GDYKVYDVRKMIAKSEQEIGKATAKYFFYSNIMNFFKTEITLANGEISGGSSGGSSGSE
TPGTSESATPESSGGSSGGSEASPASGPRHLMDPHIFTSNFNNGIGRHKTYLCYEVERLD
NGTSVKMDQHRGFLHNQAKNLLCGFYGRHAELRFLDLVPSLQLDPAQIYRVTWFISW
SPCFSWGCAGEVRAFLQENTHVRLRIFAARIYDYDPLYKEALQMLRDAGAQVSIMTY
DEFKHCWDTFVDHQGCPFQPWDGLDEHSQALSGRLRAILQNQGNSGGSSGGSSGSET
PGTSESATPESSGGSSGGSRKRPLIETNGETGEIVWDKGRDFATVRKVLSMPQVNIVKK
TEVQTGGFSKESILPKRNSDKLIARKKDWDPKKYGGFDSPTVAYSVLVVAKVEKGKSK
KLKSVKELLGITIMERSSFEKNPIDFLEAKGYKEVKKDLIIKLPKYSLFELENGRKRMLA
SAGELQKGNELALPSKYVNFLYLASHYEKLKGSPEDNEQKQLFVEQHKHYLDEIIEQIS
EFSKRVILADANLDKVLSAYNKHRDKPIREQAENIIHLFTLTNLGAPAAFKYFDTTIDRK
RYTSTKEVLDATLIHQSITGLYETRIDLSQLGGDGGSGGSGGSGGSGGSGGSGGMDKK
YSIGLAIGTNSVGWAVITDEYKVPSKKFKVLGNTDRHSIKKNLIGALLFDSGETAEATRL
KRTARRRYTRRKNRICYLQEIFSNEMAKVDDSFFHRLEESFLVEEDKKHERHPIFGNIV
DEVAYHEKYPTIYHLRKKLVDSTDKADLRLIYLALAHMIKFRGHFLIEGDLNPDNSDV
DKLFIQLVQTYNQLFEENPINASGVDAKAILSSGGSGGSGGSTNLSDIIEKETGKQLVIQ
ESILMLPEEVEEVIGNKPESDILVHTAYDESTDENVMLLTSDAPEYKPWALVIQDSNGEN
KIKMLSGGSGGSGGSTNLSDIIEKETGKQLVIQESILMLPEEVEEVIGNKPESDILVHTAY
DESTDENVMLLTSDAPEYKPWALVIQDSNGENKIKMLSGGSKRTADGSEFEPKKKRKV
>SEQ ID NO:113 hAPOBEC3A-QBE3 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSEASPASGPRHLMDPHIFTSNFNNGIGR
HKTYLCYEVERLDNGTSVKMDQHRGFLHNQAKNLLCGFYGRHAELRFLDLVPSLQLD
PAQIYRVTWFISWSPCFSWGCAGEVRAFLQENTHVRLRIFAARIYDYDPLYKEALQML
RDAGAQVSIMTYDEFKHCWDTFVDHQGCPFQPWDGLDEHSQALSGRLRAILQNQGN
SGGSSGGSSGSETPGTSESATPESSGGSSGGSLKGSPEDNEQKQLFVEQHKHYLDEIIEQI
 
SEFSKRVILADANLDKVLSAYNKHRDKPIREQAENIIHLFTLTNLGAPAAFKYFDTTIDR
KRYTSTKEVLDATLIHQSITGLYETRIDLSQLGGDGGSGGSGGSGGSGGSGGMDK
KYSIGLAIGTNSVGWAVITDEYKVPSKKFKVLGNTDRHSIKKNLIGALLFDSGETAEAT
RLKRTARRRYTRRKNRICYLQEIFSNEMAKVDDSFFHRLEESFLVEEDKKHERHPIFGNI
VDEVAYHEKYPTIYHLRKKLVDSTDKADLRLIYLALAHMIKFRGHFLIEGDLNPDNSD
VDKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNLS
DAILLSDILRVNTEITKAPLSASMIKRYDEHHQDLTLLKALVRQQLPEKYKEIFFDQSKN
GYAGYIDGGASQEEFYKFIKPILEKMDGTEELLVKLNREDLLRKQRTFDNGSIPHQIHL
GELHAILRRQEDFYPFLKDNREKIEKILTFRIPYYVGPLARGNSRFAWMTRKSEETITPW
NFEEVVDKGASAQSFIERMTNFDKNLPNEKVLPKHSLLYEYFTVYNELTKVKYVTEG
MRKPAFLSGEQKKAIVDLLFKTNRKVTVKQLKEDYFKKIECFDSVEISGVEDRFNASL
GTYHDLLKIIKDKDFLDNEENEDILEDIVLTLTLFEDREMIEERLKTYAHLFDDKVMKQ
LKRRRYTGWGRLSRKLINGIRDKQSGKTILDFLKSDGFANRNFMQLIHDDSLTFKEDIQ
KAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYV
DQELDINRLSDYDVDHIVPQSFLKDDSIDNKVLTRSDKNRGKSDNVPSEEVVKKMKNY
WRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALI
KKYPKLESEFVYGDYKVYDVRKMIAKSEQEIGSGGSGGSGGSTNLSDIIEKETGKQLVI
QESILMLPEEVEEVIGNKPESDILVHTAYDESTDENVMLLTSDAPEYKPWALVIQDSNGE
NKIKMLSGGSGGSGGSTNLSDIIEKETGKQLVIQESILMLPEEVEEVIGNKPESDILVHTA
YDESTDENVMLLTSDAPEYKPWALVIQDSNGENKIKMLSGGSKRTADGSEFEPKKKRK
V
>SEQ ID NO:114 mini-Sdd6-QBE2 
MKRTADGSEFESPKKKRKVARLSKSRRLENLIAQLPGEKKNGLFGNLIALSLGLTP
NFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQE
DFYPFLKDNREKIEKILTFRIPYYVGPLARGNSRFAWMTRKSEETITPWNFEEVVDKGA
SAQSFIERMTNFDKNLPNEKVLPKHSLLYEYFTVYNELTKVKYVTEGMRKPAFLSGEQ
KKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSL
 
HEHIANLAGSPAIKKGILQTVKVVDELVKVMGRHKPENIVIEMARENQTTQKGQKNSR
ERMKRIEEGIKELGSQILKEHPVENTQLQNEKLYLYYLQNGRDMYVDQELDINRLSDY
DVDHIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVY
GDYKVYDVRKMIAKSEQEIGKATAKYFFYSNIMNFFKTEITLANGEISGGSSGGSSGSE
TPGTSESATPESSGGSSGGSPASKPSRFKPMRTDPAKADEIRPHVGKDRAVATLWDADG
NRVLGLHSADDDGPAATAAWKPPWRDYVRLRRHVEAHAARMHQDGHKTMVMYIN
LPPCKYFDGCKLNLEDILPKGSTLWMHRVFQNGGTKIYQFNGTGRAYVSGGSSGGSSG
SETPGTSESATPESSGGSSGGSRKRPLIETNGETGEIVWDKGRDFATVRKVLSMPQVNIV
KKTEVQTGGFSKESILPKRNSDKLIARKKDWDPKKYGGFDSPTVAYSVLVVAKVEKGK
SKKLKSVKELLGITIMERSSFEKNPIDFLEAKGYKEVKKDLIIKLPKYSLFELENGRKRM
LASAGELQKGNELALPSKYVNFLYLASHYEKLKGSPEDNEQKQLFVEQHKHYLDEIIE
QISEFSKRVILADANLDKVLSAYNKHRDKPIREQAENIIHLFTLTNLGAPAAFKYFDTTID
RKRYTSTKEVLDATLIHQSITGLYETRIDLSQLGGDGGSGGSGGSGGSGGSGGSGGMD
KKYSIGLAIGTNSVGWAVITDEYKVPSKKFKVLGNTDRHSIKKNLIGALLFDSGETAEA
TRLKRTARRRYTRRKNRICYLQEIFSNEMAKVDDSFFHRLEESFLVEEDKKHERHPIFG
NIVDEVAYHEKYPTIYHLRKKLVDSTDKADLRLIYLALAHMIKFRGHFLIEGDLNPDNS
DVDKLFIQLVQTYNQLFEENPINASGVDAKAILSSGGSGGSGGSTNLSDIIEKETGKQLV
IQESILMLPEEVEEVIGNKPESDILVHTAYDESTDENVMLLTSDAPEYKPWALVIQDSNG
ENKIKMLSGGSGGSGGSTNLSDIIEKETGKQLVIQESILMLPEEVEEVIGNKPESDILVHT
AYDESTDENVMLLTSDAPEYKPWALVIQDSNGENKIKMLSGGSKRTADGSEFEPKKKR
KV
>SEQ ID NO:115 mini-Sdd6-QBE3 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSPASKPSRFKPMRTDPAKADEIRPHVG
KDRAVATLWDADGNRVLGLHSADDDGPAATAAWKPPWRDYVRLRRHVEAHAAARM
HQDGHKTMVMYINLPPCKYFDGCKLNLEDILPKGSTLWMHRVFQNGGTKIYQFNGTG
RAYVSGGSSGGSSGSETPGTSESATPESSGGSSGGSLKGSPEDNEQKQLFVEQHKHYLD
EIIEQISEFSKRVILADANLDKVLSAYNKHRDKPIREQAENIIHLFTLTNLGAPAAFKYFD
TTIDRKRYTSTKEVLDATLIHQSITGLYETRIDLSQLGGDGGSGGSGGSGGSGGSGGSG
 
GMDKKYSIGLAIGTNSVGWAVITDEYKVPSKKFKVLGNTDRHSIKKNLIGALLFDSGET
AEATRLKRTARRRYTRRKNRICYLQEIFSNEMAKVDDSFFHRLEESFLVEEDKKHERHP
IFGNIVDEVAYHEKYPTIYHLRKKLVDSTDKADLRLIYLALAHMIKFRGHFLIEGDLNPD
NSDVDKLFIQLVQTYNQLFEENPINASGVDAKAILSARLSKSRRLENLIAQLPGEKKNG
LFGNLIALSLGLTPNFKSNFDLAEDAKLQLSKDTYDDDLDNLLAQIGDQYADLFLAAK
NLSDAILLSDILRVNTEITKAPLSASMIKRYDEHHQDLTLLKALVRQQLPEKYKEIFFDQ
SKNGYAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKSEETIT
PWNFEEVVDKGASAQSFIERMTNFDKNLPNEKVLPKHSLLYEYFTVYNELTKVKYVTE
GMRKPAFLSGEQKKAIVDLLFKTNRKVTVKQLKEDYFKKIECFDSVEISGVEDRFNAS
LGTYHDLLKIIKDKDFLDNEENEDILEDIVLTLTLFEDREMIEERLKTYAHLFDDKVMK
QLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMARE
NQTTQKGQKNSRERMKRIEEGIKELGSQILKEHPVENTQLQNEKLYLYYLQNGRDMY
VDQELDINRLSDYDVDHIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTAL
IKKYPKLESEFVYGDYKVYDVRKMIAKSEQEIGSGGSGGSGGSTNLSDIIEKETGKQLV
IQESILMLPEEVEEVIGNKPESDILVHTAYDESTDENVMLLTSDAPEYKPWALVIQDSNG
ENKIKMLSGGSGGSGGSTNLSDIIEKETGKQLVIQESILMLPEEVEEVIGNKPESDILVHT
AYDESTDENVMLLTSDAPEYKPWALVIQDSNGENKIKMLSGGSKRTADGSEFEPKKKR
KV
>SEQ ID NO:116 Sdd7(R37H) -QBE2 
MKRTADGSEFESPKKKRKVARLSKSRRLENLIAQLPGEKKNGLFGNLIALSLGLTP
NFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQE
DFYPFLKDNREKIEKILTFRIPYYVGPLARGNSRFAWMTRKSEETITPWNFEEVVDKGA
SAQSFIERMTNFDKNLPNEKVLPKHSLLYEYFTVYNELTKVKYVTEGMRKPAFLSGEQ
KKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSL
HEHIANLAGSPAIKKGILQTVKVVDELVKVMGRHKPENIVIEMARENQTTQKGQKNSR
ERMKRIEEGIKELGSQILKEHPVENTQLQNEKLYLYYLQNGRDMYVDQELDINRLSDY
 
DVDHIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVY
GDYKVYDVRKMIAKSEQEIGKATAKYFFYSNIMNFFKTEITLANGEISGGSSGGSSGSE
TPGTSESATPESSGGSSGGSLEAVRARLIGEGGGPGAVPEGGDGPPAVPAEEVERLHGEL
PPPVVPGTGQKTHGRWIGPDGRVRAIVSGRDEDAALVHAQLAAKGIPDEPTRNSDVEQ
KLAAHMVANGIRHVTLVINHRPCRGFDDSCDTLVPIILPEGCTLTVHGQTDKGMRVRVR
YTGGARPWWSSGGSSGGSSGSETPGTSESATPESSGGSSGGSRKRPLIETNGETGEIVW
DKGRDFATVRKVLSMPQVNIVKKTEVQTGGFSKESILPKRNSDKLIARKKDWDPKKY
GGFDSPTVAYSVLVVAKVEKGKSKKLKSVKELLGITIMERSSFEKNPIDFLEAKGYKEV
KKDLIIKLPKYSLFELENGRKRMLASAGELQKGNELALPSKYVNFLYLASHYEKLKGS
PEDNEQKQLFVEQHKHYLDEIIEQISEFSKRVILADANLDKVLSAYNKHRDKPIREQAE
NIIHLFTLTNLGAPAAFKYFDTTIDRKRYTSTKEVLDATLIHQSITGLYETRIDLSQLGGD
GGSGGSGGSGGSGGSGGSGGMDKKYSIGLAIGTNSVGWAVITDEYKVPSKKFKVLGN
TDRHSIKKNLIGALLFDSGETAEATRLKRTARRRYTRRKNRICYLQEIFSNEMAKVDDS
FFHRLEESFLVEEDKKHERHPIFGNIVDEVAYHEKYPTIYHLRKKLVDSTDKADLRLIYL
ALAHMIKFRGHFLIEGDLNPDNSDVDKLFIQLVQTYNQLFEENPINASGVDAKAILSSG
GSGGSGGSTNLSDIIEKETGKQLVIQESILMLPEEVEEVIGNKPESDILVHTAYDESTDEN
VMLLTSDAPEYKPWALVIQDSNGENKIKMLSGGSGGSGGSTNLSDIIEKETGKQLVIQE
SILMLPEEVEEVIGNKPESDILVHTAYDESTDENVMLLTSDAPEYKPWALVIQDSNGEN
KIKMLSGGSKRTADGSEFEPKKKRKV
>SEQ ID NO:117 Sdd7(V144M) -QBE2 
MKRTADGSEFESPKKKRKVARLSKSRRLENLIAQLPGEKKNGLFGNLIALSLGLTP
NFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQE
DFYPFLKDNREKIEKILTFRIPYYVGPLARGNSRFAWMTRKSEETITPWNFEEVVDKGA
SAQSFIERMTNFDKNLPNEKVLPKHSLLYEYFTVYNELTKVKYVTEGMRKPAFLSGEQ
KKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSL
HEHIANLAGSPAIKKGILQTVKVVDELVKVMGRHKPENIVIEMARENQTTQKGQKNSR
ERMKRIEEGIKELGSQILKEHPVENTQLQNEKLYLYYLQNGRDMYVDQELDINRLSDY
DVDHIVPQSFLKDDSIDNKVLTRSDKNRGKSDNVPSEEVVKKMKNYWRQLLNAKLIT
 
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVY
GDYKVYDVRKMIAKSEQEIGKATAKYFFYSNIMNFFKTEITLANGEISGGSSGGSSGSE
TPGTSESATPESSGGSSGGSLEAVRARLIGEGGGPGAVPEGGDGPPAVPAEEVERLRGEL
PPPVVPGTGQKTHGRWIGPDGRVRAIVSGRDEDAALVHAQLAAKGIPDEPTRNSDVEQ
KLAAHMVANGIRHVTLVINHRPCRGFDDSCDTLVPIILPEGCTLTMHGQTDKGMRVRV
RYTGGARPWWSSGGSSGGSSGSETPGTSESATPESSGGSSGGSRKRPLIETNGETGEIV
WDKGRDFATVRKVLSMPQVNIVKKTEVQTGGFSKESILPKRNSDKLIARKKDWDPKK
YGGFDSPTVAYSVLVVAKVEKGKSKKLKSVKELLGITIMERSSFEKNPIDFLEAKGYKE
VKKDLIIKLPKYSLFELENGRKRMLASAGELQKGNELALPSKYVNFLYLASHYEKLKG
SPEDNEQKQLFVEQHKHYLDEIIEQISEFSKRVILADANLDKVLSAYNKHRDKPIREQA
ENIIHLFTLTNLGAPAAFKYFDTTIDRKRYTSTKEVLDATLIHQSITGLYETRIDLSQLGG
DGGSGGSGGSGGSGGSGGSGGMDKKYSIGLAIGTNSVGWAVITDEYKVPSKKFKVLG
NTDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRLIY
LALAHMIKFRGHFLIEGDLNPDNSDVDKLFIQLVQTYNQLFEENPINASGVDAKAILSS
GGSGGSGGSTNLSDIIEKETGKQLVIQESILMLPEEVEEVIGNKPESDILVHTAYDESTDE
NVMLLTSDAPEYKPWALVIQDSNGENKIKMLSGGSGGSGGSTNLSDIIEKETGKQLVIQ
ESILMLPEEVEEVIGNKPESDILVHTAYDESTDENVMLLTSDAPEYKPWALVIQDSNGEN
KIKMLSGGSKRTADGSEFEPKKKRKV
>SEQ ID NO.118 Sdd7-QBE2 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSLEAVRARLIGEGGGPGAVPEGGDGPP
AVPAEEVERLRGELPPPVVPGTGQKTHGRWIGPDGRVRAIVSGRDEDAALVHAQLAAK
GIPDEPTRNSDVEQKLAAHMVANGIRHVTLVINHRPCRGFDDSCDTLVPIILPEGCTLTV
HGQTDKGMRVRVRYTGGARPWWSSGGSSGGSSGSETPGTSESATPESSGGSSGGSLKG
SPEDNEQKQLFVEQHKHYLDEIIEQISEFSKRVILADANLDKVLSAYNKHRDKPIREQA
ENIIHLFTLTNLGAPAAFKYFDTTIDRKRYTSTKEVLDATLIHQSITGLYETRIDLSQLGG
DGGSGGSGGSGGSGGSGGSGGMDKKYSIGLAIGTNSVGWAVITDEYKVPSKKFKVLG
NTDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRLIY
 
LALAHMIKFRGHFLIEGDLNPDNSDVDKLFIQLVQTYNQLFEENPINASGVDAKAILSA
RLSKSRRLENLIAQLPGEKKNGLFGNLIALSLGLTPNFKSNFDLAEDAKLQLSKDTYDD
DLDNLLAQIGDQYADLFLAAKNLSDAILLSDILRVNTEITKAPLSASMIKRYDEHHQDL
TLLKALVRQQLPEKYKEIFFDQSKNGYAGYIDGGASQEEFYKFIKPILEKMDGTEELLV
KLNREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLPK
HSLLYEYFTVYNELTKVKYVTEGMRKPAFLSGEQKKAIVDLLFKTNRKVTVKQLKED
YFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSD
GFANRNFMQLIHDDSLTFKEDIQKAQVSGQGDSLHEHIANLAGSPAIKKGILQTVKVVD
ELVKVMGRHKPENIVIEMARENQTTQKGQKNSRERMKRIEEGIKELGSQILKEHPVEN
TQLQNEKLYLYYLQNGRDMYVDQELDINRLSDYDVDHIVPQSFLKDDSIDNKVLTRSD
KNRGKSDNVPSEEVVKKMKNYWRQLLNAKLITQRKFDNLTKAERGGLSELDKAGFIK
RQLVETRQITKHVAQILDSRMNTKYDENDKLIREVKVITLKSKLVSDFRKDFQFYKVRE
INNYHHAHDAYLNAVVGTALIKKYPKLESEFVYGDYKVYDVRKMIAKSEQEIGSGGSG
GSGGSTNLSDIIEKETGKQLVIQESILMLPEEVEEVIGNKPESDILVHTAYDESTDENVML
LTSDAPEYKPWALVIQDSNGENKIKMLSGGSGGSGGSTNLSDIIEKETGKQLVIQESILM
LPEEVEEVIGNKPESDILVHTAYDESTDENVMLLTSDAPEYKPWALVIQDSNGENKIKM
LSGGSKRTADGSEFEPKKKRKV
>SEQ ID NO: 119 Linker
   GGSGGSGGSGGS
>SEQ ID NO: 120 Linker
   GGSGGSGGSGGSGGS
>SEQ ID NO:121 Linker
   GGSGGSGGSGGSGGSGGS
>SEQ ID NO:122 Linker
   GGSGGSGGSGGSGGSGGSGGS
>SEQ ID NO:123 ABE8e(ABE) 
MKRTADGSEFESPKKKRKVMDKKYSIGLAIGTNSVGWAVITDEYKVPSKKFKVLG
NTDRHSIKKNLIGALLFDSGETAEATRLKRTARRRYTRRKNRICYLQEIFSNEMAKVDD
 
SFFHRLEESFLVEEDKKHERHPIFGNIVDEVAYHEKYPTIYHLRKKLVDSTDKADLRLIY
LALAHMIKFRGHFLIEGDLNPDNSDVDKLFIQLVQTYNQLFEENPINASGVDAKAILSA
RLSKSRRLENLIAQLPGEKKNGLFGNLIALSLGLTPNFKSNFDLAEDAKLQLSKDTYDD
DLDNLLAQIGDQYADLFLAAKNLSDAILLSDILRVNTEITKAPLSASMIKRYDEHHQDL
TLLKALVRQQLPEKYKEIFFDQSKNGYAGYIDGGASQEEFYKFIKPILEKMDGTEELLV
KLNREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLPK
HSLLYEYFTVYNELTKVKYVTEGMRKPAFLSGEQKKAIVDLLFKTNRKVTVKQLKED
YFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSD
GFANRNFMQLIHDDSLTFKEDIQKAQVSGQGDSLHEHIANLAGSPAIKKGILQTVKVVD
ELVKVMGRHKPENIVIEMARENQTTQKGQKNSRERMKRIEEGIKELGSQILKEHPVEN
TQLQNEKLYLYYLQNGRDMYVDQELDINRLSDYDVDHIVPQSFLKDDSIDNKVLTRSD
KNRGKSDNVPSEEVVKKMKNYWRQLLNAKLITQRKFDNLTKAERGGLSELDKAGFIK
RQLVETRQITKHVAQILDSRMNTKYDENDKLIREVKVITLKSKLVSDFRKDFQFYKVRE
INNYHHAHDAYLNAVVGTALIKKYPKLESEFVYGDYKVYDVRKMIAKSEQEIGKATA
KYFFYSNIMNFFKTEITLANGEIRKRPLIETNGETGEIVWDKGRDFATVRKVLSMPQVNI
VKKTEVQTGGFSKESILPKRNSDKLIARKKDWDPKKYGGFDSPTVAYSVLVVAKVEKG
KSKKLKSVKELLGITIMERSSFEKNPIDFLEAKGYKEVKKDLIIKLPKYSLFELENGRKR
MLASAGELQKGNELALPSKYVNFLYLASHYEKLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDGSGGSSGGSSGSETPGTSESATPE
SSGGSSGGSSEVEFSHEYWMRHALTLAKRARDEREVPVGAVLVLNNRVIGEGWNRAI
GLHDPTAHAEIMALRQGGLVMQNYRLIDATLYVTFEPCVMCAGAMIHSRIGRVVFGVR
NSKRGAAGSLMNVLNYPGMNHRVEITEGILADECAALLCDFYRMPRQVFNAQKKAQ
SSINKRTADGSEFEPKKKRKV
>SEQ ID NO:124 UGI 
TNLSDIIEKETGKQLVIQESILMLPEEVEEVIGNKPESDILVHTAYDESTDENVMLLT
SDAPEYKPWALVIQDSNGENKIKML
>SEQ ID NO:125 rAPOBEC1-BE4max 
MKRTADGSEFESPKKKRKVSSETGPVAVDPTLRRRIEPHEFEVFFDPRELRKETCLL
YEINWGGRHSIWRHTSQNTNKHVEVNFIEKFTTERYFCPNTRCSITWFLSWSPCGECSR
AITEFLSRYPHVTLFIYIARLYHHADPRNRQGLRDLISSGVTIQIMTEQESGYCWRNFVN
 
YSPSNEAHWPRYPHLWVRLYVLELYCIILGLPPCLNILRRKQPQLTFFTIALQSCHYQRL
PPHILWATGLKSGGSSGGSSGSETPGTSESATPESSGGSSGGSDKKYSIGLAIGTNSVGW
AVITDEYKVPSKKFKVLGNTDRHSIKKNLIGALLFDSGETAEATRLKRTARRRYTRRKN
RICYLQEIFSNEMAKVDDSFFHRLEESFLVEEDKKHERHPIFGNIVDEVAYHEKYPTIYH
LRKKLVDSTDKADLRLIYLALAHMIKFRGHFLIEGDLNPDNSDVDKLFIQLVQTYNQLF
EENPINASGVDAKAILSARLSKSRRLENLIAQLPGEKKNGLFGNLIALSLGLTPNFKSNF
DLAEDAKLQLSKDTYDDDLDNLLAQIGDQYADLFLAAKNLSDAILLSDILRVNTEITKA
PLSASMIKRYDEHHQDLTLLKALVRQQLPEKYKEIFFDQSKNGYAGYIDGGASQEEFY
KFIKPILEKMDGTEELLVKLNREDLLRKQRTFDNGSIPHQIHLGELHAILRRQEDFYPFL
KDNREKIEKILTFRIPYYVGPLARGNSRFAWMTRKSEETITPWNFEEVVDKGASAQSFIE
RMTNFDKNLPNEKVLPKHSLLYEYFTVYNELTKVKYVTEGMRKPAFLSGEQKKAIVD
LLFKTNRKVTVKQLKEDYFKKIECFDSVEISGVEDRFNASLGTYHDLLKIIKDKDFLDN
EENEDILEDIVLTLTLFEDREMIEERLKTYAHLFDDKVMKQLKRRRYTGWGRLSRKLIN
GIRDKQSGKTILDFLKSDGFANRNFMQLIHDDSLTFKEDIQKAQVSGQGDSLHEHIANL
AGSPAIKKGILQTVKVVDELVKVMGRHKPENIVIEMARENQTTQKGQKNSRERMKRIE
EGIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDNL
TKAERGGLSELDKAGFIKRQLVETRQITKHVAQILDSRMNTKYDENDKLIREVKVITLK
SKLVSDFRKDFQFYKVREINNYHHAHDAYLNAVVGTALIKKYPKLESEFVYGDYKVY
DVRKMIAKSEQEIGKATAKYFFYSNIMNFFKTEITLANGEIRKRPLIETNGETGEIVWDK
GRDFATVRKVLSMPQVNIVKKTEVQTGGFSKESILPKRNSDKLIARKKDWDPKKYGGF
DSPTVAYSVLVVAKVEKGKSKKLKSVKELLGITIMERSSFEKNPIDFLEAKGYKEVKKD
LIIKLPKYSLFELENGRKRMLASAGELQKGNELALPSKYVNFLYLASHYEKLKGSPEDN
EQKQLFVEQHKHYLDEIIEQISEFSKRVILADANLDKVLSAYNKHRDKPIREQAENIIHL
FTLTNLGAPAAFKYFDTTIDRKRYTSTKEVLDATLIHQSITGLYETRIDLSQLGGDSGGS
GGSGGSTNLSDIIEKETGKQLVIQESILMLPEEVEEVIGNKPESDILVHTAYDESTDENVM
LLTSDAPEYKPWALVIQDSNGENKIKMLSGGSGGSGGSTNLSDIIEKETGKQLVIQESIL
MLPEEVEEVIGNKPESDILVHTAYDESTDENVMLLTSDAPEYKPWALVIQDSNGENKIK
MLSGGSKRTADGSEFEPKKKRKV
>SEQ ID NO:126 mini-Sdd3- BE4max 
MKRTADGSEFESPKKKRKVPRRFNPACWQGLKPYDQAGTAEGNLFIAPGKRWNT
RPMQASKLEVGPQSDLHPQWRSRKAPWHIEGKIAAYMRQKGFTDGCVYLNARPCSGP
DGCARNLPDLLPVGSTLHVHARYIDRTGETRFYYREYRGTGKALTSGGSSGGSSGSETP
GTSESATPESSGGSSGGSDKKYSIGLAIGTNSVGWAVITDEYKVPSKKFKVLGNTDRHSI
 
KKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHM
IKFRGHFLIEGDLNPDNSDVDKLFIQLVQTYNQLFEENPINASGVDAKAILSARLSKSRR
LENLIAQLPGEKKNGLFGNLIALSLGLTPNFKSNFDLAEDAKLQLSKDTYDDDLDNLL
AQIGDQYADLFLAAKNLSDAILLSDILRVNTEITKAPLSASMIKRYDEHHQDLTLLKALV
RQQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLARGN
SRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECF
DSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVM
GRHKPENIVIEMARENQTTQKGQKNSRERMKRIEEGIKELGSQILKEHPVENTQLQNE
KLYLYYLQNGRDMYVDQELDINRLSDYDVDHIVPQSFLKDDSIDNKVLTRSDKNRGKS
DNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSEQEIGKATAKYFFYSN
IMNFFKTEITLANGEIRKRPLIETNGETGEIVWDKGRDFATVRKVLSMPQVNIVKKTEV
QTGGFSKESILPKRNSDKLIARKKDWDPKKYGGFDSPTVAYSVLVVAKVEKGKSKKLK
SVKELLGITIMERSSFEKNPIDFLEAKGYKEVKKDLIIKLPKYSLFELENGRKRMLASAG
ELQKGNELALPSKYVNFLYLASHYEKLKGSPEDNEQKQLFVEQHKHYLDEIIEQISEFS
KRVILADANLDKVLSAYNKHRDKPIREQAENIIHLFTLTNLGAPAAFKYFDTTIDRKRYT
STKEVLDATLIHQSITGLYETRIDLSQLGGDSGGSGGSGGSTNLSDIIEKETGKQLVIQESI
LMLPEEVEEVIGNKPESDILVHTAYDESTDENVMLLTSDAPEYKPWALVIQDSNGENKI
KMLSGGSGGSGGSTNLSDIIEKETGKQLVIQESILMLPEEVEEVIGNKPESDILVHTAYDE
STDENVMLLTSDAPEYKPWALVIQDSNGENKIKMLSGGSKRTADGSEFEPKKKRKV
>SEQ ID NO:127 mini-Sdd7 
EGGGPGAVPEGGDGPPAVPAEEVERLRGELPPPVVPGTGQKTHGRWIGPDGRVRAI
VSGRDEDAALVHAQLAAKGIPDEPTRNSDVEQKLAAHMVANGIRHVTLVINHRPCRGF
DDSCDTLVPIILPEGCTLTVHGQTDKGMRVRVRYTGGARPWWS
>SEQ ID NO:128 mini-Sdd9 
CPIWVKNALQELVGRKETSGKVFDVDGKPIGPDIQSGYKDRELRRGVVYETLRKSP
FFQKHFPSTATWYVSLHVEAQYAVWMLRNRIKHATVVINNTYVCSDMNRLHDNCMTA
 VPHILPEGYTMTVWKADRTEVTLRGKAPKE
>SEQ ID NO:129 Site1-CTNNB1
   GACAAACCAGAAGCCGCTCC
>SEQ ID NO:130 Site2-GAPDH
   GTTCACACCCATGACGAACA
>SEQ ID NO:131 Site3-Homo sapiens VISTA enhancer hs267
   GAACACAAAGCATAGACTGC
>SEQ ID NO:132 Site5-HBG2
   gtggggaaggggcccccaag
>SEQ ID NO:133 Site7-RNF2
   GTCATCTTAGTCATTACCTG
>SEQ ID NO:134 Site8-CaMKIIδ
   TGCTTCCATGATGCACAGAC
>SEQ ID NO:135 Site9-BCL11A
   TTTTATCACAGGCTCCAGGA
>SEQ ID NO:136 Site10-HBG2
   CTTGACCAATAGCCTTGACA
>SEQ ID NO:137 Site11-ABCA4
   CGAAGTTCGCCCTGGAGAGG
>SEQ ID NO:138 Site12-CCR5
   AAGTGTGATCACTTGGGTGG
>SEQ ID NO:139 Site13-CCR5
   CAGGACGGTCACCTTTGGGG
>SEQ ID NO:140 Site15-CCR5
   GGTACCTATCGATTGTCAGG
>SEQ ID NO:141 Site17-LINC01509
   GGATTGACCCAGGCCAGGGC
>SEQ ID NO:142 Site19-CCR5
   AGCCAGGACGGTCACCTTTG
>SEQ ID NO:143 Site21-LINC01509
   CCAAGGATTGACCCAGGCCA
>SEQ ID NO:144 Site22-PCSK9
   CCACCGCTGCGCCAAGGTGC
>SEQ ID NO:145 Site29
   CCAAGTACTTCAGGTAGCTG
> SEQ ID NO:146 Linker 
SGGSSGGSSGSETPGTSESATPESSGGSSGGSSGGSSGGSSGSETPGTSESATPESSG
GSSGGS
> SEQ ID NO:147 Linker 
PGSPAGSPTSTEEGTSESATPESGPGTSTEPSEGSAPGSPAGSPTSTEEGTSTEPSEGS
APGTSTEPSEGSAPGTSESATPESGPGSEPATS
> SEQ ID NO:148 rice site1
   GACCTCCACGCCGCCGGCAATGG
> SEQ ID NO:149 rice site2
   GAGTGCGCGGCCATGGCGAGAGG
> SEQ ID NO:150 rice site3
   TCAGCAGGAATCTACCTTTGTGG
> SEQ ID NO:151 scaffold sequence of sgRNA:
   guuuuagagcuagaaauagcaaguuaaaauaaggcuaguccguuaucaacuugaaaaaguggcaccgagucggugc
>SEQ ID NO:152 A3A- BE4max 
MKRTADGSEFESPKKKRKVEASPASGPRHLMDPHIFTSNFNNGIGRHKTYLCYEV
ERLDNGTSVKMDQHRGFLHNQAKNLLCGFYGRHAELRFLDLVPSLQLDPAQIYRVTW
FISWSPCFSWGCAGEVRAFLQENTHVRLRIFAARIYDYDPLYKEALQMLRDAGAQVSI
MTYDEFKHCWDTFVDHQGCPFQPWDGLDEHSQALSGRLRAILQNQGNSGGSSGGSS
GSETPGTSESATPESSGGSSGGSDKKYSIGLAIGTNSVGWAVITDEYKVPSKKFKVLGNT
DRHSIKKNLIGALLFDSGETAEATRLKRTARRRYTRRKNRICYLQEIFSNEMAKVDDSFF
HRLEESFLVEEDKKHERHPIFGNIVDEVAYHEKYPTIYHLRKKLVDSTDKADLRLIYLAL
AHMIKFRGHFLIEGDLNPDNSDVDKLFIQLVQTYNQLFEENPINASGVDAKAILSARLS
KSRRLENLIAQLPGEKKNGLFGNLIALSLGLTPNFKSNFDLAEDAKLQLSKDTYDDDLD
NLLAQIGDQYADLFLAAKNLSDAILLSDILRVNTEITKAPLSASMIKRYDEHHQDLTLLK
ALVRQQLPEKYKEIFFDQSKNGYAGYIDGGASQEEFYKFIKPILEKMDGTEELLVKLNR
EDLLRKQRTFDNGSIPHQIHLGELHAILRRQEDFYPFLKDNREKIEKILTFRIPYYVGPLA
RGNSRFAWMTRKSEETITPWNFEEVVDKGASAQSFIERMTNFDKNLPNEKVLPKHSLL
YEYFTVYNELTKVKYVTEGMRKPAFLSGEQKKAIVDLLFKTNRKVTVKQLKEDYFKK
IECFDSVEISGVEDRFNASLGTYHDLLKIIKDKDFLDNEENEDILEDIVLTLTLFEDREMI
EERLKTYAHLFDDKVMKQLKRRRYTGWGRLSRKLINGIRDKQSGKTILDFLKSDGFAN
RNFMQLIHDDSLTFKEDIQKAQVSGQGDSLHEHIANLAGSPAIKKGILQTVKVVDELVK
VMGRHKPENIVIEMARENQTTQKGQKNSRERMKRIEEGIKELGSQILKEHPVENTQLQ
NEKLYLYYLQNGRDMYVDQELDINRLSDYDVDHIVPQSFLKDDSIDNKVLTRSDKNRG
KSDNVPSEEVVKKMKNYWRQLLNAKLITQRKFDNLTKAERGGLSELDKAGFIKRQLV
ETRQITKHVAQILDSRMNTKYDENDKLIREVKVITLKSKLVSDFRKDFQFYKVREINNY
HHAHDAYLNAVVGTALIKKYPKLESEFVYGDYKVYDVRKMIAKSEQEIGKATAKYFF
YSNIMNFFKTEITLANGEIRKRPLIETNGETGEIVWDKGRDFATVRKVLSMPQVNIVKK
TEVQTGGFSKESILPKRNSDKLIARKKDWDPKKYGGFDSPTVAYSVLVVAKVEKGKSK
KLKSVKELLGITIMERSSFEKNPIDFLEAKGYKEVKKDLIIKLPKYSLFELENGRKRMLA
SAGELQKGNELALPSKYVNFLYLASHYEKLKGSPEDNEQKQLFVEQHKHYLDEIIEQIS
EFSKRVILADANLDKVLSAYNKHRDKPIREQAENIIHLFTLTNLGAPAAFKYFDTTIDRK
RYTSTKEVLDATLIHQSITGLYETRIDLSQLGGDSGGSGGSGGSTNLSDIIEKETGKQLVI
QESILMLPEEVEEVIGNKPESDILVHTAYDESTDENVMLLTSDAPEYKPWALVIQDSNGE
NKIKMLSGGSGGSGGSTNLSDIIEKETGKQLVIQESILMLPEEVEEVIGNKPESDILVHTA
YDESTDENVMLLTSDAPEYKPWALVIQDSNGENKIKMLSGGSKRTADGSEFEPKKKRK
 V
>SEQ ID NO:153 mini-Sdd6- BE4max 
MKRTADGSEFESPKKKRKVPASKPSRFKPMRTDPAKADEIRPHVGKDRAVATLWD
ADGNRVLGLHSADDDGPAATAAWKPPWRDYVRLRRHVEAHAAARMHQDGHKTMV
MYINLPPCKYFDGCKLNLEDILPKGSTLWMHRVFQNGGTKIYQFNGTGRAYVSGGSSG
GSSGSETPGTSESATPESSGGSSGGSDKKYSIGLAIGTNSVGWAVITDEYKVPSKKFKVL
GNTDRHSIKKNLIGALLFDSGETAEATRLKRTARRRYTRRKNRICYLQEIFSNEMAKVD
DSFFHRLEESFLVEEDKKHERHPIFGNIVDEVAYHEKYPTIYHLRKKLVDSTDKADLRLI
YLALAHMIKFRGHFLIEGDLNPDNSDVDKLFIQLVQTYNQLFEENPINASGVDAKAILS
ARLSKSRRLENLIAQLPGEKKNGLFGNLIALSLGLTPNFKSNFDLAEDAKLQLSKDTYD
DDLDNLLAQIGDQYADLFLAAKNLSDAILLSDILRVNTEITKAPLSASMIKRYDEHHQD
LTLLKALVRQQLPEKYKEIFFDQSKNGYAGYIDGGASQEEFYKFIKPILEKMDGTEELL
VKLNREDLLRKQRTFDNGSIPHQIHLGELHAILRRQEDFYPFLKDNREKIEKILTFRIPYY
VGPLARGNSRFAWMTRKSEETITPWNFEEVVDKGASAQSFIERMTNFDKNLPNEKVLP
KHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFE
DREMIEERLKTYAHLFDDKVMKQLKRRRYTGWGRLSRKLINGIRDKQSGKTILDFLKS
DGFANRNFMQLIHDDSLTFKEDIQKAQVSGQGDSLHEHIANLAGSPAIKKGILQTVKVV
DELVKVMGRHKPENIVIEMARENQTTQKGQKNSRERMKRIEEGIKELGSQILKEHPVE
NTQLQNEKLYLYYLQNGRDMYVDQELDINRLSDYDVDHIVPQSFLKDDSIDNKVLTRS
DKNRGKSDNVPSEEVVKKMKNYWRQLLNAKLITQRKFDNLTKAERGGLSELDKAGFI
KRQLVETRQITKHVAQILDSRMNTKYDENDKLIREVKVITLKSKLVSDFRKDFQFYKVR
EINNYHHAHDAYLNAVVGTALIKKYPKLESEFVYGDYKVYDVRKMIAKSEQEIGKATA
KYFFYSNIMNFFKTEITLANGEIRKRPLIETNGETGEIVWDKGRDFATVRKVLSMPQVNI
VKKTEVQTGGFSKESILPKRNSDKLIARKKDWDPKKYGGFDSPTVAYSVLVVAKVEKG
KSKKLKSVKELLGITIMERSSFEKNPIDFLEAKGYKEVKKDLIIKLPKYSLFELENGRKR
MLASAGELQKGNELALPSKYVNFLYLASHYEKLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDSGGSGGSGGSTNLSDIIEKETGK
QLVIQESILMLPEEVEEVIGNKPESDILVHTAYDESTDENVMLLTSDAPEYKPWALVIQD
SNGENKIKMLSGGSGGSGGSTNLSDIIEKETGKQLVIQESILMLPEEVEEVIGNKPESDIL
VHTAYDESTDENVMLLTSDAPEYKPWALVIQDSNGENKIKMLSGGSKRTADGSEFEPK
KKRKV
>SEQ ID NO:154 mini-Sdd7- BE4max 
MKRTADGSEFESPKKKRKVEGGGPGAVPEGGDGPPAVPAEEVERLRGELPPPVVP
GTGQKTHGRWIGPDGRVRAIVSGRDEDAALVHAQLAAKGIPDEPTRNSDVEQKLAAH
MVANGIRHVTLVINHRPCRGFDDSCDTLVPIILPEGCTLTVHGQTDKGMRVRVRYTGGA
RPWWSSGGSSGGSSGSETPGTSESATPESSGGSSGGSDKKYSIGLAIGTNSVGWAVITDE
YKVPSKKFKVLGNTDRHSIKKNLIGALLFDSGETAEATRLKRTARRRYTRRKNRICYLQ
EIFSNEMAKVDDSFFHRLEESFLVEEDKKHERHPIFGNIVDEVAYHEKYPTIYHLRKKLV
DSTDKADLRLIYLALAHMIKFRGHFLIEGDLNPDNSDVDKLFIQLVQTYNQLFEENPIN
ASGVDAKAILSARLSKSRRLENLIAQLPGEKKNGLFGNLIALSLGLTPNFKSNFDLAED
AKLQLSKDTYDDDLDNLLAQIGDQYADLFLAAKNLSDAILLSDILRVNTEITKAPLSAS
MIKRYDEHHQDLTLLKALVRQQLPEKYKEIFFDQSKNGYAGYIDGGASQEEFYKFIKPI
LEKMDGTEELLVKLNREDLLRKQRTFDNGSIPHQIHLGELHAILRRQEDFYPFLKDNRE
KIEKILTFRIPYYVGPLARGNSRFAWMTRKSEETITPWNFEEVVDKGASAQSFIERMTNF
DKNLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENEDI
LEDIVLTLTLFEDREMIEERLKTYAHLFDDKVMKQLKRRRYTGWGRLSRKLINGIRDK
QSGKTILDFLKSDGFANRNFMQLIHDDSLTFKEDIQKAQVSGQGDSLHEHIANLAGSPA
IKKGILQTVKVVDELVKVMGRHKPENIVIEMARENQTTQKGQKNSRERMKRIEEGIKE
LGSQILKEHPVENTQLQNEKLYLYYLQNGRDMYVDQELDINRLSDYDVDHIVPQSFLK
DDSIDNKVLTRSDKNRGKSDNVPSEEVVKKMKNYWRQLLNAKLITQRKFDNLTKAER
GGLSELDKAGFIKRQLVETRQITKHVAQILDSRMNTKYDENDKLIREVKVITLKSKLVS
DFRKDFQFYKVREINNYHHAHDAYLNAVVGTALIKKYPKLESEFVYGDYKVYDVRK
MIAKSEQEIGKATAKYFFYSNIMNFFKTEITLANGEIRKRPLIETNGETGEIVWDKGRDF
ATVRKVLSMPQVNIVKKTEVQTGGFSKESILPKRNSDKLIARKKDWDPKKYGGFDSPT
VAYSVLVVAKVEKGKSKKLKSVKELLGITIMERSSFEKNPIDFLEAKGYKEVKKDLIIKL
PKYSLFELENGRKRMLASAGELQKGNELALPSKYVNFLYLASHYEKLKGSPEDNEQK
QLFVEQHKHYLDEIIEQISEFSKRVILADANLDKVLSAYNKHRDKPIREQAENIIHLFTLT
NLGAPAAFKYFDTTIDRKRYTSTKEVLDATLIHQSITGLYETRIDLSQLGGDSGGSGGSG
GSTNLSDIIEKETGKQLVIQESILMLPEEVEEVIGNKPESDILVHTAYDESTDENVMLLTS
DAPEYKPWALVIQDSNGENKIKMLSGGSGGSGGSTNLSDIIEKETGKQLVIQESILMLPE
EVEEVIGNKPESDILVHTAYDESTDENVMLLTSDAPEYKPWALVIQDSNGENKIKMLSG
GSKRTADGSEFEPKKKRKV
>SEQ ID NO:155 mini-Sdd9- BE4max MKRTADGSEFESPKKKRKVCPIWVKNALQELVGRKETSGKVFDVDGKPIGPDIQS 
GYKDRELRRGVYETLRKSPFFQKHFPSTATWYVSLHVEAQYAVWMLRNRIKHATVVI
NNTYVCSDMNRLHDNCMTAVPHILPEGYTMTVWKADRTEVTLRGKAPKESGGSSGG
SSGSETPGTSESATPESSGGSSGGSDKKYSIGLAIGTNSVGWAVITDEYKVPSKKFKVLG
NTDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRLIY
LALAHMIKFRGHFLIEGDLNPDNSDVDKLFIQLVQTYNQLFEENPINASGVDAKAILSA
RLSKSRRLENLIAQLPGEKKNGLFGNLIALSLGLTPNFKSNFDLAEDAKLQLSKDTYDD
DLDNLLAQIGDQYADLFLAAKNLSDAILLSDILRVNTEITKAPLSASMIKRYDEHHQDL
TLLKALVRQQLPEKYKEIFFDQSKNGYAGYIDGGASQEEFYKFIKPILEKMDGTEELLV
KLNREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLPK
HSLLYEYFTVYNELTKVKYVTEGMRKPAFLSGEQKKAIVDLLFKTNRKVTVKQLKED
YFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSD
GFANRNFMQLIHDDSLTFKEDIQKAQVSGQGDSLHEHIANLAGSPAIKKGILQTVKVVD
ELVKVMGRHKPENIVIEMARENQTTQKGQKNSRERMKRIEEGIKELGSQILKEHPVEN
TQLQNEKLYLYYLQNGRDMYVDQELDINRLSDYDVDHIVPQSFLKDDSIDNKVLTRSD
KNRGKSDNVPSEEVVKKMKNYWRQLLNAKLITQRKFDNLTKAERGGLSELDKAGFIK
RQLVETRQITKHVAQILDSRMNTKYDENDKLIREVKVITLKSKLVSDFRKDFQFYKVRE
INNYHHAHDAYLNAVVGTALIKKYPKLESEFVYGDYKVYDVRKMIAKSEQEIGKATA
KYFFYSNIMNFFKTEITLANGEIRKRPLIETNGETGEIVWDKGRDFATVRKVLSMPQVNI
VKKTEVQTGGFSKESILPKRNSDKLIARKKDWDPKKYGGFDSPTVAYSVLVVAKVEKG
KSKKLKSVKELLGITIMERSSFEKNPIDFLEAKGYKEVKKDLIIKLPKYSLFELENGRKR
MLASAGELQKGNELALPSKYVNFLYLASHYEKLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDSGGSGGSGGSTNLSDIIEKETGK
QLVIQESILMLPEEVEEVIGNKPESDILVHTAYDESTDENVMLLTSDAPEYKPWALVIQD
SNGENKIKMLSGGSGGSGGSTNLSDIIEKETGKQLVIQESILMLPEEVEEVIGNKPESDIL
VHTAYDESTDENVMLLTSDAPEYKPWALVIQDSNGENKIKMLSGGSKRTADGSEFEPK
KKRKV
>SEQ ID NO:156 TadA-8e- BE4max 
MKRTADGSEFESPKKKRKVSEVEFSHEYWMRHALTLAKRARDEREVPVGAVLVL
NNRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFYR
 
MPRQVFNAQKKAQSSINSGGSSGGSSGSETPGTSESATPESSGGSSGGSDKKYSIGLAIG
TNSVGWAVITDEYKVPSKKFKVLGNTDRHSIKKNLIGALLFDSGETAEATRLKRTARRR
YTRRKNRICYLQEIFSNEMAKVDDSFFHRLEESFLVEEDKKHERHPIFGNIVDEVAYHE
KYPTIYHLRKKLVDSTDKADLRLIYLALAHMIKFRGHFLIEGDLNPDNSDVDKLFIQLV
QTYNQLFEENPINASGVDAKAILSARLSKSRRLENLIAQLPGEKKNGLFGNLIALSLGLT
PNFKSNFDLAEDAKLQLSKDTYDDDLDNLLAQIGDQYADLFLAAKNLSDAILLSDILR
VNTEITKAPLSASMIKRYDEHHQDLTLLKALVRQQLPEKYKEIFFDQSKNGYAGYIDGG
ASQEEFYKFIKPILEKMDGTEELLVKLNREDLLRKQRTFDNGSIPHQIHLGELHAILRRQ
EDFYPFLKDNREKIEKILTFRIPYYVGPLARGNSRFAWMTRKSEETITPWNFEEVVDKG
ASAQSFIERMTNFDKNLPNEKVLPKHSLLYEYFTVYNELTKVKYVTEGMRKPAFLSGE
QKKAIVDLLFKTNRKVTVKQLKEDYFKKIECFDSVEISGVEDRFNASLGTYHDLLKIIK
DKDFLDNEENEDILEDIVLTLTLFEDREMIEERLKTYAHLFDDKVMKQLKRRRYTGWG
RLSRKLINGIRDKQSGKTILDFLKSDGFANRNFMQLIHDDSLTFKEDIQKAQVSGQGDS
LHEHIANLAGSPAIKKGILQTVKVVDELVKVMGRHKPENIVIEMARENQTTQKGQKNS
RERMKRIEEGIKELGSQILKEHPVENTQLQNEKLYLYYLQNGRDMYVDQELDINRLSD
YDVDHIVPQSFLKDDSIDNKVLTRSDKNRGKSDNVPSEEVVKKMKNYWRQLLNAKLI
TQRKFDNLTKAERGGLSELDKAGFIKRQLVETRQITKHVAQILDSRMNTKYDENDKLIR
EVKVITLKSKLVSDFRKDFQFYKVREINNYHHAHDAYLNAVVGTALIKKYPKLESEFV
YGDYKVYDVRKMIAKSEQEIGKATAKYFFYSNIMNFFKTEITLANGEIRKRPLIETNGE
TGEIVWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFLEAK
GYKEVKKDLIIKLPKYSLFELENGRKRMLASAGELQKGNELALPSKYVNFLYLASHYE
KLKGSPEDNEQKQLFVEQHKHYLDEIIEQISEFSKRVILADANLDKVLSAYNKHRDKPI
REQAENIIHLFTLTNLGAPAAFKYFDTTIDRKRYTSTKEVLDATLIHQSITGLYETRIDLS
QLGGDSGGSGGSGGSTNLSDIIEKETGKQLVIQESILMLPEEVEEVIGNKPESDILVHTAY
DESTDENVMLLTSDAPEYKPWALVIQDSNGENKIKMLSGGSGGSGGSTNLSDIIEKETG
KQLVIQESILMLPEEVEEVIGNKPESDILVHTAYDESTDENVMLLTSDAPEYKPWALVIQ
DSNGENKIKMLSGGSKRTADGSEFEPKKKRKV
>SEQ ID NO:157 mini-Sdd7-QBE3 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSEGGGPGAVPEGGDGPPAVPAEEVERL
 
RGELPPPVVPGTGQKTHGRWIGPDGRVRAIVSGRDEDAALVHAQLAAKGIPDEPTRNS
DVEQKLAAHMVANGIRHVTLVINHRPCRGFDDSCDTLVPIILPEGCTLTVHGQTDKGM
RVRVRYTGGARPWWSSGGSSGGSSGSETPGTSESATPESSGGSSGGSLKGSPEDNEQKQ
LFVEQHKHYLDEIIEQISEFSKRVILADANLDKVLSAYNKHRDKPIREQAENIIHLFTLTN
LGAPAAFKYFDTTIDRKRYTSTKEVLDATLIHQSITGLYETRIDLSQLGGDGGSGGSGGS
GGSGGSGGSGGMDKKYSIGLAIGTNSVGWAVITDEYKVPSKKFKVLGNTDRHSIKKNL
IGALLFDSGETAEATRLKRTARRRYTRRKNRICYLQEIFSNEMAKVDDSFFHRLEESFLV
EEDKKHERHPIFGNIVDEVAYHEKYPTIYHLRKKLVDSTDKADLRLIYLALAHMIKFRG
HFLIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQL
PEKYKEIFFDQSKNGYAGYIDGGASQEEFYKFIKPILEKMDGTEELLVKLNREDLLRKQ
RTFDNGSIPHQIHLGELHAILRRQEDFYPFLKDNREKIEKILTFRIPYYVGPLARGNSRFA
WMTRKSEETITPWNFEEVVDKGASAQSFIERMTNFDKNLPNEKVLPKHSLLYEYFTVY
NELTKVKYVTEGMRKPAFLSGEQKKAIVDLLFKTNRKVTVKQLKEDYFKKIECFDSVE
ISGVEDRFNASLGTYHDLLKIIKDKDFLDNEENEDILEDIVLTLTLFEDREMIEERLKTYA
HLFDDKVMKQLKRRRYTGWGRLSRKLINGIRDKQSGKTILDFLKSDGFANRNFMQLIH
DDSLTFKEDIQKAQVSGQGDSLHEHIANLAGSPAIKKGILQTVKVVDELVKVMGRHKP
ENIVIEMARENQTTQKGQKNSRERMKRIEEGIKELGSQILKEHPVENTQLQNEKLYLYY
LQNGRDMYVDQELDINRLSDYDVDHIVPQSFLKDDSIDNKVLTRSDKNRGKSDNVPSE
EVVKKMKNYWRQLLNAKLITQRKFDNLTKAERGGLSELDKAGFIKRQLVETRQITKH
VAQILDSRMNTKYDENDKLIREVKVITLKSKLVSDFRKDFQFYKVREINNYHHAHDAY
LNAVVGTALIKKYPKLESEFVYGDYKVYDVRKMIAKSEQEIGSGGSGGSGGSTNLSDII
EKETGKQLVIQESILMLPEEVEEVIGNKPESDILVHTAYDESTDENVMLLTSDAPEYKPW
ALVIQDSNGENKIKMLSGGSGGSGGSTNLSDIIEKETGKQLVIQESILMLPEEVEEVIGN
KPESDILVHTAYDESTDENVMLLTSDAPEYKPWALVIQDSNGENKIKMLSGGSKRTADG
SEFEPKKKRKV
>SEQ ID NO: 158 QBE1-X₁ 
NIMNFFKTEITLANGEIRKRPLIETNGETGEIVWDKGRDFATVRKVLSMPQVNIVK
KTEVQTGGFSKESILPKRNSDKLIARKKDWDPKKYGGFDSPTVAYSVLVVAKVEKGKS
KKLKSVKELLGITIMERSSFEKNPIDFLEAKGYKEVKKDLIIKLPKYSLFELENGRKRML
ASAGELQKGNELALPSKYVNFLYLASHYEKLKGSPEDNEQKQLFVEQHKHYLDEIIEQI
SEFSKRVILADANLDKVLSAYNKHRDKPIREQAENIIHLFTLTNLGAPAAFKYFDTTIDR
KRYTSTKEVLDATLIHQSITGLYETRIDLSQLGGD
>SEQ ID NO: 159 QBE1-X₂ 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILSARLSKSRRLENLIAQLPGEKK
NGLFGNLIALSLGLTPNFKSNFDLAEDAKLQLSKDTYDDDLDNLLAQIGDQYADLFLA
AKNLSDAILLSDILRVNTEITKAPLSASMIKRYDEHHQDLTLLKALVRQQLPEKYKEIFF
DQSKNGYAGYIDGGASQEEFYKFIKPILEKMDGTEELLVKLNREDLLRKQRTFDNGSIP
HQIHLGELHAILRRQEDFYPFLKDNREKIEKILTFRIPYYVGPLARGNSRFAWMTRKSEE
TITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRF
NASLGTYHDLLKIIKDKDFLDNEENEDILEDIVLTLTLFEDREMIEERLKTYAHLFDDKV
MKQLKRRRYTGWGRLSRKLINGIRDKQSGKTILDFLKSDGFANRNFMQLIHDDSLTFK
EDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRD
MYVDQELDINRLSDYDVDHIVPQSFLKDDSIDNKVLTRSDKNRGKSDNVPSEEVVKK
MKNYWRQLLNAKLITQRKFDNLTKAERGGLSELDKAGFIKRQLVETRQITKHVAQILD
SRMNTKYDENDKLIREVKVITLKSKLVSDFRKDFQFYKVREINNYHHAHDAYLNAVV
GTALIKKYPKLESEFVYGDYKVYDVRKMIAKSE
>SEQ ID NO: 160 QBE1-X₃
   QEIGKATAKYFFYS
>SEQ ID NO: 161 QBE2-X₂ 
ARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDEH
HQDLTLLKALVRQQLPEKYKEIFFDQSKNGYAGYIDGGASQEEFYKFIKPILEKMDGTE
ELLVKLNREDLLRKQRTFDNGSIPHQIHLGELHAILRRQEDFYPFLKDNREKIEKILTFRI
PYYVGPLARGNSRFAWMTRKSEETITPWNFEEVVDKGASAQSFIERMTNFDKNLPNEK
VLPKHSLLYEYFTVYNELTKVKYVTEGMRKPAFLSGEQKKAIVDLLFKTNRKVTVKQL
KEDYFKKIECFDSVEISGVEDRFNASLGTYHDLLKIIKDKDFLDNEENEDILEDIVLTLTL
FEDREMIEERLKTYAHLFDDKVMKQLKRRRYTGWGRLSRKLINGIRDKQSGKTILDFL
KSDGFANRNFMQLIHDDSLTFKEDIQKAQVSGQGDSLHEHIANLAGSPAIKKGILQTVK
VVDELVKVMGRHKPENIVIEMARENQTTQKGQKNSRERMKRIEEGIKELGSQILKEHP
 
VENTQLQNEKLYLYYLQNGRDMYVDQELDINRLSDYDVDHIVPQSFLKDDSIDNKVLT
RSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFY
KVREINNYHHAHDAYLNAVVGTALIKKYPKLESEFVYGDYKVYDVRKMIAKSEQEIG
KATAKYFFYSNIMNFFKTEITLANGEI
>SEQ ID NO: 162 QBE2-X₃ 
RKRPLIETNGETGEIVWDKGRDFATVRKVLSMPQVNIVKKTEVQTGGFSKESILPK
RNSDKLIARKKDWDPKKYGGFDSPTVAYSVLVVAKVEKGKSKKLKSVKELLGITIMER
SSFEKNPIDFLEAKGYKEVKKDLIIKLPKYSLFELENGRKRMLASAGELQKGNELALPS
KYVNFLYLASHYEKLKGSPEDNEQKQLFVEQHKHYLDEIIEQISEFSKRVILADANLDK
VLSAYNKHRDKPIREQAENIIHLFTLTNLGAPAAFKYFDTTIDRKRYTSTKEVLDATLIH
QSITGLYETRIDLSQLGGD
>SEQ ID NO: 163 QBE2-X₄ 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILS
>SEQ ID NO: 164 QBE3-X₂ 
KATAKYFFYSNIMNFFKTEITLANGEIRKRPLIETNGETGEIVWDKGRDFATVRKV
LSMPQVNIVKKTEVQTGGFSKESILPKRNSDKLIARKKDWDPKKYGGFDSPTVAYSVL
VVAKVEKGKSKKLKSVKELLGITIMERSSFEKNPIDFLEAKGYKEVKKDLIIKLPKYSLF
ELENGRKRMLASAGELQKGNELALPSKYVNFLYLASHYEK
>SEQ ID NO: 165 QBE3-X₃ 
LKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGD
>SEQ ID NO: 166 QBE3-X₄ 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
 
PDNSDVDKLFIQLVQTYNQLFEENPINASGVDAKAILSARLSKSRRLENLIAQLPGEKK
NGLFGNLIALSLGLTPNFKSNFDLAEDAKLQLSKDTYDDDLDNLLAQIGDQYADLFLA
AKNLSDAILLSDILRVNTEITKAPLSASMIKRYDEHHQDLTLLKALVRQQLPEKYKEIFF
DQSKNGYAGYIDGGASQEEFYKFIKPILEKMDGTEELLVKLNREDLLRKQRTFDNGSIP
HQIHLGELHAILRRQEDFYPFLKDNREKIEKILTFRIPYYVGPLARGNSRFAWMTRKSEE
TITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRF
NASLGTYHDLLKIIKDKDFLDNEENEDILEDIVLTLTLFEDREMIEERLKTYAHLFDDKV
MKQLKRRRYTGWGRLSRKLINGIRDKQSGKTILDFLKSDGFANRNFMQLIHDDSLTFK
EDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRD
MYVDQELDINRLSDYDVDHIVPQSFLKDDSIDNKVLTRSDKNRGKSDNVPSEEVVKK
MKNYWRQLLNAKLITQRKFDNLTKAERGGLSELDKAGFIKRQLVETRQITKHVAQILD
SRMNTKYDENDKLIREVKVITLKSKLVSDFRKDFQFYKVREINNYHHAHDAYLNAVV
GTALIKKYPKLESEFVYGDYKVYDVRKMIAKSEQEIG
>SEQ ID NO:167 mini-Sdd9-QBE3-without linker 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSCPIWVKNALQELVGRKETSGKVFDV
DGKPIGPDIQSGYKDRELRRGVYETLRKSPFFQKHFPSTATWYVSLHVEAQYAVWMLR
NRIKHATVVINNTYVCSDMNRLHDNCMTAVPHILPEGYTMTVWKADRTEVTLRGKAP
KESGGSSGGSSGSETPGTSESATPESSGGSSGGSLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDMDKKYSIGLAIGTNSVGWAVITD
EYKVPSKKFKVLGNTDRHSIKKNLIGALLFDSGETAEATRLKRTARRRYTRRKNRICYL
QEIFSNEMAKVDDSFFHRLEESFLVEEDKKHERHPIFGNIVDEVAYHEKYPTIYHLRKKL
VDSTDKADLRLIYLALAHMIKFRGHFLIEGDLNPDNSDVDKLFIQLVQTYNQLFEENPI
NASGVDAKAILSARLSKSRRLENLIAQLPGEKKNGLFGNLIALSLGLTPNFKSNFDLAE
DAKLQLSKDTYDDDLDNLLAQIGDQYADLFLAAKNLSDAILLSDILRVNTEITKAPLSA
SMIKRYDEHHQDLTLLKALVRQQLPEKYKEIFFDQSKNGYAGYIDGGASQEEFYKFIKP
ILEKMDGTEELLVKLNREDLLRKQRTFDNGSIPHQIHLGELHAILRRQEDFYPFLKDNR
EKIEKILTFRIPYYVGPLARGNSRFAWMTRKSEETITPWNFEEVVDKGASAQSFIERMTN
 
FDKNLPNEKVLPKHSLLYEYFTVYNELTKVKYVTEGMRKPAFLSGEQKKAIVDLLFKT
NRKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRDK
QSGKTILDFLKSDGFANRNFMQLIHDDSLTFKEDIQKAQVSGQGDSLHEHIANLAGSPA
IKKGILQTVKVVDELVKVMGRHKPENIVIEMARENQTTQKGQKNSRERMKRIEEGIKE
LGSQILKEHPVENTQLQNEKLYLYYLQNGRDMYVDQELDINRLSDYDVDHIVPQSFLK
DDSIDNKVLTRSDKNRGKSDNVPSEEVVKKMKNYWRQLLNAKLITQRKFDNLTKAER
GGLSELDKAGFIKRQLVETRQITKHVAQILDSRMNTKYDENDKLIREVKVITLKSKLVS
DFRKDFQFYKVREINNYHHAHDAYLNAVVGTALIKKYPKLESEFVYGDYKVYDVRK
MIAKSEQEIGSGGSGGSGGSTNLSDIIEKETGKQLVIQESILMLPEEVEEVIGNKPESDILV
HTAYDESTDENVMLLTSDAPEYKPWALVIQDSNGENKIKMLSGGSGGSGGSTNLSDIIE
KETGKQLVIQESILMLPEEVEEVIGNKPESDILVHTAYDESTDENVMLLTSDAPEYKPWA
LVIQDSNGENKIKMLSGGSKRTADGSEFEPKKKRKV
>SEQ ID NO:168 mini-Sdd9-QBE3-3aa linker 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSCPIWVKNALQELVGRKETSGKVFDV
DGKPIGPDIQSGYKDRELRRGVYETLRKSPFFQKHFPSTATWYVSLHVEAQYAVWMLR
NRIKHATVVINNTYVCSDMNRLHDNCMTAVPHILPEGYTMTVWKADRTEVTLRGKAP
KESGGSSGGSSGSETPGTSESATPESSGGSSGGSLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDGGSMDKKYSIGLAIGTNSVGWA
VITDEYKVPSKKFKVLGNTDRHSIKKNLIGALLFDSGETAEATRLKRTARRRYTRRKNR
ICYLQEIFSNEMAKVDDSFFHRLEESFLVEEDKKHERHPIFGNIVDEVAYHEKYPTIYHL
RKKLVDSTDKADLRLIYLALAHMIKFRGHFLIEGDLNPDNSDVDKLFIQLVQTYNQLFE
ENPINASGVDAKAILSARLSKSRRLENLIAQLPGEKKNGLFGNLIALSLGLTPNFKSNFD
LAEDAKLQLSKDTYDDDLDNLLAQIGDQYADLFLAAKNLSDAILLSDILRVNTEITKAP
LSASMIKRYDEHHQDLTLLKALVRQQLPEKYKEIFFDQSKNGYAGYIDGGASQEEFYK
FIKPILEKMDGTEELLVKLNREDLLRKQRTFDNGSIPHQIHLGELHAILRRQEDFYPFLK
DNREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNE
 
ENEDILEDIVLTLTLFEDREMIEERLKTYAHLFDDKVMKQLKRRRYTGWGRLSRKLING
IRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVPQ
SFLKDDSIDNKVLTRSDKNRGKSDNVPSEEVVKKMKNYWRQLLNAKLITQRKFDNLT
KAERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVYD
VRKMIAKSEQEIGSGGSGGSGGSTNLSDIIEKETGKQLVIQESILMLPEEVEEVIGNKPES
DILVHTAYDESTDENVMLLTSDAPEYKPWALVIQDSNGENKIKMLSGGSGGSGGSTNLS
DIIEKETGKQLVIQESILMLPEEVEEVIGNKPESDILVHTAYDESTDENVMLLTSDAPEYK
PWALVIQDSNGENKIKMLSGGSKRTADGSEFEPKKKRKV
 The underlined part represents 3aa linker.
>SEQ ID NO:169 mini-Sdd9-QBE3-6aa linker 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSCPIWVKNALQELVGRKETSGKVFDV
DGKPIGPDIQSGYKDRELRRGVYETLRKSPFFQKHFPSTATWYVSLHVEAQYAVWMLR
NRIKHATVVINNTYVCSDMNRLHDNCMTAVPHILPEGYTMTVWKADRTEVTLRGKAP
KESGGSSGGSSGSETPGTSESATPESSGGSSGGSLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDGGSGGSMDKKYSIGLAIGTNSV
GWAVITDEYKVPSKKFKVLGNTDRHSIKKNLIGALLFDSGETAEATRLKRTARRRYTRR
KNRICYLQEIFSNEMAKVDDSFFHRLEESFLVEEDKKHERHPIFGNIVDEVAYHEKYPTI
YHLRKKLVDSTDKADLRLIYLALAHMIKFRGHFLIEGDLNPDNSDVDKLFIQLVQTYN
QLFEENPINASGVDAKAILSARLSKSRRLENLIAQLPGEKKNGLFGNLIALSLGLTPNFK
SNFDLAEDAKLQLSKDTYDDDLDNLLAQIGDQYADLFLAAKNLSDAILLSDILRVNTEI
TKAPLSASMIKRYDEHHQDLTLLKALVRQQLPEKYKEIFFDQSKNGYAGYIDGGASQE
EFYKFIKPILEKMDGTEELLVKLNREDLLRKQRTFDNGSIPHQIHLGELHAILRRQEDFY
PFLKDNREKIEKILTFRIPYYVGPLARGNSRFAWMTRKSEETITPWNFEEVVDKGASAQ
SFIERMTNFDKNLPNEKVLPKHSLLYEYFTVYNELTKVKYVTEGMRKPAFLSGEQKKA
IVDLLFKTNRKVTVKQLKEDYFKKIECFDSVEISGVEDRFNASLGTYHDLLKIIKDKDF
LDNEENEDILEDIVLTLTLFEDREMIEERLKTYAHLFDDKVMKQLKRRRYTGWGRLSR
 
KLINGIRDKQSGKTILDFLKSDGFANRNFMQLIHDDSLTFKEDIQKAQVSGQGDSLHEH
IANLAGSPAIKKGILQTVKVVDELVKVMGRHKPENIVIEMARENQTTQKGQKNSRERM
KRIEEGIKELGSQILKEHPVENTQLQNEKLYLYYLQNGRDMYVDQELDINRLSDYDVD
HIVPQSFLKDDSIDNKVLTRSDKNRGKSDNVPSEEVVKKMKNYWRQLLNAKLITQRK
FDNLTKAERGGLSELDKAGFIKRQLVETRQITKHVAQILDSRMNTKYDENDKLIREVK
VITLKSKLVSDFRKDFQFYKVREINNYHHAHDAYLNAVVGTALIKKYPKLESEFVYGD
YKVYDVRKMIAKSEQEIGSGGSGGSGGSTNLSDIIEKETGKQLVIQESILMLPEEVEEVI
GNKPESDILVHTAYDESTDENVMLLTSDAPEYKPWALVIQDSNGENKIKMLSGGSGGS
GGSTNLSDIIEKETGKQLVIQESILMLPEEVEEVIGNKPESDILVHTAYDESTDENVMLLT
SDAPEYKPWALVIQDSNGENKIKMLSGGSKRTADGSEFEPKKKRKV
 The underlined part represents 6aa linker.
>SEQ ID NO:170 mini-Sdd9-QBE3-12aa linker 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSCPIWVKNALQELVGRKETSGKVFDV
DGKPIGPDIQSGYKDRELRRGVYETLRKSPFFQKHFPSTATWYVSLHVEAQYAVWMLR
NRIKHATVVINNTYVCSDMNRLHDNCMTAVPHILPEGYTMTVWKADRTEVTLRGKAP
KESGGSSGGSSGSETPGTSESATPESSGGSSGGSLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDGGSGGSGGSGGMDKKYSIGLAI
GTNSVGWAVITDEYKVPSKKFKVLGNTDRHSIKKNLIGALLFDSGETAEATRLKRTARR
RYTRRKNRICYLQEIFSNEMAKVDDSFFHRLEESFLVEEDKKHERHPIFGNIVDEVAYHE
KYPTIYHLRKKLVDSTDKADLRLIYLALAHMIKFRGHFLIEGDLNPDNSDVDKLFIQLV
QTYNQLFEENPINASGVDAKAILSARLSKSRRLENLIAQLPGEKKNGLFGNLIALSLGLT
PNFKSNFDLAEDAKLQLSKDTYDDDLDNLLAQIGDQYADLFLAAKNLSDAILLSDILR
VNTEITKAPLSASMIKRYDEHHQDLTLLKALVRQQLPEKYKEIFFDQSKNGYAGYIDGG
ASQEEFYKFIKPILEKMDGTEELLVKLNREDLLRKQRTFDNGSIPHQIHLGELHAILRRQ
EDFYPFLKDNREKIEKILTFRIPYYVGPLARGNSRFAWMTRKSEETITPWNFEEVVDKG
ASAQSFIERMTNFDKNLPNEKVLPKHSLLYEYFTVYNELTKVKYVTEGMRKPAFLSGE
QKKAIVDLLFKTNRKVTVKQLKEDYFKKIECFDSVEISGVEDRFNASLGTYHDLLKIIK
DKDFLDNEENEDILEDIVLTLTLFEDREMIEERLKTYAHLFDDKVMKQLKRRRYTGWG
RLSRKLINGIRDKQSGKTILDFLKSDGFANRNFMQLIHDDSLTFKEDIQKAQVSGQGDS
 
LHEHIANLAGSPAIKKGILQTVKVVDELVKVMGRHKPENIVIEMARENQTTQKGQKNS
RERMKRIEEGIKELGSQILKEHPVENTQLQNEKLYLYYLQNGRDMYVDQELDINRLSD
YDVDHIVPQSFLKDDSIDNKVLTRSDKNRGKSDNVPSEEVVKKMKNYWRQLLNAKLI
TQRKFDNLTKAERGGLSELDKAGFIKRQLVETRQITKHVAQILDSRMNTKYDENDKLIR
EVKVITLKSKLVSDFRKDFQFYKVREINNYHHAHDAYLNAVVGTALIKKYPKLESEFV
YGDYKVYDVRKMIAKSEQEIGSGGSGGSGGSTNLSDIIEKETGKQLVIQESILMLPEEV
EEVIGNKPESDILVHTAYDESTDENVMLLTSDAPEYKPWALVIQDSNGENKIKMLSGGS
GGSGGSTNLSDIIEKETGKQLVIQESILMLPEEVEEVIGNKPESDILVHTAYDESTDENVM
LLTSDAPEYKPWALVIQDSNGENKIKMLSGGSKRTADGSEFEPKKKRKV
 The underlined part represents 12aa linker.
>SEQ ID NO:171 mini-Sdd9-QBE3-15aa linker 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSCPIWVKNALQELVGRKETSGKVFDV
DGKPIGPDIQSGYKDRELRRGVYETLRKSPFFQKHFPSTATWYVSLHVEAQYAVWMLR
NRIKHATVVINNTYVCSDMNRLHDNCMTAVPHILPEGYTMTVWKADRTEVTLRGKAP
KESGGSSGGSSGSETPGTSESATPESSGGSSGGSLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDGGSGGSGGSGGSGGSMDKKYSI
GLAIGTNSVGWAVITDEYKVPSKKFKVLGNTDRHSIKKNLIGALLFDSGETAEATRLKR
TARRRYTRRKNRICYLQEIFSNEMAKVDDSFFHRLEESFLVEEDKKHERHPIFGNIVDEV
AYHEKYPTIYHLRKKLVDSTDKADLRLIYLALAHMIKFRGHFLIEGDLNPDNSDVDKLF
IQLVQTYNQLFEENPINASGVDAKAILSARLSKSRRLENLIAQLPGEKKNGLFGNLIALS
LGLTPNFKSNFDLAEDAKLQLSKDTYDDDLDNLLAQIGDQYADLFLAAKNLSDAILLS
DILRVNTEITKAPLSASMIKRYDEHHQDLTLLKALVRQQLPEKYKEIFFDQSKNGYAGYI
DGGASQEEFYKFIKPILEKMDGTEELLVKLNREDLLRKQRTFDNGSIPHQIHLGELHAIL
RRQEDFYPFLKDNREKIEKILTFRIPYYVGPLARGNSRFAWMTRKSEETITPWNFEEVV
DKGASAQSFIERMTNFDKNLPNEKVLPKHSLLYEYFTVYNELTKVKYVTEGMRKPAFL
SGEQKKAIVDLLFKTNRKVTVKQLKEDYFKKIECFDSVEISGVEDRFNASLGTYHDLL
KIIKDKDFLDNEENEDILEDIVLTLTLFEDREMIEERLKTYAIILFDDKVMKQLKRRRYT
GWGRLSRKLINGIRDKQSGKTILDFLKSDGFANRNFMQLIHDDSLTFKEDIQKAQVSGQ
GDSLHEHIANLAGSPAIKKGILQTVKVVDELVKVMGRHKPENIVIEMARENQTTQKGQ
 
KNSRERMKRIEEGIKELGSQILKEHPVENTQLQNEKLYLYYLQNGRDMYVDQELDINR
LSDYDVDHIVPQSFLKDDSIDNKVLTRSDKNRGKSDNVPSEEVVKKMKNYWRQLLNA
KLITQRKFDNLTKAERGGLSELDKAGFIKRQLVETRQITKHVAQILDSRMNTKYDEND
KLIREVKVITLKSKLVSDFRKDFQFYKVREINNYHHAHDAYLNAVVGTALIKKYPKLES
EFVYGDYKVYDVRKMIAKSEQEIGSGGSGGSGGSTNLSDIIEKETGKQLVIQESILMLPE
EVEEVIGNKPESDILVHTAYDESTDENVMLLTSDAPEYKPWALVIQDSNGENKIKMLSG
GSGGSGGSTNLSDIIEKETGKQLVIQESILMLPEEVEEVIGNKPESDILVHTAYDESTDEN
VMLLTSDAPEYKPWALVIQDSNGENKIKMLSGGSKRTADGSEFEPKKKRKV
 The underlined part represents 15aa linker.
>SEQ ID NO:172 mini-Sdd9-QBE3-18aa linker 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSCPIWVKNALQELVGRKETSGKVFDV
DGKPIGPDIQSGYKDRELRRGVYETLRKSPFFQKHFPSTATWYVSLHVEAQYAVWMLR
NRIKHATVVINNTYVCSDMNRLHDNCMTAVPHILPEGYTMTVWKADRTEVTLRGKAP
KESGGSSGGSSGSETPGTSESATPESSGGSSGGSLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDGGSGGSGGSGGSGGSGGSMDK
KYSIGLAIGTNSVGWAVITDEYKVPSKKFKVLGNTDRHSIKKNLIGALLFDSGETAEAT
RLKRTARRRYTRRKNRICYLQEIFSNEMAKVDDSFFHRLEESFLVEEDKKHERHPIFGNI
VDEVAYHEKYPTIYHLRKKLVDSTDKADLRLIYLALAHMIKFRGHFLIEGDLNPDNSD
VDKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNLS
DAILLSDILRVNTEITKAPLSASMIKRYDEHHQDLTLLKALVRQQLPEKYKEIFFDQSKN
GYAGYIDGGASQEEFYKFIKPILEKMDGTEELLVKLNREDLLRKQRTFDNGSIPHQIHL
GELHAILRRQEDFYPFLKDNREKIEKILTFRIPYYVGPLARGNSRFAWMTRKSEETITPW
NFEEVVDKGASAQSFIERMTNFDKNLPNEKVLPKHSLLYEYFTVYNELTKVKYVTEG
MRKPAFLSGEQKKAIVDLLFKTNRKVTVKQLKEDYFKKIECFDSVEISGVEDRFNASL
GTYHDLLKIIKDKDFLDNEENEDILEDIVLTLTLFEDREMIEERLKTYAHLFDDKVMKQ
LKRRRYTGWGRLSRKLINGIRDKQSGKTILDFLKSDGFANRNFMQLIHDDSLTFKEDIQ
KAQVSGQGDSLHEHIANLAGSPAIKKGILQTVKVVDELVKVMGRHKPENIVIEMAREN
QTTQKGQKNSRERMKRIEEGIKELGSQILKEHPVENTQLQNEKLYLYYLQNGRDMYV
 
DQELDINRLSDYDVDHIVPQSFLKDDSIDNKVLTRSDKNRGKSDNVPSEEVVKKMKNY
WRQLLNAKLITQRKFDNLTKAERGGLSELDKAGFIKRQLVETRQITKHVAQILDSRMN
TKYDENDKLIREVKVITLKSKLVSDFRKDFQFYKVREINNYHHAHDAYLNAVVGTALI
KKYPKLESEFVYGDYKVYDVRKMIAKSEQEIGSGGSGGSGGSTNLSDIIEKETGKQLVI
QESILMLPEEVEEVIGNKPESDILVHTAYDESTDENVMLLTSDAPEYKPWALVIQDSNGE
NKIKMLSGGSGGSGGSTNLSDIIEKETGKQLVIQESILMLPEEVEEVIGNKPESDILVHTA
YDESTDENVMLLTSDAPEYKPWALVIQDSNGENKIKMLSGGSKRTADGSEFEPKKKRK
V
 The underlined part represents 18aa linker.
>SEQ ID NO:173 mini-Sdd9-QBE3-21aa linker 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSCPIWVKNALQELVGRKETSGKVFDV
DGKPIGPDIQSGYKDRELRRGVYETLRKSPFFQKHFPSTATWYVSLHVEAQYAVWMLR
NRIKHATVVINNTYVCSDMNRLHDNCMTAVPHILPEGYTMTVWKADRTEVTLRGKAP
KESGGSSGGSSGSETPGTSESATPESSGGSSGGSLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDGGSGGSGGSGGSGGSGGSGGSM
DKKYSIGLAIGTNSVGWAVITDEYKVPSKKFKVLGNTDRHSIKKNLIGALLFDSGETAE
ATRLKRTARRRYTRRKNRICYLQEIFSNEMAKVDDSFFHRLEESFLVEEDKKHERHPIF
GNIVDEVAYHEKYPTIYHLRKKLVDSTDKADLRLIYLALAHMIKFRGHFLIEGDLNPDN
SDVDKLFIQLVQTYNQLFEENPINASGVDAKAILSARLSKSRRLENLIAQLPGEKKNGL
FGNLIALSLGLTPNFKSNFDLAEDAKLQLSKDTYDDDLDNLLAQIGDQYADLFLAAKN
LSDAILLSDILRVNTEITKAPLSASMIKRYDEHHQDLTLLKALVRQQLPEKYKEIFFDQS
KNGYAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKSEETIT
PWNFEEVVDKGASAQSFIERMTNFDKNLPNEKVLPKHSLLYEYFTVYNELTKVKYVTE
GMRKPAFLSGEQKKAIVDLLFKTNRKVTVKQLKEDYFKKIECFDSVEISGVEDRFNAS
LGTYHDLLKIIKDKDFLDNEENEDILEDIVLTLTLFEDREMIEERLKTYAHLFDDKVMK
QLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMARE
NQTTQKGQKNSRERMKRIEEGIKELGSQILKEHPVENTQLQNEKLYLYYLQNGRDMY
 
VDQELDINRLSDYDVDHIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTAL
IKKYPKLESEFVYGDYKVYDVRKMIAKSEQEIGSGGSGGSGGSTNLSDIIEKETGKQLV
IQESILMLPEEVEEVIGNKPESDILVHTAYDESTDENVMLLTSDAPEYKPWALVIQDSNG
ENKIKMLSGGSGGSGGSTNLSDIIEKETGKQLVIQESILMLPEEVEEVIGNKPESDILVHT
AYDESTDENVMLLTSDAPEYKPWALVIQDSNGENKIKMLSGGSKRTADGSEFEPKKKR
KV
 The underlined part represents 21aa linker.
>SEQ ID NO:174 mini-Sdd9-QBE3-32aa linker 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSCPIWVKNALQELVGRKETSGKVFDV
DGKPIGPDIQSGYKDRELRRGVYETLRKSPFFQKHFPSTATWYVSLHVEAQYAVWMLR
NRIKHATVVINNTYVCSDMNRLHDNCMTAVPHILPEGYTMTVWKADRTEVTLRGKAP
KESGGSSGGSSGSETPGTSESATPESSGGSSGGSLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDSGGSSGGSSGSETPGTSESATPES
SGGSSGGSMDKKYSIGLAIGTNSVGWAVITDEYKVPSKKFKVLGNTDRHSIKKNLIGAL
LFDSGETAEATRLKRTARRRYTRRKNRICYLQEIFSNEMAKVDDSFFHRLEESFLVEED
KKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQL
PGEKKNGLFGNLIALSLGLTPNFKSNFDLAEDAKLQLSKDTYDDDLDNLLAQIGDQYA
DLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTF
DNGSIPHQIHLGELHAILRRQEDFYPFLKDNREKIEKILTFRIPYYVGPLARGNSRFAWM
TRKSEETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHL
FDDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKPE
NIVIEMARENQTTQKGQKNSRERMKRIEEGIKELGSQILKEHPVENTQLQNEKLYLYYL
 
QNGRDMYVDQELDINRLSDYDVDHIVPQSFLKDDSIDNKVLTRSDKNRGKSDNVPSEE
VVKKMKNYWRQLLNAKLITQRKFDNLTKAERGGLSELDKAGFIKRQLVETRQITKHV
AQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGSGGSGGSGGSTNLSDIIE
KETGKQLVIQESILMLPEEVEEVIGNKPESDILVHTAYDESTDENVMLLTSDAPEYKPWA
LVIQDSNGENKIKMLSGGSGGSGGSTNLSDIIEKETGKQLVIQESILMLPEEVEEVIGNKP
ESDILVHTAYDESTDENVMLLTSDAPEYKPWALVIQDSNGENKIKMLSGGSKRTADGSE
FEPKKKRKV
 The underlined part represents 32aa linker.
>SEQ ID NO:175 mini-Sdd9-QBE3-64aa linker 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSCPIWVKNALQELVGRKETSGKVFDV
DGKPIGPDIQSGYKDRELRRGVYETLRKSPFFQKHFPSTATWYVSLHVEAQYAVWMLR
NRIKHATVVINNTYVCSDMNRLHDNCMTAVPHILPEGYTMTVWKADRTEVTLRGKAP
KESGGSSGGSSGSETPGTSESATPESSGGSSGGSLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDSGGSSGGSSGSETPGTSESATPES
SGGSSGGSSGGSSGGSSGSETPGTSESATPESSGGSSGGSMDKKYSIGLAIGTNSVGWAV
ITDEYKVPSKKFKVLGNTDRHSIKKNLIGALLFDSGETAEATRLKRTARRRYTRRKNRI
CYLQEIFSNEMAKVDDSFFHRLEESFLVEEDKKHERHPIFGNIVDEVAYHEKYPTIYHLR
KKLVDSTDKADLRLIYLALAHMIKFRGHFLIEGDLNPDNSDVDKLFIQLVQTYNQLFEE
NPINASGVDAKAILSARLSKSRRLENLIAQLPGEKKNGLFGNLIALSLGLTPNFKSNFDL
AEDAKLQLSKDTYDDDLDNLLAQIGDQYADLFLAAKNLSDAILLSDILRVNTEITKAPL
SASMIKRYDEHHQDLTLLKALVRQQLPEKYKEIFFDQSKNGYAGYIDGGASQEEFYKFI
KPILEKMDGTEELLVKLNREDLLRKQRTFDNGSIPHQIHLGELHAILRRQEDFYPFLKD
NREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLING
IRDKQSGKTILDFLKSDGFANRNFMQLIHDDSLTFKEDIQKAQVSGQGDSLHEHIANLA
 
GSPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVPQ
SFLKDDSIDNKVLTRSDKNRGKSDNVPSEEVVKKMKNYWRQLLNAKLITQRKFDNLT
KAERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVYD
VRKMIAKSEQEIGSGGSGGSGGSTNLSDIIEKETGKQLVIQESILMLPEEVEEVIGNKPES
DILVHTAYDESTDENVMLLTSDAPEYKPWALVIQDSNGENKIKMLSGGSGGSGGSTNLS
DIIEKETGKQLVIQESILMLPEEVEEVIGNKPESDILVHTAYDESTDENVMLLTSDAPEYK
PWALVIQDSNGENKIKMLSGGSKRTADGSEFEPKKKRKV
 The underlined part represents 64aa linker.
>SEQ ID NO:176 mini-Sdd7-QBE2 
MKRTADGSEFESPKKKRKVARLSKSRRLENLIAQLPGEKKNGLFGNLIALSLGLTP
NFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQE
DFYPFLKDNREKIEKILTFRIPYYVGPLARGNSRFAWMTRKSEETITPWNFEEVVDKGA
SAQSFIERMTNFDKNLPNEKVLPKHSLLYEYFTVYNELTKVKYVTEGMRKPAFLSGEQ
KKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSL
HEHIANLAGSPAIKKGILQTVKVVDELVKVMGRHKPENIVIEMARENQTTQKGQKNSR
ERMKRIEEGIKELGSQILKEHPVENTQLQNEKLYLYYLQNGRDMYVDQELDINRLSDY
DVDHIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVY
GDYKVYDVRKMIAKSEQEIGKATAKYFFYSNIMNFFKTEITLANGEISGGSSGGSSGSE
TPGTSESATPESSGGSSGGSEGGGPGAVPEGGDGPPAVPAEEVERLRGELPPPVVPGTGQ
KTHGRWIGPDGRVRAIVSGRDEDAALVHAQLAAKGIPDEPTRNSDVEQKLAAHMVAN
GIRHVTLVINHRPCRGFDDSCDTLVPIILPEGCTLTVHGQTDKGMRVRVRYTGGARPW
WSSGGSSGGSSGSETPGTSESATPESSGGSSGGSRKRPLIETNGETGEIVWDKGRDFATV
RKVLSMPQVNIVKKTEVQTGGFSKESILPKRNSDKLIARKKDWDPKKYGGFDSPTVAY
SVLVVAKVEKGKSKKLKSVKELLGITIMERSSFEKNPIDFLEAKGYKEVKKDLIIKLPKY
SLFELENGRKRMLASAGELQKGNELALPSKYVNFLYLASHYEKLKGSPEDNEQKQLF
VEQHKHYLDEIIEQISEFSKRVILADANLDKVLSAYNKHRDKPIREQAENIIHLFTLTNLG
 
APAAFKYFDTTIDRKRYTSTKEVLDATLIHQSITGLYETRIDLSQLGGDGGSGGSGGSGG
SGGSGGSGGMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHF
LIEGDLNPDNSDVDKLFIQLVQTYNQLFEENPINASGVDAKAILSSGGSGGSGGSTNLS
DIIEKETGKQLVIQESILMLPEEVEEVIGNKPESDILVHTAYDESTDENVMLLTSDAPEYK
PWALVIQDSNGENKIKMLSGGSGGSGGSTNLSDIIEKETGKQLVIQESILMLPEEVEEVI
GNKPESDILVHTAYDESTDENVMLLTSDAPEYKPWALVIQDSNGENKIKMLSGGSKRTA
DGSEFEPKKKRKV
>SEQ ID NO:177 mini-Sdd7-QBE4 
MKRTADGSEFESPKKKRKVEIGKATAKYFFYSNIMNFFKTEITLANGEIRKRPLIET
NGETGEIVWDKGRDFATVRKVLSMPQVNIVKKTEVQTGGFSKESILPKRNSDKLIARK
KDWDPKKYGGFDSPTVAYSVLVVAKVEKGKSKKLKSVKELLGITIMERSSFEKNPIDFL
EAKGYKEVKKDLIIKLPKYSLFELENGRKRMLASAGELQKGNELALPSKYVNFLYLAS
HYEKLKGSPEDNEQKQLFVEQHKHYLDEIIEQISEFSKRVILADANLDKVLSAYNKHRD
KPIREQAENIIHLFTLTNLGAPAAFKYFDTTIDRKRYTSTKEVLDATLIHQSITGLYETRID
LSQLGGDGGSGGSGGSGGSGGSGGSGGMDKKYSIGLAIGTNSVGWAVITDEYKVPSK
KFKVLGNTDRHSIKKNLIGALLFDSGETAEATRLKRTARRRYTRRKNRICYLQEIFSNE
MAKVDDSFFHRLEESFLVEEDKKHERHPIFGNIVDEVAYHEKYPTIYHLRKKLVDSTDK
ADLRLIYLALAHMIKFRGHFLIEGDLNPDNSDVDKLFIQLVQTYNQLFEENPINASGVD
AKAILSARLSKSRRLENLIAQLPGEKKNGLFGNLIALSLGLTPNFKSNFDLAEDAKLQLS
KDTYDDDLDNLLAQIGDQYADLFLAAKNLSDAILLSDILRVNTEITKAPLSASMIKRYD
EHHQDLTLLKALVRQQLPEKYKEIFFDQSKNGYAGYIDGGASQEEFYKFIKPILEKMDG
TEELLVKLNREDLLRKQRTFDNGSIPHQIHLGELHAILRRQEDFYPFLKDNREKIEKILTF
RIPYYVGPLARGNSRFAWMTRKSEETITPWNFEEVVDKGASAQSFIERMTNFDKNLPN
EKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSIDNK
VLTRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSESG
 
GSSGGSSGSETPGTSESATPESSGGSSGGSEGGGPGAVPEGGDGPPAVPAEEVERLRGEL
PPPVVPGTGQKTHGRWIGPDGRVRAIVSGRDEDAALVHAQLAAKGIPDEPTRNSDVEQ
KLAAHMVANGIRHVTLVINHRPCRGFDDSCDTLVPIILPEGCTLTVHGQTDKGMRVRVR
YTGGARPWWSSGGSSGGSSGSETPGTSESATPESSGGSSGGSQSGGSGGSGGSTNLSDII
EKETGKQLVIQESILMLPEEVEEVIGNKPESDILVHTAYDESTDENVMLLTSDAPEYKPW
ALVIQDSNGENKIKMLSGGSGGSGGSTNLSDIIEKETGKQLVIQESILMLPEEVEEVIGN
KPESDILVHTAYDESTDENVMLLTSDAPEYKPWALVIQDSNGENKIKMLSGGSKRTADG
SEFEPKKKRKV
>SEQ ID NO:178 mini-Sdd7-QBE1 
MKRTADGSEFESPKKKRKVNIMNFFKTEITLANGEIRKRPLIETNGETGEIVWDKG
RDFATVRKVLSMPQVNIVKKTEVQTGGFSKESILPKRNSDKLIARKKDWDPKKYGGFD
SPTVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDNE
QKQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGDGGSGG
SGGSGGSGGSGGSGGMDKKYSIGLAIGTNSVGWAVITDEYKVPSKKFKVLGNTDRHSI
KKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHM
IKFRGHFLIEGDLNPDNSDVDKLFIQLVQTYNQLFEENPINASGVDAKAILSARLSKSRR
LENLIAQLPGEKKNGLFGNLIALSLGLTPNFKSNFDLAEDAKLQLSKDTYDDDLDNLL
AQIGDQYADLFLAAKNLSDAILLSDILRVNTEITKAPLSASMIKRYDEHHQDLTLLKALV
RQQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLARGN
SRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECF
DSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVM
GRHKPENIVIEMARENQTTQKGQKNSRERMKRIEEGIKELGSQILKEHPVENTQLQNE
KLYLYYLQNGRDMYVDQELDINRLSDYDVDHIVPQSFLKDDSIDNKVLTRSDKNRGKS
DNVPSEEVVKKMKNYWRQLLNAKLITQRKFDNLTKAERGGLSELDKAGFIKRQLVET
RQITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSESGGSSGGSSGSETPG
TSESATPESSGGSSGGSEGGGPGAVPEGGDGPPAVPAEEVERLRGELPPPVVPGTGQKT
 
HGRWIGPDGRVRAIVSGRDEDAALVHAQLAAKGIPDEPTRNSDVEQKLAAHMVANGI
RHVTLVINHRPCRGFDDSCDTLVPIILPEGCTLTVHGQTDKGMRVRVRYTGGARPWWS
SGGSSGGSSGSETPGTSESATPESSGGSSGGSQEIGKATAKYFFYSSGGSGGSGGSTNLS
DIIEKETGKQLVIQESILMLPEEVEEVIGNKPESDILVHTAYDESTDENVMLLTSDAPEYK
PWALVIQDSNGENKIKMLSGGSGGSGGSTNLSDIIEKETGKQLVIQESILMLPEEVEEVI
GNKPESDILVHTAYDESTDENVMLLTSDAPEYKPWALVIQDSNGENKIKMLSGGSKRTA
DGSEFEPKKKRKV
>SEQ ID NO:179 Sdd7-QBE3 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSLEAVRARLIGEGGGPGAVPEGGDGPP
AVPAEEVERLRGELPPPVVPGTGQKTHGRWIGPDGRVRAIVSGRDEDAALVHAQLAAK
GIPDEPTRNSDVEQKLAAHMVANGIRHVTLVINHRPCRGFDDSCDTLVPIILPEGCTLTV
HGQTDKGMRVRVRYTGGARPWWSSGGSSGGSSGSETPGTSESATPESSGGSSGGSLKG
SPEDNEQKQLFVEQHKHYLDEIIEQISEFSKRVILADANLDKVLSAYNKHRDKPIREQA
ENIIHLFTLTNLGAPAAFKYFDTTIDRKRYTSTKEVLDATLIHQSITGLYETRIDLSQLGG
DGGSGGSGGSGGSGGSGGSGGMDKKYSIGLAIGTNSVGWAVITDEYKVPSKKFKVLG
NTDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRLIY
LALAHMIKFRGHFLIEGDLNPDNSDVDKLFIQLVQTYNQLFEENPINASGVDAKAILSA
RLSKSRRLENLIAQLPGEKKNGLFGNLIALSLGLTPNFKSNFDLAEDAKLQLSKDTYDD
DLDNLLAQIGDQYADLFLAAKNLSDAILLSDILRVNTEITKAPLSASMIKRYDEHHQDL
TLLKALVRQQLPEKYKEIFFDQSKNGYAGYIDGGASQEEFYKFIKPILEKMDGTEELLV
KLNREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLPK
HSLLYEYFTVYNELTKVKYVTEGMRKPAFLSGEQKKAIVDLLFKTNRKVTVKQLKED
YFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSD
GFANRNFMQLIHDDSLTFKEDIQKAQVSGQGDSLHEHIANLAGSPAIKKGILQTVKVVD
ELVKVMGRHKPENIVIEMARENQTTQKGQKNSRERMKRIEEGIKELGSQILKEHPVEN
TQLQNEKLYLYYLQNGRDMYVDQELDINRLSDYDVDHIVPQSFLKDDSIDNKVLTRSD
KNRGKSDNVPSEEVVKKMKNYWRQLLNAKLITQRKFDNLTKAERGGLSELDKAGFIK
 
RQLVETRQITKHVAQILDSRMNTKYDENDKLIREVKVITLKSKLVSDFRKDFQFYKVRE
INNYHHAHDAYLNAVVGTALIKKYPKLESEFVYGDYKVYDVRKMIAKSEQEIGSGGSG
GSGGSTNLSDIIEKETGKQLVIQESILMLPEEVEEVIGNKPESDILVHTAYDESTDENVML
LTSDAPEYKPWALVIQDSNGENKIKMLSGGSGGSGGSTNLSDIIEKETGKQLVIQESILM
LPEEVEEVIGNKPESDILVHTAYDESTDENVMLLTSDAPEYKPWALVIQDSNGENKIKM
LSGGSKRTADGSEFEPKKKRKV
>SEQ ID NO:180 Sdd7-BE4max 
MKRTADGSEFESPKKKRKVLEAVRARLIGEGGGPGAVPEGGDGPPAVPAEEVERL
RGELPPPVVPGTGQKTHGRWIGPDGRVRAIVSGRDEDAALVHAQLAAKGIPDEPTRNS
DVEQKLAAHMVANGIRHVTLVINHRPCRGFDDSCDTLVPIILPEGCTLTVHGQTDKGM
RVRVRYTGGARPWWSSGGSSGGSSGSETPGTSESATPESSGGSSGGSDKKYSIGLAIGT
NSVGWAVITDEYKVPSKKFKVLGNTDRHSIKKNLIGALLFDSGETAEATRLKRTARRRY
TRRKNRICYLQEIFSNEMAKVDDSFFHRLEESFLVEEDKKHERHPIFGNIVDEVAYHEK
YPTIYHLRKKLVDSTDKADLRLIYLALAHMIKFRGHFLIEGDLNPDNSDVDKLFIQLVQ
TYNQLFEENPINASGVDAKAILSARLSKSRRLENLIAQLPGEKKNGLFGNLIALSLGLTP
NFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQE
DFYPFLKDNREKIEKILTFRIPYYVGPLARGNSRFAWMTRKSEETITPWNFEEVVDKGA
SAQSFIERMTNFDKNLPNEKVLPKHSLLYEYFTVYNELTKVKYVTEGMRKPAFLSGEQ
KKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSL
HEHIANLAGSPAIKKGILQTVKVVDELVKVMGRHKPENIVIEMARENQTTQKGQKNSR
ERMKRIEEGIKELGSQILKEHPVENTQLQNEKLYLYYLQNGRDMYVDQELDINRLSDY
DVDHIVPQSFLKDDSIDNKVLTRSDKNRGKSDNVPSEEVVKKMKNYWRQLLNAKLIT
QRKFDNLTKAERGGLSELDKAGFIKRQLVETRQITKIIVAQILDSRMNTKYDENDKLIRE
VKVITLKSKLVSDFRKDFQFYKVREINNYHHAHDAYLNAVVGTALIKKYPKLESEFVY
GDYKVYDVRKMIAKSEQEIGKATAKYFFYSNIMNFFKTEITLANGEIRKRPLIETNGET
GEIVWDKGRDFATVRKVLSMPQVNIVKKTEVQTGGFSKESILPKRNSDKLIARKKDWD
PKKYGGFDSPTVAYSVLVVAKVEKGKSKKLKSVKELLGITIMERSSFEKNPIDFLEAKG
YKEVKKDLIIKLPKYSLFELENGRKRMLASAGELQKGNELALPSKYVNFLYLASHYEK
LKGSPEDNEQKQLFVEQHKHYLDEIIEQISEFSKRVILADANLDKVLSAYNKHRDKPIR
EQAENIIHLFTLTNLGAPAAFKYFDTTIDRKRYTSTKEVLDATLIHQSITGLYETRIDLSQ
 
LGGDSGGSGGSGGSTNLSDIIEKETGKQLVIQESILMLPEEVEEVIGNKPESDILVHTAYD
ESTDENVMLLTSDAPEYKPWALVIQDSNGENKIKMLSGGSGGSGGSTNLSDIIEKETGK
QLVIQESILMLPEEVEEVIGNKPESDILVHTAYDESTDENVMLLTSDAPEYKPWALVIQD
SNGENKIKMLSGGSKRTADGSEFEPKKKRKV
>SEQ ID NO: 181 QBE4-X₁ 
EIGKATAKYFFYSNIMNFFKTEITLANGEIRKRPLIETNGETGEIVWDKGRDFATVR
KVLSMPQVNIVKKTEVQTGGFSKESILPKRNSDKLIARKKDWDPKKYGGFDSPTVAYS
VLVVAKVEKGKSKKLKSVKELLGITIMERSSFEKNPIDFLEAKGYKEVKKDLIIKLPKYS
LFELENGRKRMLASAGELQKGNELALPSKYVNFLYLASHYEKLKGSPEDNEQKQLFV
EQHKHYLDEIIEQISEFSKRVILADANLDKVLSAYNKHRDKPIREQAENIIHLFTLTNLGA
PAAFKYFDTTIDRKRYTSTKEVLDATLIHQSITGLYETRIDLSQLGGD
>SEQ ID NO: 182 QBE4-X₂ 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILSARLSKSRRLENLIAQLPGEKK
NGLFGNLIALSLGLTPNFKSNFDLAEDAKLQLSKDTYDDDLDNLLAQIGDQYADLFLA
AKNLSDAILLSDILRVNTEITKAPLSASMIKRYDEHHQDLTLLKALVRQQLPEKYKEIFF
DQSKNGYAGYIDGGASQEEFYKFIKPILEKMDGTEELLVKLNREDLLRKQRTFDNGSIP
HQIHLGELHAILRRQEDFYPFLKDNREKIEKILTFRIPYYVGPLARGNSRFAWMTRKSEE
TITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRF
NASLGTYHDLLKIIKDKDFLDNEENEDILEDIVLTLTLFEDREMIEERLKTYAHLFDDKV
MKQLKRRRYTGWGRLSRKLINGIRDKQSGKTILDFLKSDGFANRNFMQLIHDDSLTFK
EDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRD
MYVDQELDINRLSDYDVDHIVPQSFLKDDSIDNKVLTRSDKNRGKSDNVPSEEVVKK
MKNYWRQLLNAKLITQRKFDNLTKAERGGLSELDKAGFIKRQLVETRQITKHVAQILD
SRMNTKYDENDKLIREVKVITLKSKLVSDFRKDFQFYKVREINNYHHAHDAYLNAVV
GTALIKKYPKLESEFVYGDYKVYDVRKMIAKSE
> QBE4-X₃
   Q
> SEQ ID NO: 183 QBE84-X₂ 
ARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDEH
HQDLTLLKALVRQQLPEKYKEIFFDQSKNGYAGYIDGGASQEEFYKFIKPILEKMDGTE
ELLVKLNREDLLRKQRTFDNGSIPHQIHLGELHAILRRQEDFYPFLKDNREKIEKILTFRI
PYYVGPLARGNSRFAWMTRKSEETITPWNFEEVVDKGASAQSFIERMTNFDKNLPNEK
VLPKHSLLYEYFTVYNELTKVKYVTEGMRKPAFLSGEQKKAIVDLLFKTNRKVTVKQL
KEDYFKKIECFDSVEISGVEDRFNASLGTYHDLLKIIKDKDFLDNEENEDILEDIVLTLTL
FEDREMIEERLKTYAHLFDDKVMKQLKRRRYTGWGRLSRKLINGIRDKQSGKTILDFL
KSDGFANRNFMQLIHDDSLTFKEDIQKAQVSGQGDSLHEHIANLAGSPAIKKGILQTVK
VVDELVKVMGRHKPENIVIEMARENQTTQKGQKNSRERMKRIEEGIKELGSQILKEHP
VENTQLQNEKLYLYYLQNGRDMYVDQELDINRLSDYDVDHIVPQSFLKDDSIDNKVLT
RSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFY
KVREINNYHHAHDAYLNAVVGTALIKKYPKLESEFVYGDYKVYDVRKMIAKSE
> SEQ ID NO: 184 QBE84-X₃ 
QEIGKATAKYFFYSNIMNFFKTEITLANGEIRKRPLIETNGETGEIVWDKGRDFATV
RKVLSMPQVNIVKKTEVQTGGFSKESILPKRNSDKLIARKKDWDPKKYGGFDSPTVAY
SVLVVAKVEKGKSKKLKSVKELLGITIMERSSFEKNPIDFLEAKGYKEVKKDLIIKLPKY
SLFELENGRKRMLASAGELQKGNELALPSKYVNFLYLASHYEKLKGSPEDNEQKQLF
VEQHKHYLDEIIEQISEFSKRVILADANLDKVLSAYNKHRDKPIREQAENIIHLFTLTNLG
APAAFKYFDTTIDRKRYTSTKEVLDATLIHQSITGLYETRIDLSQLGGD
> SEQ ID NO: 185 QBE84-X₄ 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILS
> SEQ ID NO: 186 QBE85-X₂ 
EKKNGLFGNLIALSLGLTPNFKSNFDLAEDAKLQLSKDTYDDDLDNLLAQIGDQY
ADLFLAAKNLSDAILLSDILRVNTEITKAPLSASMIKRYDEHHQDLTLLKALVRQQLPE
KYKEIFFDQSKNGYAGYIDGGASQEEFYKFIKPILEKMDGTEELLVKLNREDLLRKQRT
 
FDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYN
ELTKVKYVTEGMRKPAFLSGEQKKAIVDLLFKTNRKVTVKQLKEDYFKKIECFDSVEIS
GVEDRFNASLGTYHDLLKIIKDKDFLDNEENEDILEDIVLTLTLFEDREMIEERLKTYAH
LFDDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKPE
NIVIEMARENQTTQKGQKNSRERMKRIEEGIKELGSQILKEHPVENTQLQNEKLYLYYL
QNGRDMYVDQELDINRLSDYDVDHIVPQSFLKDDSIDNKVLTRSDKNRGKSDNVPSEE
VVKKMKNYWRQLLNAKLITQRKFDNLTKAERGGLSELDKAGFIKRQLVETRQITKHV
AQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFK
TEITLANGEIRKRPLIETNGETGEIVWDKGRDFATVRKVLSMPQVNIVKKTEVQTGGFS
KESILPKRNSDKLIARKKDWDPKKYGGFDSPTVAYSVLVVAKVEKGKSKKLKSVKELL
GITIMERSSFEKNPIDFLEAKGYKEVKKDLIIKLPKYSLFELENGRKRMLASAGELQKG
NELALPSKYVNFLYLASHYEK
> SEQ ID NO: 187 QBE85-X₃ 
LKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGD
> SEQ ID NO: 188 QBE85-X₄ 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILSARLSKSRRLENLIAQLPG
> SEQ ID NO: 189 QBE87-X₁ 
KATAKYFFYSNIMNFFKTEITLANGEIRKRPLIETNGETGEIVWDKGRDFATVRKV
LSMPQVNIVKKTEVQTGGFSKESILPKRNSDKLIARKKDWDPKKYGGFDSPTVAYSVL
VVAKVEKGKSKKLKSVKELLGITIMERSSFEKNPIDFLEAKGYKEVKKDLIIKLPKYSLF
ELENGRKRMLASAGELQKGNELALPSKYVNFLYLASHYEKLKGSPEDNEQKQLFVEQ
HKHYLDEIIEQISEFSKRVILADANLDKVLSAYNKHRDKPIREQAENIIHLFTLTNLGAPA
AFKYFDTTIDRKRYTSTKEVLDATLIHQSITGLYETRIDLSQLGGD
> SEQ ID NO: 190 QBE87-X₂ 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPIN
> SEQ ID NO: 191 QBE87-X₃ 
ASGVDAKAILSARLSKSRRLENLIAQLPGEKKNGLFGNLIALSLGLTPNFKSNFDL
AEDAKLQLSKDTYDDDLDNLLAQIGDQYADLFLAAKNLSDAILLSDILRVNTEITKAPL
SASMIKRYDEHHQDLTLLKALVRQQLPEKYKEIFFDQSKNGYAGYIDGGASQEEFYKFI
KPILEKMDGTEELLVKLNREDLLRKQRTFDNGSIPHQIHLGELHAILRRQEDFYPFLKD
NREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLING
IRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVPQ
SFLKDDSIDNKVLTRSDKNRGKSDNVPSEEVVKKMKNYWRQLLNAKLITQRKFDNLT
KAERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVYD
VRKMIAKSEQEIG
> SEQ ID NO: 192 QBE88-X₁ 
EKGKSKKLKSVKELLGITIMERSSFEKNPIDFLEAKGYKEVKKDLIIKLPKYSLFEL
ENGRKRMLASAGELQKGNELALPSKYVNFLYLASHYEKLKGSPEDNEQKQLFVEQHK
HYLDEIIEQISEFSKRVILADANLDKVLSAYNKHRDKPIREQAENIIHLFTLTNLGAPAAF
KYFDTTIDRKRYTSTKEVLDATLIHQSITGLYETRIDLSQLGGD
> SEQ ID NO: 193 QBE88-X₂ 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPIN
> SEQ ID NO: 194 QBE88-X₃ 
ASGVDAKAILSARLSKSRRLENLIAQLPGEKKNGLFGNLIALSLGLTPNFKSNFDL
AEDAKLQLSKDTYDDDLDNLLAQIGDQYADLFLAAKNLSDAILLSDILRVNTEITKAPL
SASMIKRYDEHHQDLTLLKALVRQQLPEKYKEIFFDQSKNGYAGYIDGGASQEEFYKFI
KPILEKMDGTEELLVKLNREDLLRKQRTFDNGSIPHQIHLGELHAILRRQEDFYPFLKD
NREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLING
IRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVPQ
SFLKDDSIDNKVLTRSDKNRGKSDNVPSEEVVKKMKNYWRQLLNAKLITQRKFDNLT
KAERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVYD
VRKMIAKSEQEIGKATAKYFFYSNIMNFFKTEITLANGEIRKRPLIETNGETGEIVWDKG
RDFATVRKVLSMPQVNIVKKTEVQTGGFSKESILPKRNSDKLIARKKDWDPKKYGGFD
SPTVAYSVLVVAKV
> SEQ ID NO: 195 QBE89-X₁ 
KATAKYFFYSNIMNFFKTEITLANGEIRKRPLIETNGETGEIVWDKGRDFATVRKV
LSMPQVNIVKKTEVQTGGFSKESILPKRNSDKLIARKKDWDPKKYGGFDSPTVAYSVL
VVAKVEKGKSKKLKSVKELLGITIMERSSFEKNPIDFLEAKGYKEVKKDLIIKLPKYSLF
ELENGRKRMLASAGELQKGNELALPSKYVNFLYLASHYEKLKGSPEDNEQKQLFVEQ
HKHYLDEIIEQISEFSKRVILADANLDKVLSAYNKHRDKPIREQAENIIHLFTLTNLGAPA
AFKYFDTTIDRKRYTSTKEVLDATLIHQSITGLYETRIDLSQLGGD
> SEQ ID NO: 196 QBE89-X₂ 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILSARLSKSRRLENLIAQLPGEKK
NGLFGNLIALSLGLTPNFKSNFDLAEDAKLQLSKDTYDDDLDNLLAQIGDQYADLFLA
AKNLSDAILLSDILRVNTEITKAPLSASMIKRYDEHHQDLTLLKALVRQQLPEKYKEIFF
DQSKNGYAGYIDGGASQEEFYKFIKPILEKMDGTEELLVKLNREDLLRKQRTFDNGSIP
 HQIHLGELHAILRRQEDFYPFLKDNREKIEKILTFRIPYYVGPLARGNSRFAWMTRK
> SEQ ID NO: 197 QBE89-X₃ 
SEETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHL
FDDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKPE
NIVIEMARENQTTQKGQKNSRERMKRIEEGIKELGSQILKEHPVENTQLQNEKLYLYYL
QNGRDMYVDQELDINRLSDYDVDHIVPQSFLKDDSIDNKVLTRSDKNRGKSDNVPSEE
VVKKMKNYWRQLLNAKLITQRKFDNLTKAERGGLSELDKAGFIKRQLVETRQITKHV
AQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIG
> SEQ ID NO: 198 QBE90-X₁ 
EKGKSKKLKSVKELLGITIMERSSFEKNPIDFLEAKGYKEVKKDLIIKLPKYSLFEL
ENGRKRMLASAGELQKGNELALPSKYVNFLYLASHYEKLKGSPEDNEQKQLFVEQHK
HYLDEIIEQISEFSKRVILADANLDKVLSAYNKHRDKPIREQAENIIHLFTLTNLGAPAAF
KYFDTTIDRKRYTSTKEVLDATLIHQSITGLYETRIDLSQLGGD
> SEQ ID NO: 199 QBE90-X₂ 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILSARLSKSRRLENLIAQLPGEKK
NGLFGNLIALSLGLTPNFKSNFDLAEDAKLQLSKDTYDDDLDNLLAQIGDQYADLFLA
AKNLSDAILLSDILRVNTEITKAPLSASMIKRYDEHHQDLTLLKALVRQQLPEKYKEIFF
DQSKNGYAGYIDGGASQEEFYKFIKPILEKMDGTEELLVKLNREDLLRKQRTFDNGSIP
HQIHLGELHAILRRQEDFYPFLKDNREKIEKILTFRIPYYVGPLARGNSRFAWMTRK
> SEQ ID NO: 200 QBE90-X₃ 
SEETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHL
FDDKVMKQLKRRRYTGWGRLSRKLINGIRDKQSGKTILDFLKSDGFANRNFMQLIHD
 
DSLTFKEDIQKAQVSGQGDSLHEHIANLAGSPAIKKGILQTVKVVDELVKVMGRHKPE
NIVIEMARENQTTQKGQKNSRERMKRIEEGIKELGSQILKEHPVENTQLQNEKLYLYYL
QNGRDMYVDQELDINRLSDYDVDHIVPQSFLKDDSIDNKVLTRSDKNRGKSDNVPSEE
VVKKMKNYWRQLLNAKLITQRKFDNLTKAERGGLSELDKAGFIKRQLVETRQITKHV
AQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFK
TEITLANGEIRKRPLIETNGETGEIVWDKGRDFATVRKVLSMPQVNIVKKTEVQTGGFS
KESILPKRNSDKLIARKKDWDPKKYGGFDSPTVAYSVLVVAKV
> SEQ ID NO:201 mini-Sdd9-QBE84 
ARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDEH
HQDLTLLKALVRQQLPEKYKEIFFDQSKNGYAGYIDGGASQEEFYKFIKPILEKMDGTE
ELLVKLNREDLLRKQRTFDNGSIPHQIHLGELHAILRRQEDFYPFLKDNREKIEKILTFRI
PYYVGPLARGNSRFAWMTRKSEETITPWNFEEVVDKGASAQSFIERMTNFDKNLPNEK
VLPKHSLLYEYFTVYNELTKVKYVTEGMRKPAFLSGEQKKAIVDLLFKTNRKVTVKQL
KEDYFKKIECFDSVEISGVEDRFNASLGTYHDLLKIIKDKDFLDNEENEDILEDIVLTLTL
FEDREMIEERLKTYAHLFDDKVMKQLKRRRYTGWGRLSRKLINGIRDKQSGKTILDFL
KSDGFANRNFMQLIHDDSLTFKEDIQKAQVSGQGDSLHEHIANLAGSPAIKKGILQTVK
VVDELVKVMGRHKPENIVIEMARENQTTQKGQKNSRERMKRIEEGIKELGSQILKEHP
VENTQLQNEKLYLYYLQNGRDMYVDQELDINRLSDYDVDHIVPQSFLKDDSIDNKVLT
RSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFY
KVREINNYHHAHDAYLNAVVGTALIKKYPKLESEFVYGDYKVYDVRKMIAKSESGGS
SGGSSGSETPGTSESATPESSGGSSGGSCPIWVKNALQELVGRKETSGKVFDVDGKPIGP
DIQSGYKDRELRRGVYETLRKSPFFQKHFPSTATWYVSLHVEAQYAVWMLRNRIKHAT
VVINNTYVCSDMNRLHDNCMTAVPHILPEGYTMTVWKADRTEVTLRGKAPKESGGSS
GGSSGSETPGTSESATPESSGGSSGGSQEIGKATAKYFFYSNIMNFFKTEITLANGEIRKR
PLIETNGETGEIVWDKGRDFATVRKVLSMPQVNIVKKTEVQTGGFSKESILPKRNSDKL
IARKKDWDPKKYGGFDSPTVAYSVLVVAKVEKGKSKKLKSVKELLGITIMERSSFEKN
PIDFLEAKGYKEVKKDLIIKLPKYSLFELENGRKRMLASAGELQKGNELALPSKYVNFL
YLASHYEKLKGSPEDNEQKQLFVEQHKHYLDEIIEQISEFSKRVILADANLDKVLSAYN
KHRDKPIREQAENIIHLFTLTNLGAPAAFKYFDTTIDRKRYTSTKEVLDATLIHQSITGLY
ETRIDLSQLGGDGGSGGSGGSGGSGGSGGMDKKYSIGLAIGTNSVGWAVITDEYK
VPSKKFKVLGNTDRHSIKKNLIGALLFDSGETAEATRLKRTARRRYTRRKNRICYLQEIF
 
SNEMAKVDDSFFHRLEESFLVEEDKKHERHPIFGNIVDEVAYHEKYPTIYHLRKKLVDS
TDKADLRLIYLALAHMIKFRGHFLIEGDLNPDNSDVDKLFIQLVQTYNQLFEENPINAS
GVDAKAILS
> SEQ ID NO:202 mini-Sdd9-QBE85 
EKKNGLFGNLIALSLGLTPNFKSNFDLAEDAKLQLSKDTYDDDLDNLLAQIGDQY
ADLFLAAKNLSDAILLSDILRVNTEITKAPLSASMIKRYDEHHQDLTLLKALVRQQLPE
KYKEIFFDQSKNGYAGYIDGGASQEEFYKFIKPILEKMDGTEELLVKLNREDLLRKQRT
FDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYN
ELTKVKYVTEGMRKPAFLSGEQKKAIVDLLFKTNRKVTVKQLKEDYFKKIECFDSVEIS
GVEDRFNASLGTYHDLLKIIKDKDFLDNEENEDILEDIVLTLTLFEDREMIEERLKTYAH
LFDDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKPE
NIVIEMARENQTTQKGQKNSRERMKRIEEGIKELGSQILKEHPVENTQLQNEKLYLYYL
QNGRDMYVDQELDINRLSDYDVDHIVPQSFLKDDSIDNKVLTRSDKNRGKSDNVPSEE
VVKKMKNYWRQLLNAKLITQRKFDNLTKAERGGLSELDKAGFIKRQLVETRQITKHV
AQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFK
TEITLANGEIRKRPLIETNGETGEIVWDKGRDFATVRKVLSMPQVNIVKKTEVQTGGFS
KESILPKRNSDKLIARKKDWDPKKYGGFDSPTVAYSVLVVAKVEKGKSKKLKSVKELL
GITIMERSSFEKNPIDFLEAKGYKEVKKDLIIKLPKYSLFELENGRKRMLASAGELQKG
NELALPSKYVNFLYLASHYEKSGGSSGGSSGSETPGTSESATPESSGGSSGGSCPIWVKN
ALQELVGRKETSGKVFDVDGKPIGPDIQSGYKDRELRRGVYETLRKSPFFQKHFPSTAT
WYVSLHVEAQYAVWMLRNRIKHATVVINNTYVCSDMNRLHDNCMTAVPHILPEGYT
MTVWKADRTEVTLRGKAPKESGGSSGGSSGSETPGTSESATPESSGGSSGGSLKGSPED
NEQKQLFVEQHKHYLDEIIEQISEFSKRVILADANLDKVLSAYNKHRDKPIREQAENIIH
LFTLTNLGAPAAFKYFDTTIDRKRYTSTKEVLDATLIHQSITGLYETRIDLSQLGGDGGS
GGSGGSGGSGGSGGSGGMDKKYSIGLAIGTNSVGWAVITDEYKVPSKKFKVLGNTDR
HSIKKNLIGALLFDSGETAEATRLKRTARRRYTRRKNRICYLQEIFSNEMAKVDDSFFHR
LEESFLVEEDKKHERHPIFGNIVDEVAYHEKYPTIYHLRKKLVDSTDKADLRLIYLALA
HMIKFRGHFLIEGDLNPDNSDVDKLFIQLVQTYNQLFEENPINASGVDAKAILSARLSK
SRRLENLIAQLPG
> SEQ ID NO:203 mini-Sdd9-QBE87 
KATAKYFFYSNIMNFFKTEITLANGEIRKRPLIETNGETGEIVWDKGRDFATVRKV
LSMPQVNIVKKTEVQTGGFSKESILPKRNSDKLIARKKDWDPKKYGGFDSPTVAYSVL
VVAKVEKGKSKKLKSVKELLGITIMERSSFEKNPIDFLEAKGYKEVKKDLIIKLPKYSLF
ELENGRKRMLASAGELQKGNELALPSKYVNFLYLASHYEKLKGSPEDNEQKQLFVEQ
HKHYLDEIIEQISEFSKRVILADANLDKVLSAYNKHRDKPIREQAENIIHLFTLTNLGAPA
AFKYFDTTIDRKRYTSTKEVLDATLIHQSITGLYETRIDLSQLGGDGGSGGSGGSGGSG
GSGGSGGMDKKYSIGLAIGTNSVGWAVITDEYKVPSKKFKVLGNTDRHSIKKNLIGAL
LFDSGETAEATRLKRTARRRYTRRKNRICYLQEIFSNEMAKVDDSFFHRLEESFLVEED
KKHERHPIFGNIVDEVAYHEKYPTIYHLRKKLVDSTDKADLRLIYLALAHMIKFRGHFL
IEGDLNPDNSDVDKLFIQLVQTYNQLFEENPINSGGSSGGSSGSETPGTSESATPESSGGS
SGGSCPIWVKNALQELVGRKETSGKVFDVDGKPIGPDIQSGYKDRELRRGVYETLRKS
PFFQKHFPSTATWYVSLHVEAQYAVWMLRNRIKHATVVINNTYVCSDMNRLHDNCMT
AVPHILPEGYTMTVWKADRTEVTLRGKAPKESGGSSGGSSGSETPGTSESATPESSGGS
SGGSASGVDAKAILSARLSKSRRLENLIAQLPGEKKNGLFGNLIALSLGLTPNFKSNFDL
AEDAKLQLSKDTYDDDLDNLLAQIGDQYADLFLAAKNLSDAILLSDILRVNTEITKAPL
SASMIKRYDEHHQDLTLLKALVRQQLPEKYKEIFFDQSKNGYAGYIDGGASQEEFYKFI
KPILEKMDGTEELLVKLNREDLLRKQRTFDNGSIPHQIHLGELHAILRRQEDFYPFLKD
NREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLING
IRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVPQ
SFLKDDSIDNKVLTRSDKNRGKSDNVPSEEVVKKMKNYWRQLLNAKLITQRKFDNLT
KAERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVYD
VRKMIAKSEQEIG
> SEQ ID NO:204 mini-Sdd9-QBE88 
EKGKSKKLKSVKELLGITIMERSSFEKNPIDFLEAKGYKEVKKDLIIKLPKYSLFEL
ENGRKRMLASAGELQKGNELALPSKYVNFLYLASHYEKLKGSPEDNEQKQLFVEQHK
HYLDEIIEQISEFSKRVILADANLDKVLSAYNKHRDKPIREQAENIIHLFTLTNLGAPAAF
KYFDTTIDRKRYTSTKEVLDATLIHQSITGLYETRIDLSQLGGDGGSGGSGGSGGSGGS
 
GGSGGMDKKYSIGLAIGTNSVGWAVITDEYKVPSKKFKVLGNTDRHSIKKNLIGALLF
DSGETAEATRLKRTARRRYTRRKNRICYLQEIFSNEMAKVDDSFFHRLEESFLVEEDKK
HERHPIFGNIVDEVAYHEKYPTIYHLRKKLVDSTDKADLRLIYLALAHMIKFRGHFLIEG
DLNPDNSDVDKLFIQLVQTYNQLFEENPINSGGSSGGSSGSETPGTSESATPESSGGSSG
GSCPIWVKNALQELVGRKETSGKVFDVDGKPIGPDIQSGYKDRELRRGVYETLRKSPF
FQKHFPSTATWYVSLHVEAQYAVWMLRNRIKHATVVINNTYVCSDMNRLHDNCMTA
VPHILPEGYTMTVWKADRTEVTLRGKAPKESGGSSGGSSGSETPGTSESATPESSGGSS
GGSASGVDAKAILSARLSKSRRLENLIAQLPGEKKNGLFGNLIALSLGLTPNFKSNFDL
AEDAKLQLSKDTYDDDLDNLLAQIGDQYADLFLAAKNLSDAILLSDILRVNTEITKAPL
SASMIKRYDEHHQDLTLLKALVRQQLPEKYKEIFFDQSKNGYAGYIDGGASQEEFYKFI
KPILEKMDGTEELLVKLNREDLLRKQRTFDNGSIPHQIHLGELHAILRRQEDFYPFLKD
NREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLING
IRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVPQ
SFLKDDSIDNKVLTRSDKNRGKSDNVPSEEVVKKMKNYWRQLLNAKLITQRKFDNLT
KAERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVYD
VRKMIAKSEQEIGKATAKYFFYSNIMNFFKTEITLANGEIRKRPLIETNGETGEIVWDKG
RDFATVRKVLSMPQVNIVKKTEVQTGGFSKESILPKRNSDKLIARKKDWDPKKYGGFD
SPTVAYSVLVVAKV
> SEQ ID NO:205 mini-Sdd9-QBE89 
KATAKYFFYSNIMNFFKTEITLANGEIRKRPLIETNGETGEIVWDKGRDFATVRKV
LSMPQVNIVKKTEVQTGGFSKESILPKRNSDKLIARKKDWDPKKYGGFDSPTVAYSVL
VVAKVEKGKSKKLKSVKELLGITIMERSSFEKNPIDFLEAKGYKEVKKDLIIKLPKYSLF
ELENGRKRMLASAGELQKGNELALPSKYVNFLYLASHYEKLKGSPEDNEQKQLFVEQ
HKHYLDEIIEQISEFSKRVILADANLDKVLSAYNKHRDKPIREQAENIIHLFTLTNLGAPA
AFKYFDTTIDRKRYTSTKEVLDATLIHQSITGLYETRIDLSQLGGDGGSGGSGGSGGSG
GSGGSGGMDKKYSIGLAIGTNSVGWAVITDEYKVPSKKFKVLGNTDRHSIKKNLIGAL
LFDSGETAEATRLKRTARRRYTRRKNRICYLQEIFSNEMAKVDDSFFHRLEESFLVEED
KKHERHPIFGNIVDEVAYHEKYPTIYHLRKKLVDSTDKADLRLIYLALAHMIKFRGHFL
 
IEGDLNPDNSDVDKLFIQLVQTYNQLFEENPINASGVDAKAILSARLSKSRRLENLIAQL
PGEKKNGLFGNLIALSLGLTPNFKSNFDLAEDAKLQLSKDTYDDDLDNLLAQIGDQYA
DLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTF
DNGSIPHQIHLGELHAILRRQEDFYPFLKDNREKIEKILTFRIPYYVGPLARGNSRFAWM
TRKSGGSSGGSSGSETPGTSESATPESSGGSSGGSCPIWVKNALQELVGRKETSGKVFD
VDGKPIGPDIQSGYKDRELRRGVYETLRKSPFFQKHFPSTATWYVSLHVEAQYAVWML
RNRIKHATVVINNTYVCSDMNRLHDNCMTAVPHILPEGYTMTVWKADRTEVTLRGKA
PKESGGSSGGSSGSETPGTSESATPESSGGSSGGSSEETITPWNFEEVVDKGASAQSFIER
MTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLING
IRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVPQ
SFLKDDSIDNKVLTRSDKNRGKSDNVPSEEVVKKMKNYWRQLLNAKLITQRKFDNLT
KAERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVYD
VRKMIAKSEQEIG
> SEQ ID NO:206 mini-Sdd9-QBE90 
EKGKSKKLKSVKELLGITIMERSSFEKNPIDFLEAKGYKEVKKDLIIKLPKYSLFEL
ENGRKRMLASAGELQKGNELALPSKYVNFLYLASHYEKLKGSPEDNEQKQLFVEQHK
HYLDEIIEQISEFSKRVILADANLDKVLSAYNKHRDKPIREQAENIIHLFTLTNLGAPAAF
KYFDTTIDRKRYTSTKEVLDATLIHQSITGLYETRIDLSQLGGDGGSGGSGGSGGSGGS
GGSGGMDKKYSIGLAIGTNSVGWAVITDEYKVPSKKFKVLGNTDRHSIKKNLIGALLF
DSGETAEATRLKRTARRRYTRRKNRICYLQEIFSNEMAKVDDSFFHRLEESFLVEEDKK
HERHPIFGNIVDEVAYHEKYPTIYHLRKKLVDSTDKADLRLIYLALAHMIKFRGHFLIEG
DLNPDNSDVDKLFIQLVQTYNQLFEENPINASGVDAKAILSARLSKSRRLENLIAQLPG
EKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYK
EIFFDQSKNGYAGYIDGGASQEEFYKFIKPILEKMDGTEELLVKLNREDLLRKQRTFDN
GSIPHQIHLGELHAILRRQEDFYPFLKDNREKIEKILTFRIPYYVGPLARGNSRFAWMTR
KSGGSSGGSSGSETPGTSESATPESSGGSSGGSCPIWVKNALQELVGRKETSGKVFDVD
 
GKPIGPDIQSGYKDRELRRGVYETLRKSPFFQKHFPSTATWYVSLHVEAQYAVWMLRN
RIKHATVVINNTYVCSDMNRLHDNCMTAVPHILPEGYTMTVWKADRTEVTLRGKAPK
ESGGSSGGSSGSETPGTSESATPESSGGSSGGSSEETITPWNFEEVVDKGASAQSFIERM
TNFDKNLPNEKVLPKHSLLYEYFTVYNELTKVKYVTEGMRKPAFLSGEQKKAIVDLLF
KTNRKVTVKQLKEDYFKKIECFDSVEISGVEDRFNASLGTYHDLLKIIKDKDFLDNEEN
EDILEDIVLTLTLFEDREMIEERLKTYAHLFDDKVMKQLKRRRYTGWGRLSRKLINGIR
DKQSGKTILDFLKSDGFANRNFMQLIHDDSLTFKEDIQKAQVSGQGDSLHEHIANLAGS
PAIKKGILQTVKVVDELVKVMGRHKPENIVIEMARENQTTQKGQKNSRERMKRIEEGI
KELGSQILKEHPVENTQLQNEKLYLYYLQNGRDMYVDQELDINRLSDYDVDHIVPQSF
LKDDSIDNKVLTRSDKNRGKSDNVPSEEVVKKMKNYWRQLLNAKLITQRKFDNLTKA
ERGGLSELDKAGFIKRQLVETRQITKHVAQILDSRMNTKYDENDKLIREVKVITLKSKL
VSDFRKDFQFYKVREINNYHHAHDAYLNAVVGTALIKKYPKLESEFVYGDYKVYDVR
KMIAKSEQEIGKATAKYFFYSNIMNFFKTEITLANGEIRKRPLIETNGETGEIVWDKGRD
FATVRKVLSMPQVNIVKKTEVQTGGFSKESILPKRNSDKLIARKKDWDPKKYGGFDSP
TVAYSVLVVAKV
> SEQ ID NO:207 QBE84-N 
ARLSKSRRLENLIAQLPGEKKNGLFGNLIALSLGLTPNFKSNFDLAEDAKLQLSKD
TYDDDLDNLLAQIGDQYADLFLAAKNLSDAILLSDILRVNTEITKAPLSASMIKRYDEH
HQDLTLLKALVRQQLPEKYKEIFFDQSKNGYAGYIDGGASQEEFYKFIKPILEKMDGTE
ELLVKLNREDLLRKQRTFDNGSIPHQIHLGELHAILRRQEDFYPFLKDNREKIEKILTFRI
PYYVGPLARGNSRFAWMTRKSEETITPWNFEEVVDKGASAQSFIERMTNFDKNLPNEK
VLPKHSLLYEYFTVYNELTKVKYVTEGMRKPAFLSGEQKKAIVDLLFKTNRKVTVKQL
KEDYFKKIECFDSVEISGVEDRFNASLGTYHDLLKIIKDKDFLDNEENEDILEDIVLTLTL
FEDREMIEERLKTYAHLFDDKVMKQLKRRRYTGWGRLSRKLINGIRDKQSGKTILDFL
KSDGFANRNFMQLIHDDSLTFKEDIQKAQVSGQGDSLHEHIANLAGSPAIKKGILQTVK
VVDELVKVMGRHKPENIVIEMARENQTTQKGQKNSRERMKRIEEGIKELGSQILKEHP
VENTQLQNEKLYLYYLQNGRDMYVDQELDINRLSDYDVDHIVPQSFLKDDSIDNKVLT
RSDKNRGKSDNVPSEEVVKKMKNYWRQLLNAKLITQRKFDNLTKAERGGLSELDKA
GFIKRQLVETRQITKHVAQILDSRMNTKYDENDKLIREVKVITLKSKLVSDFRKDFQFY
KVREINNYHHAHDAYLNAVVGTALIKKYPKLESEFVYGDYKVYDVRKMIAKSE
> SEQ ID NO:208 QBE84-C 
QEIGKATAKYFFYSNIMNFFKTEITLANGEIRKRPLIETNGETGEIVWDKGRDFATV
RKVLSMPQVNIVKKTEVQTGGFSKESILPKRNSDKLIARKKDWDPKKYGGFDSPTVAY
 
SVLVVAKVEKGKSKKLKSVKELLGITIMERSSFEKNPIDFLEAKGYKEVKKDLIIKLPKY
SLFELENGRKRMLASAGELQKGNELALPSKYVNFLYLASHYEKLKGSPEDNEQKQLF
VEQHKHYLDEIIEQISEFSKRVILADANLDKVLSAYNKHRDKPIREQAENIIHLFTLTNLG
APAAFKYFDTTIDRKRYTSTKEVLDATLIHQSITGLYETRIDLSQLGGDGGSGGSGGSGG
SGGSGGSGGMDKKYSIGLAIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEE
DKKHERHPIFGNIVDEVAYHEKYPTIYHLRKKLVDSTDKADLRLIYLALAHMIKFRGHF
LIEGDLNPDNSDVDKLFIQLVQTYNQLFEENPINASGVDAKAILS
> SEQ ID NO:209 QBE85-N 
EKKNGLFGNLIALSLGLTPNFKSNFDLAEDAKLQLSKDTYDDDLDNLLAQIGDQY
ADLFLAAKNLSDAILLSDILRVNTEITKAPLSASMIKRYDEHHQDLTLLKALVRQQLPE
KYKEIFFDQSKNGYAGYIDGGASQEEFYKFIKPILEKMDGTEELLVKLNREDLLRKQRT
FDNGSIPHQIHLGELHAILRRQEDFYPFLKDNREKIEKILTFRIPYYVGPLARGNSRFAW
MTRKSEETITPWNFEEVVDKGASAQSFIERMTNFDKNLPNEKVLPKHSLLYEYFTVYN
ELTKVKYVTEGMRKPAFLSGEQKKAIVDLLFKTNRKVTVKQLKEDYFKKIECFDSVEIS
GVEDRFNASLGTYHDLLKIIKDKDFLDNEENEDILEDIVLTLTLFEDREMIEERLKTYAH
LFDDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKPE
NIVIEMARENQTTQKGQKNSRERMKRIEEGIKELGSQILKEHPVENTQLQNEKLYLYYL
QNGRDMYVDQELDINRLSDYDVDHIVPQSFLKDDSIDNKVLTRSDKNRGKSDNVPSEE
VVKKMKNYWRQLLNAKLITQRKFDNLTKAERGGLSELDKAGFIKRQLVETRQITKHV
AQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFK
TEITLANGEIRKRPLIETNGETGEIVWDKGRDFATVRKVLSMPQVNIVKKTEVQTGGFS
KESILPKRNSDKLIARKKDWDPKKYGGFDSPTVAYSVLVVAKVEKGKSKKLKSVKELL
GITIMERSSFEKNPIDFLEAKGYKEVKKDLIIKLPKYSLFELENGRKRMLASAGELQKG
NELALPSKYVNFLYLASHYEK
> SEQ ID NO:210 QBE85-C 
LKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDGGSGGSGGSGGSGGSGGSGGMDKKYSIGLAIGTNSVGWAVITDEYKVPSKK
FKVLGNTDRHSIKKNLIGALLFDSGETAEATRLKRTARRRYTRRKNRICYLQEIFSNEM
AKVDDSFFHRLEESFLVEEDKKHERHPIFGNIVDEVAYHEKYPTIYHLRKKLVDSTDKA
 
DLRLIYLALAHMIKFRGHFLIEGDLNPDNSDVDKLFIQLVQTYNQLFEENPINASGVDA
KAILSARLSKSRRLENLIAQLPG
> SEQ ID NO:211 QBE87-N 
KATAKYFFYSNIMNFFKTEITLANGEIRKRPLIETNGETGEIVWDKGRDFATVRKV
LSMPQVNIVKKTEVQTGGFSKESILPKRNSDKLIARKKDWDPKKYGGFDSPTVAYSVL
VVAKVEKGKSKKLKSVKELLGITIMERSSFEKNPIDFLEAKGYKEVKKDLIIKLPKYSLF
ELENGRKRMLASAGELQKGNELALPSKYVNFLYLASHYEKLKGSPEDNEQKQLFVEQ
HKHYLDEIIEQISEFSKRVILADANLDKVLSAYNKHRDKPIREQAENIIHLFTLTNLGAPA
AFKYFDTTIDRKRYTSTKEVLDATLIHQSITGLYETRIDLSQLGGDGGSGGSGGSGGSG
GSGGSGGMDKKYSIGLAIGTNSVGWAVITDEYKVPSKKFKVLGNTDRHSIKKNLIGAL
LFDSGETAEATRLKRTARRRYTRRKNRICYLQEIFSNEMAKVDDSFFHRLEESFLVEED
KKHERHPIFGNIVDEVAYHEKYPTIYHLRKKLVDSTDKADLRLIYLALAHMIKFRGHFL
IEGDLNPDNSDVDKLFIQLVQTYNQLFEENPIN
> SEQ ID NO:212 QBE87-C 
ASGVDAKAILSARLSKSRRLENLIAQLPGEKKNGLFGNLIALSLGLTPNFKSNFDL
AEDAKLQLSKDTYDDDLDNLLAQIGDQYADLFLAAKNLSDAILLSDILRVNTEITKAPL
SASMIKRYDEHHQDLTLLKALVRQQLPEKYKEIFFDQSKNGYAGYIDGGASQEEFYKFI
KPILEKMDGTEELLVKLNREDLLRKQRTFDNGSIPHQIHLGELHAILRRQEDFYPFLKD
NREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLING
IRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVPQ
SFLKDDSIDNKVLTRSDKNRGKSDNVPSEEVVKKMKNYWRQLLNAKLITQRKFDNLT
KAERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVYD
VRKMIAKSEQEIG
> SEQ ID NO:213 QBE88-N 
EKGKSKKLKSVKELLGITIMERSSFEKNPIDFLEAKGYKEVKKDLIIKLPKYSLFEL
ENGRKRMLASAGELQKGNELALPSKYVNFLYLASHYEKLKGSPEDNEQKQLFVEQHK
 
HYLDEIIEQISEFSKRVILADANLDKVLSAYNKHRDKPIREQAENIIHLFTLTNLGAPAAF
KYFDTTIDRKRYTSTKEVLDATLIHQSITGLYETRIDLSQLGGDGGSGGSGGSGGSGGS
GGSGGMDKKYSIGLAIGTNSVGWAVITDEYKVPSKKFKVLGNTDRHSIKKNLIGALLF
DSGETAEATRLKRTARRRYTRRKNRICYLQEIFSNEMAKVDDSFFHRLEESFLVEEDKK
HERHPIFGNIVDEVAYHEKYPTIYHLRKKLVDSTDKADLRLIYLALAHMIKFRGHFLIEG
DLNPDNSDVDKLFIQLVQTYNQLFEENPIN
> SEQ ID NO:214 QBE88-C 
ASGVDAKAILSARLSKSRRLENLIAQLPGEKKNGLFGNLIALSLGLTPNFKSNFDL
AEDAKLQLSKDTYDDDLDNLLAQIGDQYADLFLAAKNLSDAILLSDILRVNTEITKAPL
SASMIKRYDEHHQDLTLLKALVRQQLPEKYKEIFFDQSKNGYAGYIDGGASQEEFYKFI
KPILEKMDGTEELLVKLNREDLLRKQRTFDNGSIPHQIHLGELHAILRRQEDFYPFLKD
NREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDL
LFKTNRKVTVKQLKEDYFKKIECFDSVEISGVEDRFNASLGTYHDLLKIIKDKDFLDNE
ENEDILEDIVLTLTLFEDREMIEERLKTYAHLFDDKVMKQLKRRRYTGWGRLSRKLING
IRDKQSGKTILDFLKSDGFANRNFMQLIHDDSLTFKEDIQKAQVSGQGDSLHEHIANLA
GSPAIKKGILQTVKVVDELVKVMGRHKPENIVIEMARENQTTQKGQKNSRERMKRIEE
GIKELGSQILKEHPVENTQLQNEKLYLYYLQNGRDMYVDQELDINRLSDYDVDHIVPQ
SFLKDDSIDNKVLTRSDKNRGKSDNVPSEEVVKKMKNYWRQLLNAKLITQRKFDNLT
KAERGGLSELDKAGFIKRQLVETRQITKHVAQILDSRMNTKYDENDKLIREVKVITLKS
KLVSDFRKDFQFYKVREINNYHHAHDAYLNAVVGTALIKKYPKLESEFVYGDYKVYD
VRKMIAKSEQEIGKATAKYFFYSNIMNFFKTEITLANGEIRKRPLIETNGETGEIVWDKG
RDFATVRKVLSMPQVNIVKKTEVQTGGFSKESILPKRNSDKLIARKKDWDPKKYGGFD
SPTVAYSVLVVAKV
> SEQ ID NO:215 QBE89-N 
KATAKYFFYSNIMNFFKTEITLANGEIRKRPLIETNGETGEIVWDKGRDFATVRKV
LSMPQVNIVKKTEVQTGGFSKESILPKRNSDKLIARKKDWDPKKYGGFDSPTVAYSVL
VVAKVEKGKSKKLKSVKELLGITIMERSSFEKNPIDFLEAKGYKEVKKDLIIKLPKYSLF
ELENGRKRMLASAGELQKGNELALPSKYVNFLYLASHYEKLKGSPEDNEQKQLFVEQ
HKHYLDEIIEQISEFSKRVILADANLDKVLSAYNKHRDKPIREQAENIIHLFTLTNLGAPA
AFKYFDTTIDRKRYTSTKEVLDATLIHQSITGLYETRIDLSQLGGDGGSGGSGGSGGSG
GSGGSGGMDKKYSIGLAIGTNSVGWAVITDEYKVPSKKFKVLGNTDRHSIKKNLIGAL
LFDSGETAEATRLKRTARRRYTRRKNRICYLQEIFSNEMAKVDDSFFHRLEESFLVEED
 
KKHERHPIFGNIVDEVAYHEKYPTIYHLRKKLVDSTDKADLRLIYLALAHMIKFRGHFL
IEGDLNPDNSDVDKLFIQLVQTYNQLFEENPINASGVDAKAILSARLSKSRRLENLIAQL
PGEKKNGLFGNLIALSLGLTPNFKSNFDLAEDAKLQLSKDTYDDDLDNLLAQIGDQYA
DLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTF
DNGSIPHQIHLGELHAILRRQEDFYPFLKDNREKIEKILTFRIPYYVGPLARGNSRFAWM
TRK
> SEQ ID NO:216 QBE89-C 
SEETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHL
FDDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKPE
NIVIEMARENQTTQKGQKNSRERMKRIEEGIKELGSQILKEHPVENTQLQNEKLYLYYL
QNGRDMYVDQELDINRLSDYDVDHIVPQSFLKDDSIDNKVLTRSDKNRGKSDNVPSEE
VVKKMKNYWRQLLNAKLITQRKFDNLTKAERGGLSELDKAGFIKRQLVETRQITKHV
AQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIG
> SEQ ID NO:217 QBE90-N 
EKGKSKKLKSVKELLGITIMERSSFEKNPIDFLEAKGYKEVKKDLIIKLPKYSLFEL
ENGRKRMLASAGELQKGNELALPSKYVNFLYLASHYEKLKGSPEDNEQKQLFVEQHK
HYLDEIIEQISEFSKRVILADANLDKVLSAYNKHRDKPIREQAENIIHLFTLTNLGAPAAF
KYFDTTIDRKRYTSTKEVLDATLIHQSITGLYETRIDLSQLGGDGGSGGSGGSGGSGGS
GGSGGMDKKYSIGLAIGTNSVGWAVITDEYKVPSKKFKVLGNTDRHSIKKNLIGALLF
DSGETAEATRLKRTARRRYTRRKNRICYLQEIFSNEMAKVDDSFFHRLEESFLVEEDKK
HERHPIFGNIVDEVAYHEKYPTIYHLRKKLVDSTDKADLRLIYLALAHMIKFRGHFLIEG
DLNPDNSDVDKLFIQLVQTYNQLFEENPINASGVDAKAILSARLSKSRRLENLIAQLPG
EKKNGLFGNLIALSLGLTPNFKSNFDLAEDAKLQLSKDTYDDDLDNLLAQIGDQYADL
FLAAKNLSDAILLSDILRVNTEITKAPLSASMIKRYDEHHQDLTLLKALVRQQLPEKYK
EIFFDQSKNGYAGYIDGGASQEEFYKFIKPILEKMDGTEELLVKLNREDLLRKQRTFDN
GSIPHQIHLGELHAILRRQEDFYPFLKDNREKIEKILTFRIPYYVGPLARGNSRFAWMTR
K
> SEQ ID NO:218 QBE90-C 
SEETITPWNFEEVVDKGASAQSFIERMTNFDKNLPNEKVLPKHSLLYEYFTVYNEL
TKVKYVTEGMRKPAFLSGEQKKAIVDLLFKTNRKVTVKQLKEDYFKKIECFDSVEISG
VEDRFNASLGTYHDLLKIIKDKDFLDNEENEDILEDIVLTLTLFEDREMIEERLKTYAHL
FDDKVMKQLKRRRYTGWGRLSRKLINGIRDKQSGKTILDFLKSDGFANRNFMQLIHD
DSLTFKEDIQKAQVSGQGDSLHEHIANLAGSPAIKKGILQTVKVVDELVKVMGRHKPE
NIVIEMARENQTTQKGQKNSRERMKRIEEGIKELGSQILKEHPVENTQLQNEKLYLYYL
QNGRDMYVDQELDINRLSDYDVDHIVPQSFLKDDSIDNKVLTRSDKNRGKSDNVPSEE
VVKKMKNYWRQLLNAKLITQRKFDNLTKAERGGLSELDKAGFIKRQLVETRQITKHV
AQILDSRMNTKYDENDKLIREVKVITLKSKLVSDFRKDFQFYKVREINNYHHAHDAYL
NAVVGTALIKKYPKLESEFVYGDYKVYDVRKMIAKSEQEIGKATAKYFFYSNIMNFFK
TEITLANGEIRKRPLIETNGETGEIVWDKGRDFATVRKVLSMPQVNIVKKTEVQTGGFS
KESILPKRNSDKLIARKKDWDPKKYGGFDSPTVAYSVLVVAKV
> SEQ ID NO:219 Linker
   GGSGGSGGSGGSGGSGGSGGSGGS
> SEQ ID NO:220 gRNA scaffold 
GUUUGAGAGCUAUGCUGGAAACAGCAUAGCAAGUUCAAAUAAGGCUAGUC
CGUUAUCAACUUGAAAAAGUGGCACCGAGUCGGUGC
> SEQ ID NO:221 gRNA scaffold

GUUUUAGAGCUAGAAAUAGCAAGUUAAAAUAAGGCUAGUCCGUAAUCAAC
UUUUAAAGAGUGAGCACUGAUUCGGUGC

## Claims

1. A base editing fusion protein, wherein the base editing fusion protein comprises a deaminase domain and paired DNA-binding protein domains having a DNA single-strand cleavage function; and
the paired DNA-binding protein domains having the DNA single-strand cleavage function comprise a deaminase N-terminal linker domain and a deaminase C-terminal linker domain that are linked via an optional linker to the N-terminus and the C-terminus of the deaminase domain, respectively.

2. The base editing fusion protein according to claim 1, wherein the base editing fusion protein comprises the following structure: [deaminase N-terminal linker domain]-[deaminase]-[deaminase C-terminal linker domain], wherein "-" is an optional linker.

3. The base editing fusion protein according to claim 2, wherein the deaminase N-terminal linker domain and the deaminase C-terminal linker domain are independently composed of 1 or 2 single domains; and
optionally, the base editing fusion protein comprises the following structure: [X₁]-[X₂]-[deaminase]-[X₃]-[X₄], wherein X₁₋₄ are single domains, and "-" is an optional linker.

4. The base editing fusion protein according to claim 3, wherein the X₁₋₄ single domains are selected from the following combinations:
(1) X₁ is SEQ ID NO: 158, X₂ is SEQ ID NO: 159, X₃ is SEQ ID NO: 160, and X₄ is absent;
(2) X₁ is absent, X₂ is SEQ ID NO: 161, X₃ is SEQ ID NO: 162, and X₄ is SEQ ID NO: 163;
(3) X₁ is absent, X₂ is SEQ ID NO: 164, X₃ is SEQ ID NO: 165, and X₄ is SEQ ID NO: 166;
(4) X₁ is SEQ ID NO: 181, X₂ is SEQ ID NO: 182, X₃ is a sequence Q, and X₄ is absent;
(5) X₁ is absent, X₂ is SEQ ID NO: 183, X₃ is SEQ ID NO: 184, and X₄ is SEQ ID NO: 185;
(6) X₁ is absent, X₂ is SEQ ID NO: 186, X₃ is SEQ ID NO: 187, and X₄ is SEQ ID NO: 188;
(7) X₁ is SEQ ID NO: 189, X₂ is SEQ ID NO: 190, X₃ is SEQ ID NO: 191, and X₄ is absent;
(8) X₁ is SEQ ID NO: 192, X₂ is SEQ ID NO: 193, X₃ is SEQ ID NO: 194, and X₄ is absent;
(9) X₁ is SEQ ID NO: 195, X₂ is SEQ ID NO: 196, X₃ is SEQ ID NO: 197, and X₄ is absent; and
(10) X₁ is SEQ ID NO: 198, X₂ is SEQ ID NO: 199, X₃ is SEQ ID NO: 200, and X₄ is absent.

5. The base editing fusion protein according to any one of claims 1 to 4, wherein the deaminase is a cytidine deaminase or an adenosine deaminase.

6. The base editing fusion protein according to claim 5, wherein the cytidine deaminase is an APOBEC family deaminase, an SCP1.201 family deaminase or a homolog thereof;
preferably, the APOBEC family deaminase is an AID deaminase, an APOBEC1 deaminase, an APOBEC2 deaminase, an APOBEC3A deaminase, an APOBEC3B deaminase, an APOBEC3C deaminase, an APOBEC3D deaminase, an APOBEC3F deaminase, an APOBEC3G deaminase, an APOBEC3H deaminase, or a variant thereof; and
preferably, the SCP1.201 family deaminase is an Sdd2 deaminase, an Sdd3 deaminase, an Sdd4 deaminase, an Sdd6 deaminase, an Sdd7 deaminase, an Sdd9 deaminase, an Sdd10 deaminase, an Sdd59 deaminase, a mini-Sdd3 deaminase, a mini-Sdd6 deaminase, a mini-Sdd7 deaminase, a mini-Sdd9 deaminase, or a variant thereof.

7. The base editing fusion protein according to claim 5, wherein the adenosine deaminase is QB34, TadA8e, TadA7.10, a TadA dimer, or a variant thereof, and optionally, the TadA dimer comprises TadA7.10 and wild-type TadA.

8. The base editing fusion protein according to any one of claims 1 to 7, wherein the base editing fusion protein further comprises a uracil DNA glycosylase inhibitor (UGI) domain and/or a nuclear localization sequence (NLS).

9. The base editing fusion protein according to any one of claims 1 to 8, wherein the linker has a length of 0 to 100 amino acids;
preferably, the linker has a length of 12 to 100 amino acids; and
optionally, the linker comprises an amino acid sequence (GGGS)n (SEQ ID NO: 47), (GGGGS)n (SEQ ID NO: 48), (G)n, (EAAAK)n (SEQ ID NO: 49), (GGS)n, (SGGS)n (SEQ ID NO: 51), SGSETPGTSESATPES (SEQ ID NO: 52), or an (XP)n motif, or a combination thereof, wherein n is independently an integer from 1 to 32, and wherein X is any amino acid.

10. The base editing fusion protein according to any one of claims 1 to 9, wherein the base editing fusion protein comprises one or more of the following sequences:
(i) an amino acid sequence set forth in any one of SEQ ID NOs: 89-92, 94, 96, 99, 101, 103, 105-115, 118, 179, and 201-206;
(ii) an amino acid sequence having at least 80%, 82%, 85%, 87%, 90%, 92%, 95%, 96%, 97%, 98% or 99% sequence identity to the amino acid sequence set forth in any one of SEQ ID NOs: 89-92, 94, 96, 99, 101, 103, 105-115, 118, 179, and 201-206;
(iii) an amino acid sequence obtained by adding, substituting, deleting or inserting one or more amino acid residues in the amino acid sequence set forth in any one of SEQ ID NOs: 89-92, 94, 96, 99, 101, 103, 105-115, 118, 179, and 201-206; or
(iv) an amino acid sequence encoded by a nucleotide sequence that hybridizes with a polynucleotide sequence encoding the amino acid sequence set forth in any one of SEQ ID NOs: 89-92, 94, 96, 99, 101, 103, 105-115, 118, 179, and 201-206 under stringency conditions, wherein the stringency conditions are moderate stringency conditions, moderate-high stringency conditions, high stringency conditions, or very high stringency conditions.

11. The base editing fusion protein according to any one of claims 1 to 10, wherein the base editing fusion protein forms a complex with a guide RNA and binds to a target DNA.

12. A base editing system, wherein the base editing system comprises at least one of the following components i) to v):
i) a base editing fusion protein, and a guide RNA;
ii) an expression construct comprising a nucleotide sequence encoding a base editing fusion protein, and a guide RNA;
iii) a base editing fusion protein, and an expression construct comprising a nucleotide sequence encoding a guide RNA;
iv) an expression construct comprising a nucleotide sequence encoding a base editing fusion protein, and an expression construct comprising a nucleotide sequence encoding a guide RNA; and
v) an expression construct comprising a nucleotide sequence encoding a base editing fusion protein and a nucleotide sequence encoding a guide RNA;
wherein the base editing fusion protein comprises a deaminase domain and paired DNA-binding protein domains having a DNA single-strand cleavage function; and
the paired DNA-binding protein domains having the DNA single-strand cleavage function comprise a deaminase N-terminal linker domain and a deaminase C-terminal linker domain that are linked via an optional linker to the N-terminus and the C-terminus of the deaminase domain, respectively.

13. The base editing system according to claim 12, wherein the base editing fusion protein comprises the following structure: [deaminase N-terminal linker domain]-[deaminase]-[deaminase C-terminal linker domain], wherein "-" is an optional linker.

14. The base editing system according to claim 13, wherein the deaminase N-terminal linker domain and the deaminase C-terminal linker domain are independently composed of 1 or 2 single domains; and
optionally, the base editing fusion protein comprises the following structure: [X₁]-[X₂]-[deaminase]-[X₃]-[X₄], wherein X₁₋₄ are single domains, and "-" is an optional linker.

15. The base editing system according to claim 14, wherein the X₁₋₄ single domains are selected from the following combinations:
(1) X₁ is SEQ ID NO: 158, X₂ is SEQ ID NO: 159, X₃ is SEQ ID NO: 160, and X₄ is absent;
(2) X₁ is absent, X₂ is SEQ ID NO: 161, X₃ is SEQ ID NO: 162, and X₄ is SEQ ID NO: 163;
(3) X₁ is absent, X₂ is SEQ ID NO: 164, X₃ is SEQ ID NO: 165, and X₄ is SEQ ID NO: 166;
(4) X₁ is SEQ ID NO: 181, X₂ is SEQ ID NO: 182, X₃ is a sequence Q, and X₄ is absent;
(5) X₁ is absent, X₂ is SEQ ID NO: 183, X₃ is SEQ ID NO: 184, and X₄ is SEQ ID NO: 185;
(6) X₁ is absent, X₂ is SEQ ID NO: 186, X₃ is SEQ ID NO: 187, and X₄ is SEQ ID NO: 188;
(7) X₁ is SEQ ID NO: 189, X₂ is SEQ ID NO: 190, X₃ is SEQ ID NO: 191, and X₄ is absent;
(8) X₁ is SEQ ID NO: 192, X₂ is SEQ ID NO: 193, X₃ is SEQ ID NO: 194, and X₄ is absent;
(9) X₁ is SEQ ID NO: 195, X₂ is SEQ ID NO: 196, X₃ is SEQ ID NO: 197, and X₄ is absent; and
(10) X₁ is SEQ ID NO: 198, X₂ is SEQ ID NO: 199, X₃ is SEQ ID NO: 200, and X₄ is absent.

16. The base editing system according to any one of claims 12 to 15, wherein the deaminase is a cytidine deaminase or an adenosine deaminase.

17. The base editing system according to claim 16, wherein the cytidine deaminase is an APOBEC family deaminase, an SCP1.201 family deaminase or a homolog thereof;
preferably, the APOBEC family deaminase is an AID deaminase, an APOBEC1 deaminase, an APOBEC2 deaminase, an APOBEC3A deaminase, an APOBEC3B deaminase, an APOBEC3C deaminase, an APOBEC3D deaminase, an APOBEC3F deaminase, an APOBEC3G deaminase, an APOBEC3H deaminase, or a variant thereof; and
preferably, the SCP1.201 family deaminase is an Sdd2 deaminase, an Sdd3 deaminase, an Sdd4 deaminase, an Sdd6 deaminase, an Sdd7 deaminase, an Sdd9 deaminase, an Sdd10 deaminase, an Sdd59 deaminase, a mini-Sdd3 deaminase, a mini-Sdd6 deaminase, a mini-Sdd7 deaminase, a mini-Sdd9 deaminase, or a variant thereof.

18. The base editing system according to claim 16, wherein the adenosine deaminase is QB34, TadA8e, TadA7.10, a TadA dimer, or a variant thereof, and optionally, the TadA dimer comprises TadA7.10 and wild-type TadA.

19. The base editing system according to any one of claims 12 to 18, wherein the base editing fusion protein further comprises a uracil DNA glycosylase inhibitor (UGI) domain and/or a nuclear localization sequence (NLS).

20. The base editing system according to any one of claims 12 to 19, wherein the linker has a length of 0 to 100 amino acids;
preferably, the linker has a length of 12 to 100 amino acids; and
optionally, the linker comprises an amino acid sequence (GGGS)n (SEQ ID NO: 47), (GGGGS)n (SEQ ID NO: 48), (G)n, (EAAAK)n (SEQ ID NO: 49), (GGS)n, (SGGS)n (SEQ ID NO: 51), SGSETPGTSESATPES (SEQ ID NO: 52), or an (XP)n motif, or a combination thereof, wherein n is independently an integer from 1 to 32, and wherein X is any amino acid.

21. The base editing system according to any one of claims 12 to 20, wherein the base editing fusion protein comprises one or more of the following sequences:
(i) an amino acid sequence set forth in any one of SEQ ID NOs: 89-92, 94, 96, 99, 101, 103, 105-115, 118, 179, and 201-206;
(ii) an amino acid sequence having at least 80%, 82%, 85%, 87%, 90%, 92%, 95%, 96%, 97%, 98% or 99% sequence identity to the amino acid sequence set forth in any one of SEQ ID NOs: 89-92, 94, 96, 99, 101, 103, 105-115, 118, 179, and 201-206;
(iii) an amino acid sequence obtained by adding, substituting, deleting or inserting one or more amino acid residues in the amino acid sequence set forth in any one of SEQ ID NOs: 89-92, 94, 96, 99, 101, 103, 105-115, 118, 179, and 201-206; or
(iv) an amino acid sequence encoded by a nucleotide sequence that hybridizes with a polynucleotide sequence encoding the amino acid sequence set forth in any one of SEQ ID NOs: 89-92, 94, 96, 99, 101, 103, 105-115, 118, 179, and 201-206 under stringency conditions, wherein the stringency conditions are moderate stringency conditions, moderate-high stringency conditions, high stringency conditions, or very high stringency conditions.

22. The base editing system according to any one of claims 12 to 21, wherein the base editing fusion protein forms a complex with a guide RNA and binds to a target DNA.

23. A polynucleotide, wherein the polynucleotide encodes:
(a) the base editing fusion protein according to any one of claims 1 to 11; or
(b) the base editing system according to any one of claims 12 to 22.

24. An expression construct, wherein the expression construct comprises the polynucleotide according to claim 23.

25. The expression construct according to claim 24, wherein the expression construct is selected from expression constructs from viruses, bacteria, yeast, plants, and mammalian cells.

26. A host cell, wherein the host cell contains the base editing fusion protein according to any one of claims 1 to 11, the base editing system according to any one of claims 12 to 22, the polynucleotide according to claim 23, or the expression construct according to claim 24 or 25.

27. A method for producing at least one genetically modified cell, comprising introducing the base editing fusion protein according to any one of claims 1 to 11, the base editing system according to any one of claims 12 to 22, the polynucleotide according to claim 23, or the expression construct according to claim 24 or 25 into the at least one cell to cause a base substitution in a nucleic acid sequence within a target nucleotide region in the at least one cell.

28. The method according to claim 27, wherein the base editing system is introduced into the cell by a method selected from: calcium phosphate transfection, protoplast fusion, electroporation, liposome transfection, microinjection, viral infection (such as baculovirus, vaccinia virus, adenovirus, adeno-associated virus, lentivirus, or other viruses), N-acetylgalactosamine (GalNAc)-mediated transformation, particle bombardment, PEG-mediated protoplast transformation and/or *Agrobacterium*-mediated transformation.

29. The method according to claim 27 or 28, wherein the cell is a cell derived from a prokaryote such as bacteria; or a eukaryote such as plants, fungi or vertebrates.

30. The method according to claim 29, wherein the vertebrates are mammals such as human, mouse, rat, monkey, dog, pig, sheep, cattle, and cat; and the plants are crop plants, such as wheat, rice, corn, soybean, sunflower, sorghum, oilseed rape, alfalfa, cotton, barley, millet, sugar cane, tomato, tobacco, cassava or potato.

31. A pharmaceutical composition, wherein the pharmaceutical composition comprises the base editing fusion protein according to any one of claims 1 to 11, the base editing system according to any one of claims 12 to 22, the polynucleotide according to claim 23, the expression construct according to claim 24 or 25, or the host cell according to claim 26; and
optionally, the pharmaceutical composition further comprises a pharmaceutically acceptable carrier.

32. A kit, wherein the kit comprises the base editing fusion protein according to any one of claims 1 to 11, the base editing system according to any one of claims 12 to 22, the polynucleotide according to claim 23, the expression construct according to claim 24 or 25, or the host cell according to claim 26.

33. A method for modifying a target nucleic acid, wherein the method comprises a step of bringing the target nucleic acid into contact with the base editing fusion protein according to any one of claims 1 to 11, the base editing system according to any one of claims 12 to 22, the polynucleotide according to claim 23, the expression construct according to claim 24 or 25, the pharmaceutical composition according to claim 31, or the kit according to claim 32.

34. Use of the base editing fusion protein according to any one of claims 1 to 11, the base editing system according to any one of claims 12 to 22, the polynucleotide according to claim 23, the expression construct according to claim 24 or 25, the host cell according to claim 26, or the pharmaceutical composition according to claim 31 in preparation of a reagent for modifying a target nucleic acid.

35. Use of the base editing fusion protein according to any one of claims 1 to 11, the base editing system according to any one of claims 12 to 22, the polynucleotide according to claim 23, the expression construct according to claim 24 or 25, or the host cell according to claim 26 in preparation of a medicament for preventing and/or treating a disease associated with or caused by a gene mutation.
